Europäisches Patentamt

(19) European Patent Office

Office européen des brevets

(11) Publication number: **0 173 378 B1**

(12) **EUROPEAN PATENT SPECIFICATION**

(45) Date of publication of patent specification: **12.06.91**

(51) Int. Cl.⁵: **C12N 15/63**, C12N 15/81, C11D 3/386, C12P 21/02, C12N 9/02

(21) Application number: 85201235.0

(22) Date of filing: 25.07.85

(54) **Process for preparing a polypeptide by culturing a transformed microorganism, a transformed microorganism suitable therefor and DNA sequences suitable for preparing such microorganism.**

(30) Priority: 27.07.84 EP 84201114
07.02.85 GB 8503160

(43) Date of publication of application:
05.03.86 Bulletin 86/10

(45) Publication of the grant of the patent:
12.06.91 Bulletin 91/24

(84) Designated Contracting States:
AT BE CH DE FR GB IT LI NL SE

(56) References cited:
EP-A- 0 066 994     EP-A- 0 086 139
EP-A- 0 098 533     EP-A- 0 103 887
WO-A-84/04539     WO-A-85/01063

CHEMICAL ABSTRACTS, vol. 100, no.7, February 1984, p. 147, ref. no. 46308t; Columbus, Ohio, US; G. BRANLANT et al.:"Molecular cloning of the glyceraldehyde-3-phosphate dehydrogenase genes of Bacillus stearothermophilus and Escherichia coli, and their expression in Escherichia coli."

(73) Proprietor: **UNILEVER NV**
**Burgemeester s'Jacobplein 1 P.O. Box 760**
**NL-3000 DK Rotterdam(NL)BE CH DE FR IT LI**
**NL SE AT**

Proprietor: **UNILEVER PLC**
**Unilever House Blackfriars P.O. Box 68**
**London EC4P 4BQ(GB)GB**

(72) Inventor: **Ledeboer, Adrianus Marinus**
**Burg. leFèvre de Montignyplein 8**
**NL-3055 NL Rotterdam(NL)**
Inventor: **Maat, Jan**
**Molenstraat 2**
**NL-2681 BS Monster(NL)**
Inventor: **Verrips, Cornelis Theodorus**
**Hagedoorn 18**
**NL-3142 KB Maassluis(NL)**
Inventor: **Visser, Christiaan**
**Minoeserf 77**
**NL-2907 VJ Capelle a/d Ijssel(NL)**
Inventor: **Janowicz, Zbigniew Alojzy**
**Adalbert-Stifter-Strasse 49**
**W-4006 Erkrath-Unterfeldhaus(DE)**

Rank Xerox (UK) Business Services

CHEMICAL ABSTRACTS, vol. 96, no.5, March 1982, p. 121, ref.no. 63538j; Columbus, Ohio, US; C.A. LEE et al.:"Plasmid-directed synthesis of enzymes required for D-mannitol transport and utilization in Escherichia coli."

CHEMICAL ABSTRACTS, vol. 94, no. 21, May 1981, p. 375, ref.no. 170905g; Columbus, Ohio, US; E. LOHMEIER et al.:"Cloning and expression of the fumarate reductase gene of Escherichia coli."

JOURNAL OF BIOCHMISTRY, vol. 91, no. 4, April 1982, pp. 1205-1212; Tokyo, JP; M IWAKURA et al.:"Cloning of dihydrofolate reductase gene of Escherichia coli K12."

CHEMICAL ABSTRACTS, vol. 99, no. 1, July 4, 1983, p. 227, ref. no. 2206q; Columbus, Ohio, US; M.J. WAITES et al.:"Dihydroxyacetone synthese: a special transketolase for formaldehyde fixation from the methylotrophic yeast Candida boidinii CBS 5777."

CHEMICAL ABSTRACTS, vol. 96, no. 13, March 29, 1982, p. 560, ref.no. 102478m; Columbus, Ohio, US; M. BRAVO et al.:"Enzymic oxidation of methanol to produce formaldehyde and hydrogen peroxide."

CHEMICAL ABSTRACTS, vol. 95, no. 5, August 3, 1981, p. 291, ref.no. 37855v; Columbus, Ohio, US; J. GEISSLER et al.:"Yeast methanol oxidases: An unusual type of flavoprotein."

NUCLEIC ACIDS RESEARCH, vol. 13, no. 9, May 1985, pp. 3063-3082, Oxford, GB; A.M. LEDEBOER et al.:"Molecular cloning and characterization of a gene coding for methanol oxidase in Hansenula polymorpha."

Cell 35 (1983), pp. 403-410

Nature vol. 296 (1982) pp. 39-42

Inventor: **Hollenberg, Cornelis Petrus**
**Chopin-Strasse 7**
**W-4000 Düsseldorf(DE)**

(74) Representative: **van der Toorren, Johannes, Drs. et al**
**UNILEVER N.V. Patent Division Postbus 137**
**NL-3130 AC Vlaardingen(NL)**

## Description

The present invention relates to a process for preparing a polypeptide by culturing a transformed microorganism under suitable conditions, optionally concentrating the polypeptide and collecting same in a manner known per se. Such polypeptide can be an oxidoreductase, but also other enzymes or other proteins. The invention is a result from research leading to an improved process for the preparation of oxidoreductases.

Oxidoreductases, especially those which use oxygen as electron acceptor, are enzymes suitable for use in bleaching and/or detergent compositions in which they can be used for the in situ formation of bleaching agents, e.g. $H_2O_2$, during the washing or bleaching process. See for example GB-PS 1 225 713, DE-PA 2 557 623, and GB-PA 2 101 167.

Up to now a need exists for oxidase-enzymes that are more suitable for use under the conditions of manufacture and use of detergent and bleaching products.

For an economically feasible production of these oxidases it is further required to reach a yield of these enzymes in fermentation processes in the order of that of alcohol oxidase of H. polymorpha, which is up to 20% of the cellular protein (van Dijken et al., 1976).

One way of finding new microorganisms producing enzymes in higher amounts or finding new oxidase-enzymes having improved properties is to check all sorts of microorganisms and try to isolate the relevant oxidases, which are then checked for their abilities to generate peroxides and their stabilities under the conditions of manufacture and use of detergent and bleaching products. One can hope that some day a suitable enzyme will be found, but the chance of success is unpredictable and probably very low.

Another way is to apply another trial and error method of crossing the natural microorganisms producing these oxidases by classical genetic techniques, in the hope that some day one will find a more productive microorganism or a more suitable enzyme, but again the chance of success is rather low.

Clearly, a need exists for a method for preparing oxidase-enzymes in higher yield and/or without the concomitant formation of catalase and/or having improved properties during storage and/or use in e.g. bleach and/or detergent compositions. The problem of trial and error can be overcome by a process for preparing an oxidase-enzyme by culturing a microorganism under suitable conditions, and preferably concentrating the enzyme and collecting the concentrated enzyme in a manner known per se, which process is characterized in that a microorganism is used that has been obtained by recombinant DNA technology and which is capable of producing said oxidase-enzyme.

The microorganisms suitable for use in a process for preparing an oxidase-enzyme can be obtained by recombinant DNA technology, whereby a microorganism is transformed by a DNA sequence coding for an oxidase-enzyme (so-called structural gene) together with one or more other DNA sequences which regulate the expression of the structural gene in a particular microorganism or group of organisms, either via introduction of an episomal vector containing said sequences or via a vector containing said sequences which is also equipped with DNA sequences capable of being integrated into the chromosome of the microorganism.

The determination of a structural gene coding for the enzyme alcohol oxidase (EC 1.1.3.13) originating from H. polymorpha together with its regulatory 5'- and 3'-flanking regions will be described as an example of the invention without the scope of the invention being limited to this example. The spirit of the invention is also applicable to the isolation of DNA sequences of other oxidase-enzymes such as glycerol oxidase, glucose oxidase, D-amino acid oxidase etc.; the incorporation of the DNA sequences or modifications thereof into the genome of microorganisms or into episomal vectors used for transforming microorganisms and the culturing of the transformed microorganisms so obtained as such or for producing the desired oxidase-enzymes, as well as other polypeptides.

Although the microorganisms to be used can be bacteria, e.g. of the genus Bacillus, as well as moulds, the use of yeasts is preferred for technological and economical reasons. In particular a mould or yeast can be selected from the genera Aspergillus, Candida, Geotrichum, Hansenula, Lenzites, Nadsonia, Pichia, Poria, Polyporus, Saccharomyces, Sporobolomyces, Torulopsis, Trichosporon and Zendera, more particularly from the species A. japonicus, A. niger, A. oryzae, C. boidinii, H. polymorpha, Pichia pastoris and Kloeckera sp. 2201. The latter name is sometimes used instead of C. boidinii.

Many $C_1$-utilizing yeasts have been isolated during the last decade, and for Hansenula polymorpha and Candida boidinii the methanol metabolism has been studied extensively (for a review see Veenhuis et al., 1983).

The first step in this metabolism is the oxidation of methanol to formaldehyde and $H_2O_2$ catalysed by MOX. Formaldehyde is oxidized further by the action of formaldehyde dehydrogenase and formate dehydrogenase. $H_2O_2$ is split into water and oxygen by catalase.

3

Alternatively, methanol is assimilated into cellular material. After its conversion into formaldehyde, this product is fixed through the xylulose monophosphate pathway into carbohydrates. Dihydroxyacetone synthase (DHAS) plays a crucial role in this assimilation process.

The appearance of MOX, formate dehydrogenase, formaldehyde dehydrogenase, DHAS and catalase is subject to glucose repression, e.g. on 0.5% glucose. However, synthesis of MOX is derepressed by growth in low concentrations of glucose (0.1%), contrary to the synthesis of DHAS, which is still fully repressed under these conditions (Roggenkamp et al., 1984).

Regulation, i.e. the possibility to switch "on" or "off" of the gene for the polypeptide concerned, is desirable, because it allows for biomass production, when desired, by selecting a suitable substrate, such as, for example melasse, and for production of the polypeptide concerned, when desired, by using methanol or mixtures of methanol and other carbon sources. Methanol is a rather cheap substrate, so the polypeptide production may be carried out in a very economical way.

After derepression of the gene coding for alcohol oxidase (MOX) by growth on methanol, large microbodies, the peroxisomes are formed. While glucose-grown cells contain only a small peroxisome, up to 80% of the internal volume of the cell is replaced by peroxisomes in the derepressed state. The conversion of methanol into formaldehyde and $H_2O_2$ as well as the degradation of $H_2O_2$ has been shown to occur in these peroxisomes, while further oxidation or assimilation of formaldehyde most probably occurs in the cytoplasm. This process is a perfect example of compartmentalization of toxic products, of a strong co-ordinate derepression of several cellular processes and of the selective translocation of at least two of the enzymes involved in this process.

Most of the enzymes involved in the methanol metabolism have been purified and characterized (Sahm, 1977, Bystrykh et al, 1981). Especially methanol oxidase (EC 1.1.3.13) has been studied in detail. It is an octamer consisting of identical monomers with an $M_r$ value of about 74 kd and it contains FAD as a prosthetic group. Up to now no cleavable signal sequence for translocation could be detected, as concluded from electro-elephoresis studies with in vivo and in vitro synthesized products (Roa and Blobel, 1983) or from in vitro synthesis in the presence of microsomal membranes (Roggenkamp et al., 1984).

Under derepressed conditions, up to 20% of the cellular protein consists of MOX.

Materials and methods

a) Microorganisms and cultivation conditions

Hansenula polymorpha CBS 4732 was obtained from Dr J.P. van Dijken (University of Technology, Delft, The Netherlands). Cells were grown at 37°C in 1 litre Erlenmeyer flasks containing 300 ml minimal medium (Veenhuis et al., 1978), supplemented with 0.5% (v/v) methanol or 0.5% (v/v) ethanol as indicated. Phage lambda L47.1 and the P2 lysogenic E. coli K12 strain Q 364 were obtained from Dr P. van der Elsen (Free University of Amsterdam, The Netherlands) and propagated as described (Loenen and Brammar, 1980).

E. coli K12 strains BHB 2600, BHB 2688 and BHB 2690 (Hohn, 1979) were obtained from Dr M. van Montagu (University of Gent, Belgium), while E. coli K12 strain JM 101.7118 and the M13 derivatives M13 mp 8, 9, 18 and 19 were obtained from Bethesda Research Laboratories Inc. (Gaithersburg, MD, U.S.A.).

b) Enzymes

All enzymes used were obtained from Amersham International PLC, Amersham, U.K., except alpha-helicase which was obtained from Pharm Industrie, Clichy, France. Enzyme incubations were performed according to the instructions of the manufacturer. ATP:RNA adenyl transferase was purified as described by Edens et al. (1982).

c) Other materials

[35S] methionine, [alpha-35S] dATP, [alpha-32P] dNTP's, [alpha-32P] ATP and [gamma-32P] ATP were obtained from Amersham International PLC, Amersham, U.K.

Nitrobenzyloxy-methyl (NBM) paper was obtained from Schleicher and Schuell, and converted into the diazo form (DBM) according to the instructions of the manufacturer.

Nitrocellulose filters (type HATF) were obtained from Millipore.

RNA isolation, fractionation and analysis

Hansenula polymorpha cells were grown to mid-exponential phase, either in the presence of methanol or ethanol. The cells were disrupted by forcing them repeatedly through a French Press at 16 000 psi, in a buffer containing 10 mM Tris-HCl pH 8, 5 mM $MgCl_2$, 1% NaCl, 6% para-aminosalicylic acid, 1% sodium dodecylsulphate(SDS) and 5% phenol. The purification of polyadenylated RNA was subsequently performed, as described previously (Edens et al., 1982). One gram cells yielded four mg total RNA and 0.1 mg polyadenylated RNA. Five microgram samples of total RNA or polyadenylated RNA were radioactively labelled at their 3'-ends with ATP:RNA adenyl transferase and [alpha-$^{32}$P] ATP, and subsequently separated on a 2.5% polyacrylamide gel containing 7 M urea (Edens et al., 1982). For the preparative isolation of a specific mRNA fraction, 40 micrograms polyadenylated RNA was mixed with four micrograms of labelled polyadenylated RNA and separated on the denaturing polyacrylamide gel. The radioactive 2.4 kb RNA class was eluted from slices of the gel and freed from impurities by centrifugation through a 5-30% glycerol gradient in 100 mM NaCl, 10 mM Tris-HCl pH 7.5, 1 mM EDTA and 0.1% SDS for 15 h at 24 000 rev./min. in a Beckmann centrifuge using an SW 60 rotor at 20°C. The radioactive fractions were pooled and precipitated with ethanol. Polyadenylated RNA was translated in vitro in a rabbit reticulocyte lysate according to Pelham and Jackson (1976), using [$^{35}$S] methionine as a precursor. The translation products were immuno-precipitated with MOX antiserum as described by Valerio et al. (1983).

cDNA synthesis

One third of the RNA fraction, isolated from the polyacrylamide gel, was used to procure a radioactive cDNA with reverse transcriptase (Edens et al., 1982). Using [alpha-$^{32}$P] dATP and [alpha-$^{32}$P] dCTP of a high specific activity (more than 3000 Ci/mM), 20 000 cpm of high molecular weight cDNA was formed during 1 h at 42°C in the presence of human placental ribonuclease inhibitor.

DNA isolation

Ten g of Hansenula polymorpha cells were washed with 1 M sorbitol and resuspended in 100 ml 1.2 M sorbitol, 10 mM EDTA and 100 mM citric acid pH 5.8, to which 100 microliter beta-mercapto-ethanol was added. Cells were spheroplasted by incubation with 500 mg alpha-helicase for 1 h at 30°C. Spheroplasts were collected by centrifugation at 4000 rev./min. in a Sorvall GSA rotor, resuspended in 40 ml 20 mM Tris-HCl pH 8, 50 mM EDTA and lysed by adding 2.5% SDS. Incompletely lysed cells were pelleted for 30 min. at 20 000 rev./min. in a Sorvall SS34 rotor and DNA was isolated from the viscous supernatant by centrifugation using a CsCl-ethidium bromide density gradient at 35 000 rev./min. for 48 h in a Beckmann centrifuge using a 60 Ti rotor. 2 mg of DNA was isolated with a mean length of 30 kb.

Preparation of a clone bank in phage lambda L47.1

150 microgram Hansenula polymorpha DNA was partially digested with Sau3Al and sedimented through a 10-40% sucrose gradient in 1 M NaCl, 20 mM Tris-HCl pH 8 and 5 mM EDTA for 22 h at 23 000 rev./min. in an SW 25 rotor. The gradient was fractionated and samples of the fractions were separated on a 0.6% agarose gel in TBE buffer (89 mM Tris, 89 mM Boric acid, 2.5 mM EDTA).

Fractions that contained DNA of 5-20 kb were pooled and the DNA was precipitated with ethanol. Phage lambda L47.1 was grown, and its DNA was isolated as described by Ledeboer et al. (1984). The DNA was digested with BamHl and arms were isolated by centrifugation through a potassium acetate gradient as described by Maniatis et al. (1982). Two microgram phage lambda DNA arms and 0.5 /ug Sau3Al digested Hansenula polymorpha DNA thus obtained were ligated and packaged in vitro using a protocol from Hohn (1979). Phages were plated on E. coli strain Q 364 to a plaque density of 20,000 pfu per 14 cm Petri dish. Plaques were blotted onto a nitro-cellulose filter (Benton and Davis, 1977) and the blot was hybridized with the radioactive cDNA probe isolated as described above. Hybridization conditions were the same as described by Ledeboer et al. (1984) and hybridizing plaques were detected by autoradiography.

Isolation and partial amino acid sequence analysis of alcohol oxidase (MOX)

Hansenula polymorpha cells grown on methanol were disintegrated by ultrasonification and the cell debris was removed by centrifugation. The MOX-containing protein fraction was isolated by (NH$_4$)$_2$SO$_4$ precipitation . (40-60% saturation). After dialysis of the precipitate, MOX was separated from catalase and other proteins by ion-exchange chromatography (DEAE-Sepharose) and gel filtration (Sephacryl S-400). Antibodies against MOX were raised in rabbits by conventional methods using complete and incomplete Freund's adjuvants (Difco Lab, Detroit, U.S.A.). Sequence analysis of alcohol oxidase treated with performic acid was performed on a Beckman sequenator. Identification of the residues was done with HPLC. The amino acid composition was determined on a Chromaspek analyser (Rank Hilger, U.K.), using standard procedures and staining by ninhydrine. The carboxy terminal amino acid was determined as described by Ambler (1972).

Chemical synthesis of deoxyoligonucleotides

Deoxyoligonucleotides were synthesized on a Biosearch SAM I gene machine, using the phosphite technique (Matteucci and Caruthers, 1981). They were purified on 16% or 20% polyacrylamide gels in TBE.

Hybridization with deoxyoligonucleotide probes

The deoxyoligonucleotides were radioactively labelled with $T_4$-polynucleotide kinase and [gamma-$^{32}$P] ATP. The DNA of the MOX clones obtained was digested with different restriction enzymes, separated on 1% agarose gel and blotted onto DBM paper. Hybridizations were performed as described by Wallace et al. (1981).

DNA sequence analysis

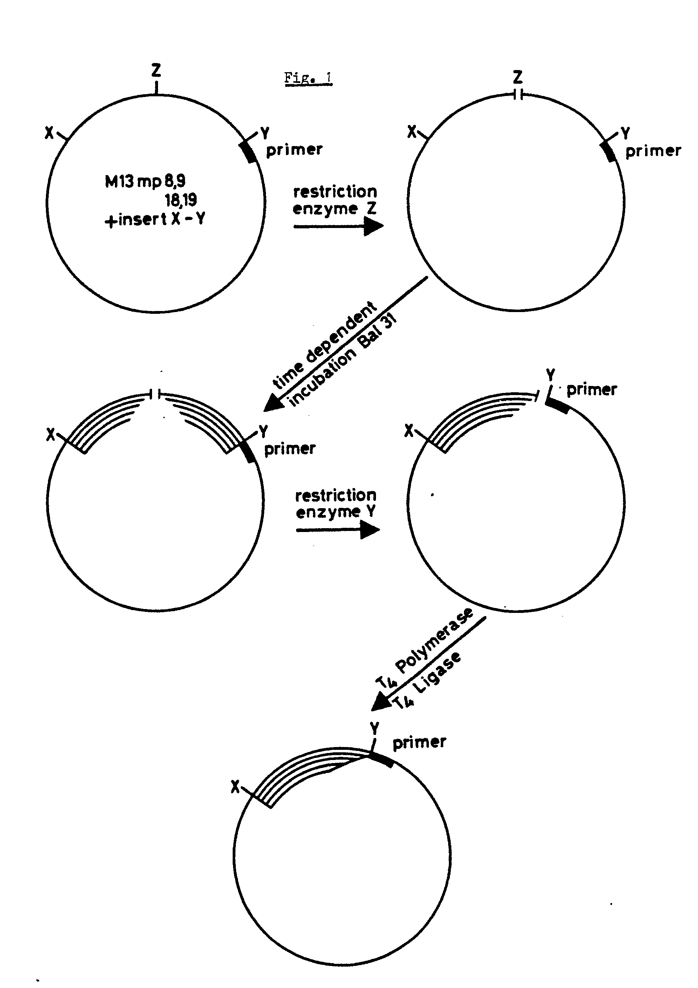

From clone 4 (see Example 1) containing the complete MOX gene, several subclones were made in phage M13mp-8, -9 or M13mp-18, -19 derivatives by standard techniques. Small subclones (less than 0.5 kb), cloned in two orientations, were sequenced directly from both sides. From the larger subclones, also cloned in two orientations, sequence data were obtained by an exonuclease Bal31 digestion strategy (see Fig. 1). For each of both cloned orientations the RF M13 DNA is digested with a restriction enzyme that preferably cleaves only in the middle of the insert. Subsequently, both orientations of the clones were cut at this unique site, and digested with exonuclease Bal31 at different time intervals. Incubation times and conditions were chosen such that about 100-150 nucleotides were eliminated during each time interval. Each fraction was digested subsequently with the restriction enzyme, recognizing the restriction site situated near the position at which the sequence reaction is primed in the M13 derivatives. Ends were made blunt end by incubation with $T_4$-polymerase and all dNTP's, and the whole mix was ligated under diluted conditions, thereby favouring the formation of internal RF molecules. The whole ligation mix was used to transform to E. coli strain JM 101-7118. From each time interval several plaques were picked up and sequenced using recently described modifications of the Sanger sequencing protocol (Biggin et al., 1983).

The isolation of auxotrophic mutants

LEU-1 (CBS N° 7171) is an auxotrophic derivative of H. polymorpha strain NCYC 495 lacking $\beta$-isopropylmalate dehydrogenase activity. The isolation of this mutant has been described by Gleeson et al. (1984).

LR9 (CBS N° 7172) is an auxotrophic derivative of H. polymorpha ATCC 34438, lacking orotidine 5'-decarboxylase activity.

For the isolation, all procedures were carried out at 30°C instead of 37°C, which is the optimal temperature for growth of this yeast. Yeast cells were mutagenized with 3% ethylmethanesulphonate for 2 hr (Fink, 1970). The reaction was stopped with 6% sodium thiosulphate (final concentration) and the solution was incubated for another 10 min. Mutagenized cells were then washed once with $H_2O$ and incubated for 2 days on YEPD or YNB supplemented with uracil for segregation and enrichment of uracil-auxotrophs followed by a 15 hr cultivation on MN without nitrogen source. Finally a nystatin enrichment was employed for 12 hr on NM with a concentration of 10 /ug antibiotic per ml. The treated cells were plated on YNB plates containing 200 /ug uracil per ml and 0.8 mg 5-fluoroorotic acid (Boeke et al., 1984). Usually $10^6$ cells were plated on a single plate. Resistant colonies were picked after 3 days of incubation, replica plated twice on YNB plates to establish the auxotrophy. From the auxotrophic mutants ura$^-$ cells were isolated. Alternatively, 1.5 x $10^6$ yeast cells were incubated in one ml of YNB liquid medium supplemented with 200 /ug of uracil and 0.8 mg of 5-fluoroorotic acid. After incubation of 2 days, the treated cells were plated on YNB containing uracil, replica-plated twice on YNB and analysed as described above.

Such resistant mutants have been shown to be uracil auxotrophs affected at the URA3 or the URA5 locus in S. cerevisiae (F. Lacroute, personal communication). Of about 600 resistant colonies of H. polymorpha tested, 52 exhibited a uracil phenotype. Since URA3 and URA5 mutations in S. cerevisiae lack orotidine 5'-decarboxylase and orotidine 5'-phosphate pyrophosphorylase, respectively (Jones and Fink, 1982), the obtained uracil auxotrophs of H. polymorpha were tested for both enzymatic activities (Lieberman et al., 1955). Mutants affected in either of the two enzymes were found (Table I). They have been

6

designated odcl and oppl mutants, respectively. The odcl mutants exhibit adequate low reversion frequencies (Table II) and thus are suitable for transformation purposes by complementation.

Isolation of autonomous replication sequences (HARS) from H. polymorpha

Chromosomal DNA from H. polymorpha was partially digested either with SalI or BamHI and ligated into the single SalI and BamHI site of the integrative plasmid YIp5, respectively. The ligation mixture was used to transform E. coli 490 to ampicillin resistance. YIp5 is an integrative plasmid containing the URA3 gene as a selective marker (Stinchcomb et al., 1980).

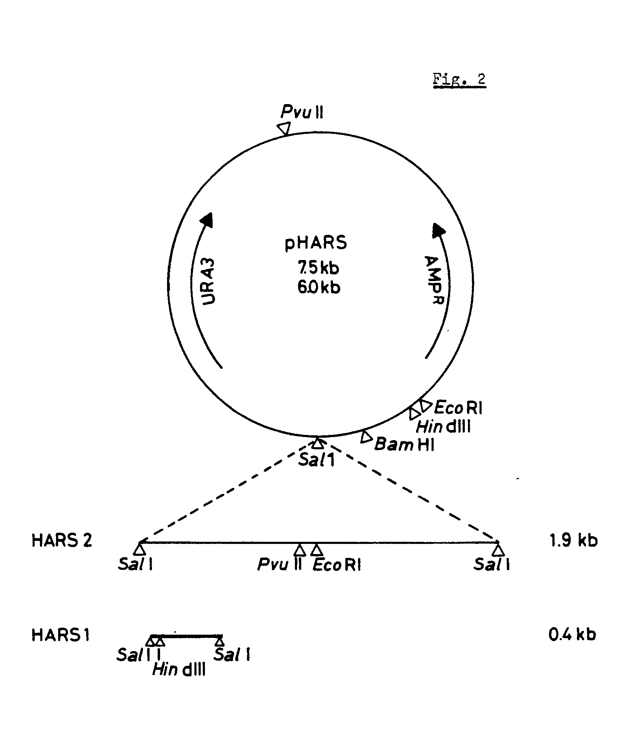

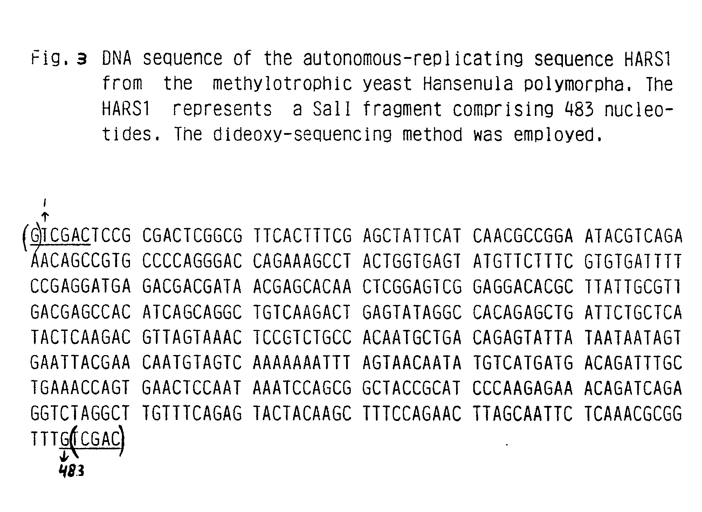

The plasmid pool of H. polymorpha SalI clones was used to transform H. polymorpha mutant LR9. A total of 27 transformants was obtained being also positive in the $\beta$-lactamase assay. From all of them, plasmids could be recovered after transformation of E. coli 490 with yeast minilysates. Restriction analysis of the plasmids revealed that most of the inserts show the same pattern. The two different plasmids, pHARS1 and pHARS2, containing inserts of 0.4 and 1.6 kb respectively, were used for further studies (Fig. 2). Both plasmids transform H. polymorpha mutant LR9 with a frequency of about 500-1,500 transformants per /ug of DNA using the transformation procedure of intact cells treated with polyethyleneglycol. Southern analysis of the H. polymorpha transformants after retransformation with pHARS1 and pHARS2 recovered from E. coli plasmid preparations shows the expected plasmid bands and thus excludes integration of the URA3 gene as a cause of the uracil protrophy. Therefore, we conclude that the HARS sequences like ARS1 (Stinchcomb et al., 1982) allow autonomous replication in H. polymorpha. Neither HARS1 nor HARS2 enabled autonomous replication in S. cerevisiae. HARS1 was sequenced completely as shown in Fig. 3.

Estimation of plasmid copy number in H. polymorpha transformants

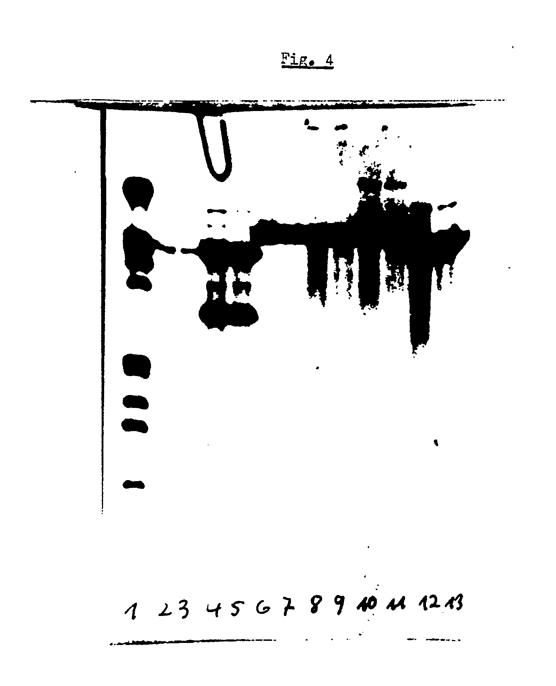

The copy number of plasmids conferring autonomous replication in H. polymorpha either by ARS sequences or by HARS sequences was estimated by Southern blot analysis (Fig. 4). For comparison, plasmid YRP17 in S. cerevisiae (Fig. 4, lanes 6, 7), which has a copy number of 5-10 per cell (Struhl et al., 1979) and the high copy number plasmid pRB58 in S. cerevisiae (Fig. 4, lanes 4, 5) with about 30-50 copies per cell were used. YRP17 is a URA3-containing yeast plasmid, bearing an ARS sequence (Stinchcomb et al., 1982), while pRB58 is a 2 /um derivative containing the URA3 gene (Carlson and Botstein, 1982). A Kluyveromyces lactis transformant carrying 2 integrated copies of pBR pBR322 was used as a control (Fig. 4, lanes 2, 3). The intensity of staining in the autoradiogram reveals that the plasmid YRP17 in H. polymorpha has practically the same copy number as in S. cerevisiae, whereas plasmids pHARS-1 and pHARS-2 show a copy number which is in the range of about 30-40 copies per cell like pRB58 in S. cerevisiae. This proves once more the autonomously replicating character of the HARS sequence.

Transformation procedures

Several protocols were used.

a) H. polymorpha strain LEU-1 was transformed using a procedure adapted from Beggs (1978). The strain was grown at 37° C with vigorous aeration in 500 ml YEPD liquid medium up to an $OD_{600}$ of 0.5. The cells were harvested, washed with 20 ml distilled water and resuspended in 20 ml 1.2 M sorbitol, 25 mM EDTA pH 8.0, 150 mM DTT and incubated at room temperature for 15 minutes. Cells were collected by centrifugation and taken up in 20 ml 1.2 M sorbitol, 0.01 M EDTA, 0.1 M sodium citrate pH 5.8 and 2% v/v beta-glucuronidase solution (Sigma 1500000 units/ml) and incubated at 37° C for 105 minutes. After 1 hr, the final concentration of beta-glucuronidase was brought to 4% v/v. For transformation, 3 ml aliquots of the protoplasts were added to 7 ml of ice cold 1.2 M sorbitol, 10 mM Tris-HCl pH 7. Protoplasts were harvested by centrifugation at 2000 rpm for 5 minutes and washed three times in ice cold sorbitol buffer. Washed cells were resuspended in 0.2 ml 1.2 M sorbitol, 10 mM $CaCl_2$, 10 mM Tris-HCl pH 7 on ice. 2 /ug of YEP13 DNA - an autonomous replicating S. cerevisiae plasmid consisting of the LEU2 gene of S. cerevisiae and the 2 micron-ori (Broach et al., 1979) - were added to 100 ml of cells and incubated at room temperature. 0.5 ml of a solution of 20% PEG 4000 in 10 mM $CaCl_2$, 10 mM Tris-HCl pH 7.5 was added and the whole mixture was incubated for 2 minutes at room temperature. Cells were collected by brief (5 sec.) centrifugation in an MSG microfuge set at high speed and resuspended in 0.1 ml YEPD 1.2 M sorbitol pH 7.0, and incubated for 15 minutes at room temperature. The cells were plated directly by surface spreading on plates containing 2% Difco agar, 2% glucose, 0.67% Difco yeast nitrogen base and 20 mg/l of each of L-adenine Hemisulphate, methionine, uracil, histidine, tryptophan, lysine and 1.2 M sorbitol. Leu[+] transformants appear after 5 days incubation at 37° C with a frequency of 50 colonies/ug DNA, while no transformants appear if no DNA is added.

b) Alternatively, H. polymorpha LEU-1 was transformed with YEP13, using a procedure adapted from Das et al. (1984). Exponentially growing cells were grown up to an $OD_{600}$ of 0.4, washed in TE buffer (50 mM Tris-HCl pH 8.0, 1 mM EDTA) and resuspended in 20 ml TE buffer. 0.5 ml cells were incubated with 0.5 ml 0.2 M LiCl for 1 hr at 30°C. To 100 ml of these cells 4 ug YEP13 in 20 ml TE buffer was added and the sample was incubated for a further 30 minutes at 30°C. An equal volume of 70% v/v PEG 4000 was added and the mixture was incubated for 1 hr at 30°C, followed by 5 min. at 42°C. After addition of 1 ml $H_2O$, cells were collected by a brief centrifugation as described under a), washed twice with $H_2O$ and resuspended in 0.1 ml YEPD 1.2 M sorbitol and incubated for 15 minutes at room temperature. Cells were plated as described. Leu[+] transformants appear with a frequency of 30/ug DNA.

c) The H. polymorpha URA mutant LR9 was transformed with YRP17, a plasmid containing the URA3 gene of S. cerevisiae as a selective marker and an autonomously replicating sequence (ARS) for S. cerevisiae (Stinchomb et al, 1982). Using the protoplast method described by Beggs (1978), 2-5 transformants/ug DNA were obtained. This number was enlarged, using the $LiSO_4$ method of Ito et al. (1983), up to 15-20 transformants per ug of DNA. However, the best procedure was the procedure described by Klebe et al. (1983), using intact cells treated with PEG 4000. Up to 300 transformants were obtained per ug DNA. The $LiSO_4$ procedure, as well as the Klebe procedure, was performed at 37°C.

Transformation of H. polymorpha based on autonomous replication of the vector was indicated by two characteristics: (1) the instability of the uracil[+] pheno-type. After growth of transformants on YEPD for ten generations, more than 99% had lost the ability to grow on selective medium (Table II). (2) Autonomous replication was further ascertained by transforming E. coli cells with yeast minilysates and retransformation of H. polymorpha. Subsequent Southern analysis showed the presence of the expected plasmid.

H. polymorpha LR9 could not be transformed with pRB58, or with pHH85, constructed by insertion of the whole 2 micron circle DNA (Hollenberg, 1982) into the PstI site of the ampicillin gene of plasmid YIP5. YIP5, containing the DNA sequence of HARS1 or HARS2, was transferred to H. polymorpha LR9 using the Klebe protocol with a frequency of 500-1500 transformants per ug of DNA. Thus, transformation frequency is 2-5 times higher than described above, using the heterologous ARS 1 in YRP17 of S. cerevisiae. Similarly, the stability of the HARS plasmid in transformants is slightly higher than the ARS 1 plasmid (Table II).

## Transformation of H. polymorpha by integration of the URA3 gene from S. cerevisiae

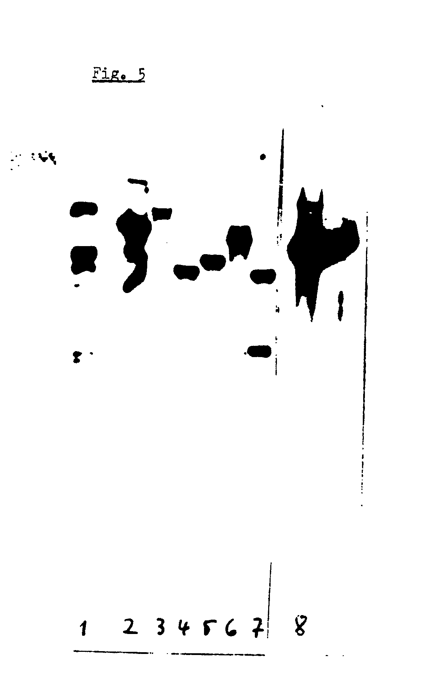

The URA3 gene of S. cerevisiae shows no homology to the ODC gene in H. polymorpha, as revealed by Southern hybridisation of nick-translated YIp5 plasmid DNA to chromosomal DNA of H. polymorpha. Therefore, low-frequency integration of the URA3 gene at random sites of the H. polymorpha genome had to be anticipated. Transformation of mutant LR9 with the integrative vector YIp5 resulted in 30-40 colonies per ug of DNA on YNB plates using the polyethyleneglycol method, whereas no transformants were obtained in the control experiment using YIp5 for transformation of S. cerevisiae mutant YNN27. Analysis of 38 transformants revealed 4 stable integrants after growth on non-selective medium. The integration event was further demonstrated by Southern analysis (Fig. 5).

A second procedure for generating integration of the URA3 gene into chromosomal DNA of H. polymorpha was performed by enrichment of stable Ura[+] transformants from transformants carrying plasmid pHARS1. Transformants were grown in liquid YEPD up to a density of $10^9$ cells per ml. An aliquot containing $5 \times 10^6$ cells was used to inoculate 100 ml of fresh medium and was grown up to a cell density of $10^9$ per ml. The procedure was repeated until about 100 generations had been reached. Since the reversion rate of mutant LR9 is $2 \times 10^{-9}$ and the frequency of plasmid loss per 10 generations is 97% in pHARS1 transformants, the predominant part of the Ura[+] cells after 100 generations should be integrants. The Ura[+] colonies tested were all shown to maintain a stable Ura[+] phenotype indicating an integration of the URA3 gene. This was further verified by Southern blot analysis. In addition, these data indicate that the integration frequency is $5 \times 10^{-6}$.

Example 1
CLONING OF THE GENE FOR ALCOHOL OXIDASE (MOX) FROM HANSENULA POLYMORPHA
Characterization of polyadenylated RNA

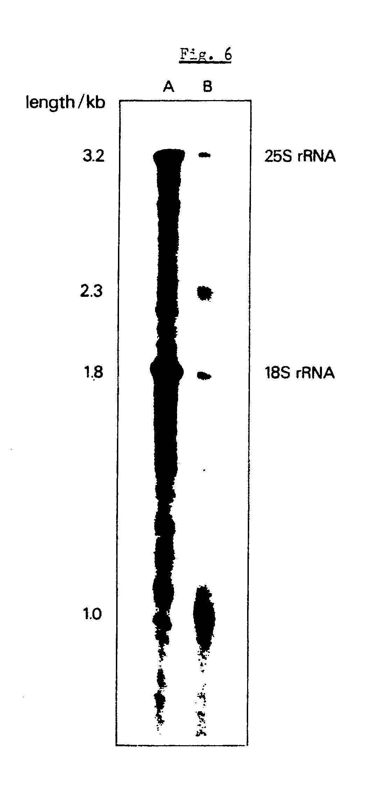

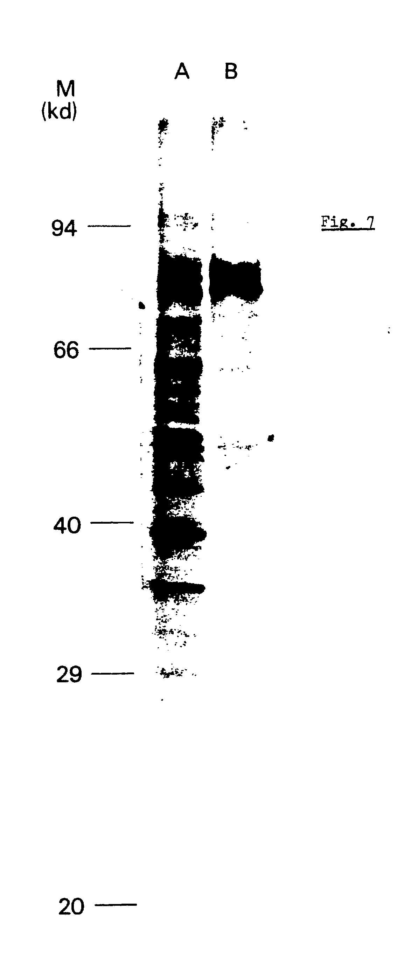

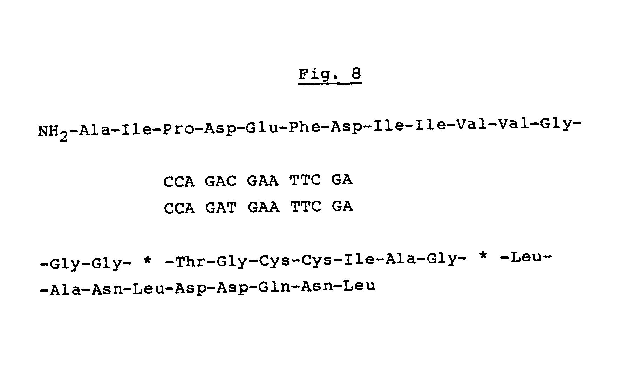

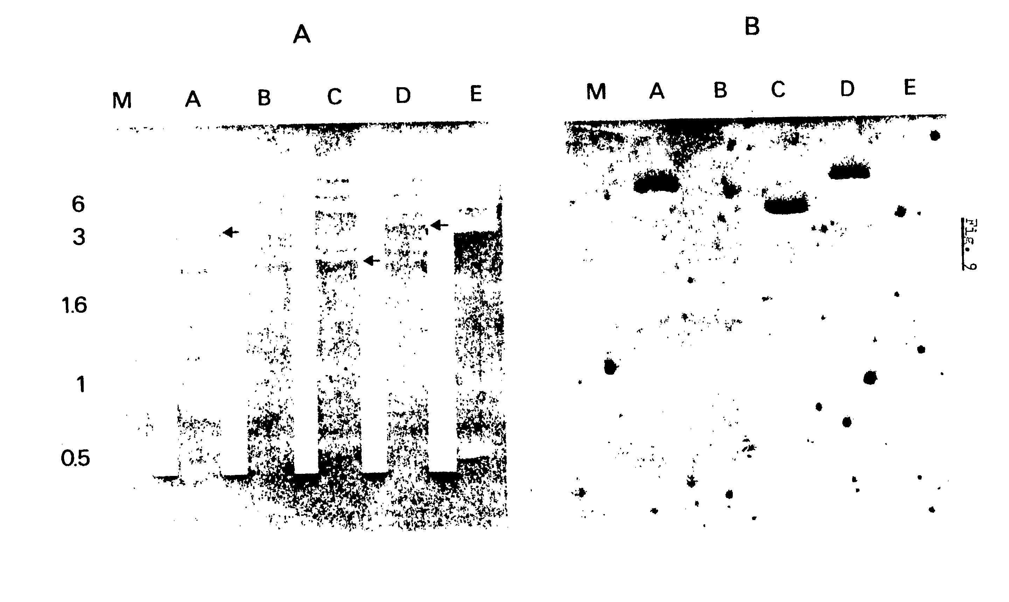

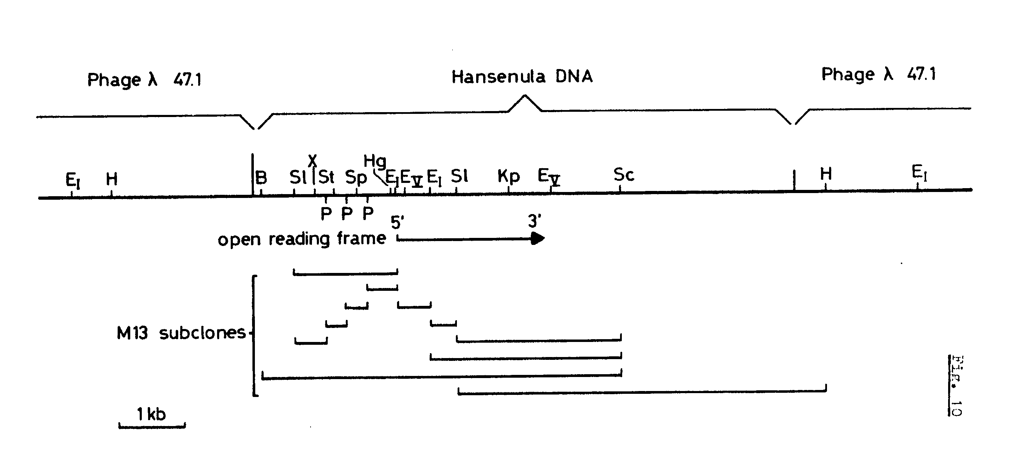

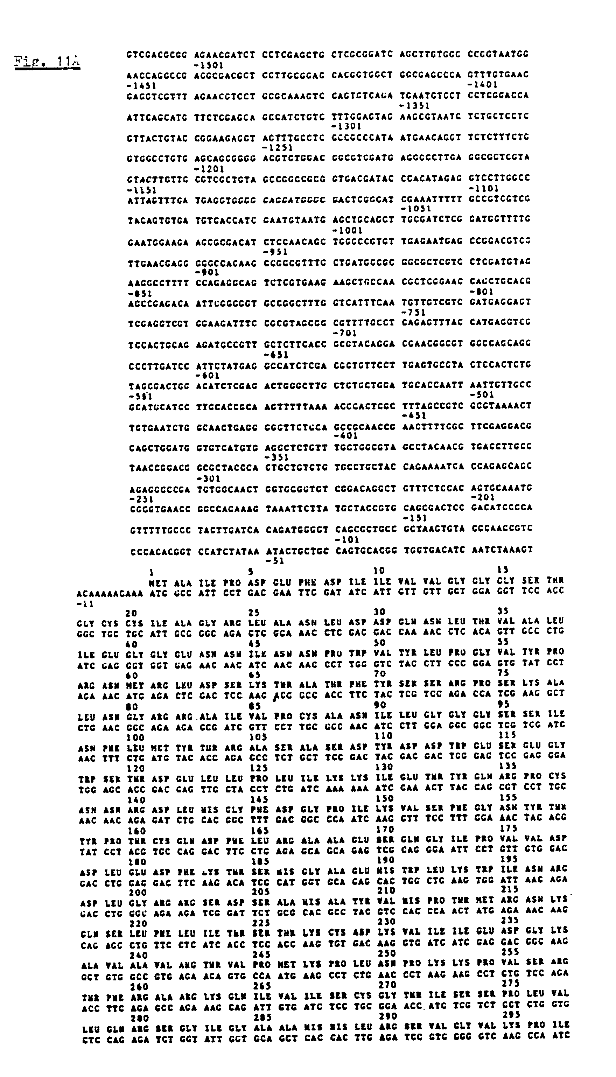

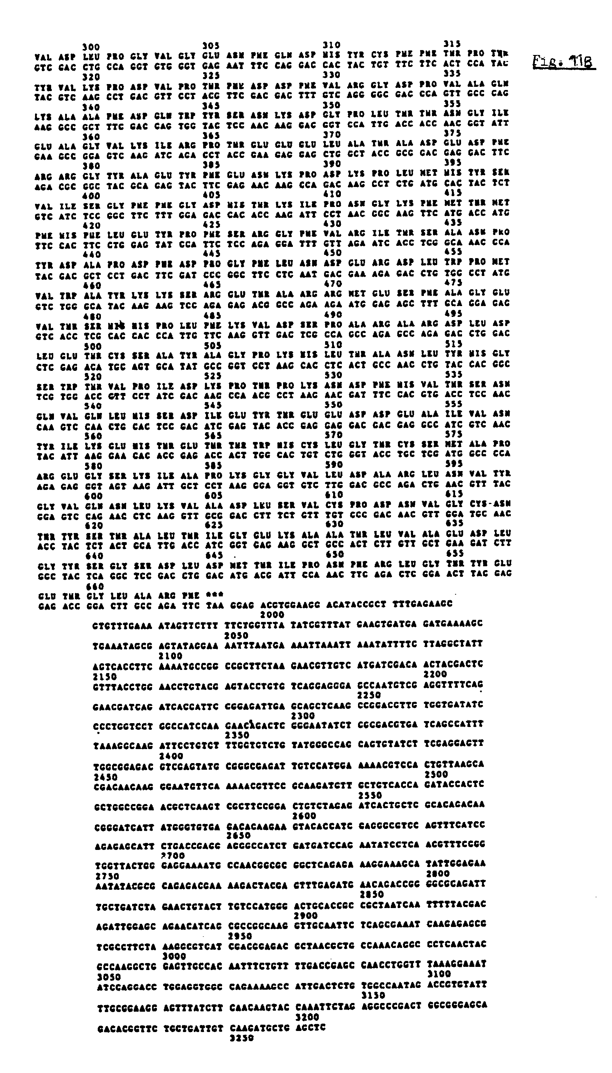

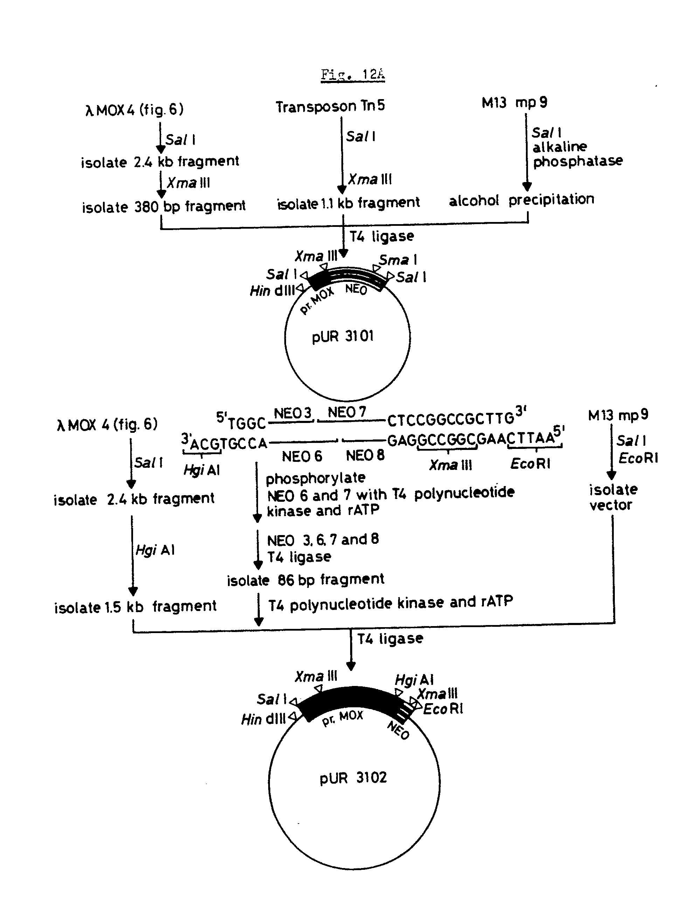

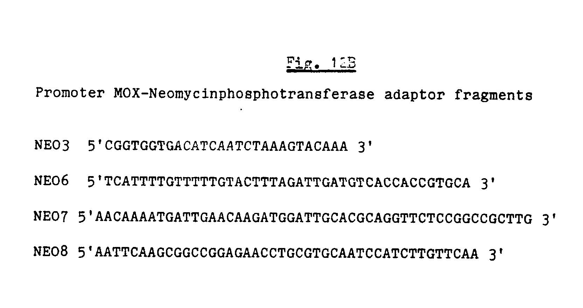

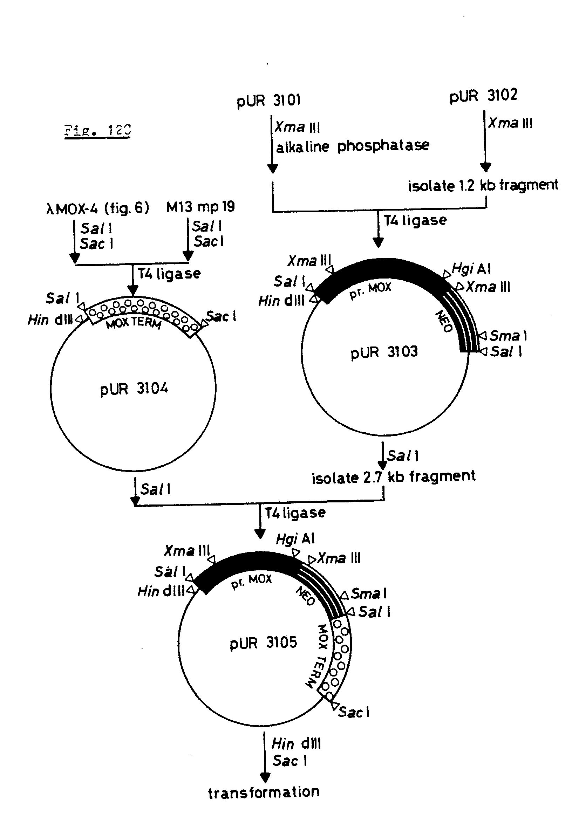

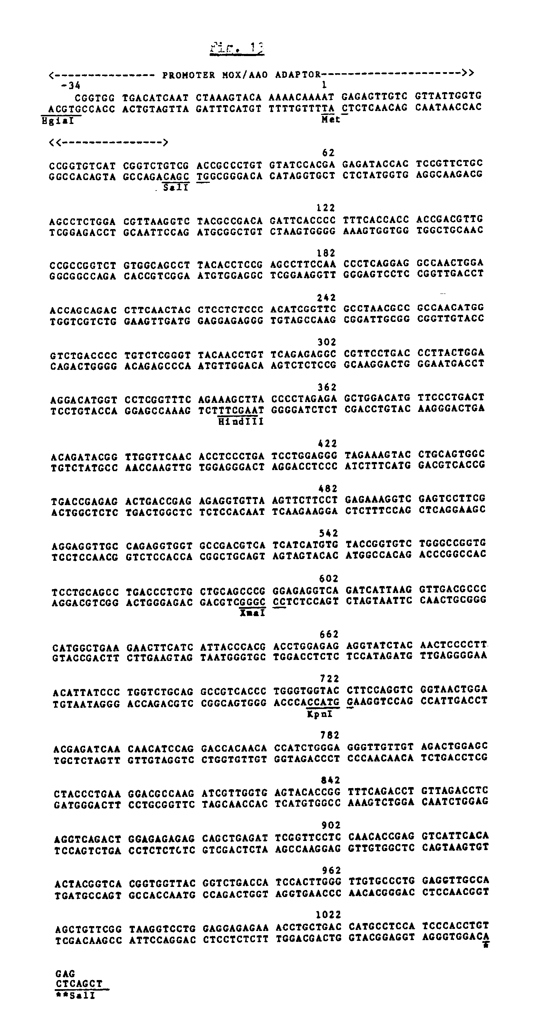

Total RNA and polyadenylated RNA, isolated from cells grown on methanol, were labelled at their 3'-termini with ATP:RNA adenyl transferase, and separated on a denaturing polyacrylamide gel (Fig. 6). Apart from the rRNA bands, two classes of RNA appear in the poly-adenylated RNA lane, respectively 1 kb and 2.3 kb in length. Since these RNA classes are not found in polyadenylated RNA of ethanol-grown cells (result not shown), they obviously are transcripts of genes derepressed by growth on methanol. The 2.3 kb class can code for a protein of 700 to 800 amino acids, depending on the length of the non-translated sequences. Likewise, the 1 kb class codes for a protein of 250-300 amino acids. Enzymes that are derepressed by growth on methanol and are 700 to 800 amino acids long, most likely are MOX (Kato et al., 1976; Roa and Blobel, 1983) and DHAS (Bystrykh et al., 1981). Derepressed enzymes in the 250 to 300 amino acid range are probably formaldehyde and formate dehydrogenase (Schütte et al., 1976). The polyadenylated RNA was characterized further by in vitro translation in a reticulocyte cell free translation system. Two microliters of the polyadenylated RNA directed protein mixture were separated directly on a 10% SDS polyacrylamide gel, while the remaining 18 microliters were subjected to immuno-precipitation with antiserum against MOX (Fig. 7). Six strong bands dominate in the total protein mixture, having molecular weights of respectively 78kd, 74kd, 58kd, 42kd, 39kd and 36kd. Essentially the same molecular weights were found by Roa and Blobel (1983) in a total cell extract from methanol-grown H. polymorpha cells.

The 74kd protein can tentatively be assigned to the monomer of MOX, the 58kd protein to the monomer of catalase and the 39kd and 36kd proteins to the monomers of formaldehyde dehydrogenase and formate dehydrogenase, respectively. The 78kd polypeptide possibly is DHAS, while the 42kd polypeptide remains unidentified. After immuno-precipitation, both high molecular weight proteins react with the MOX antiserum.

## Cloning of the gene for MOX

Although the 2.3 kb mRNA class induced by growth on methanol obviously codes for at least 2 polypeptides, it seemed a good candidate for screening a Hansenula polymorpha clone bank by hybridization. The 5-20 kb fraction of partially Sau3AI digested H. polymorpha DNA was cloned in phage lambda L47.1.

Per microgram insert DNA, 300 000 plaques were obtained while the background was less than 1:1000. Two Benton Davis blots, containing about 20 000 plaques each, were hybridized with 15 000 cpm of the mRNA-derived cDNA probe. After 3 weeks of autoradiography about 40-50 hybridizing plaques could be detected. All plaques were picked up and five were purified further by plating at lower density and by a second hybridization with the cDNA probe. From four, single hybridizing plaques (1, 3, 4, 5) DNA was isolated. The insert length varied from 8 to 13 kb.

### Hybridization selection using organic-synthetic DNA probes

The sequence of 30 amino acids at the amino terminus of purified MOX was determined (Fig. 8).

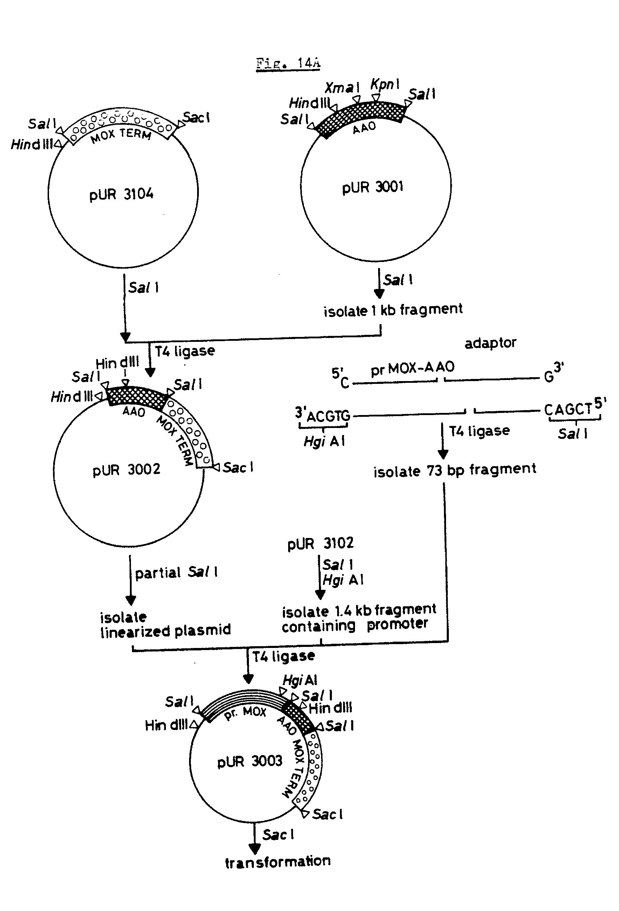

Using the most abundant codon use for the yeast S. cerevisiae, a sequence of 14 bases could be derived from part of this protein sequence, with only one ambiguity. Both probes, indicated in Fig. 4, were synthesised. In both probes an EcoRI site is present. DBM blots were made from the DNA of the MOX clones digested with the restriction enzymes BamHI, EcoRI/HindIII, HindIII/SalI and PstI/SalI and separated on 1.5% agarose gels. After hybridization of the blot with a mixture of both radioactively labelled probes, the clones 1, 4 and 5 hybridize, while clone 3 does not, as shown for the HindIII/SalI blot in Fig. 9. However, the probes did not hybridize with the EcoRI/HindIII digested DNA of these clones (result not shown). Since an EcoRI site is present in the probes, the hybridizing DNA in the clones probably is cut by this enzyme too. Consequently the hybridization overlap has become too small to allow the formation of stable hybrids.

### Restriction map and sequence analysis

By comparing restriction enzyme digests and by cross-hybridization experiments it was concluded that clones 1, 4 and 5 covered identical stretches of DNA.

In order to definitely establish the nature of this stretch of cloned DNA the insert of clone 4 was analyzed in detail. Hybridization with the amino terminal probe showed that the complete MOX gene (ca. 2 kb) was present, including 2 kb sequences upstream and 3.5 kb downstream (Fig. 10).

DNA sequence analysis of the smallest EcoRI fragment revealed the nucleotide sequence corresponding to the amino terminus of MOX as was determined by amino acid sequence analysis.

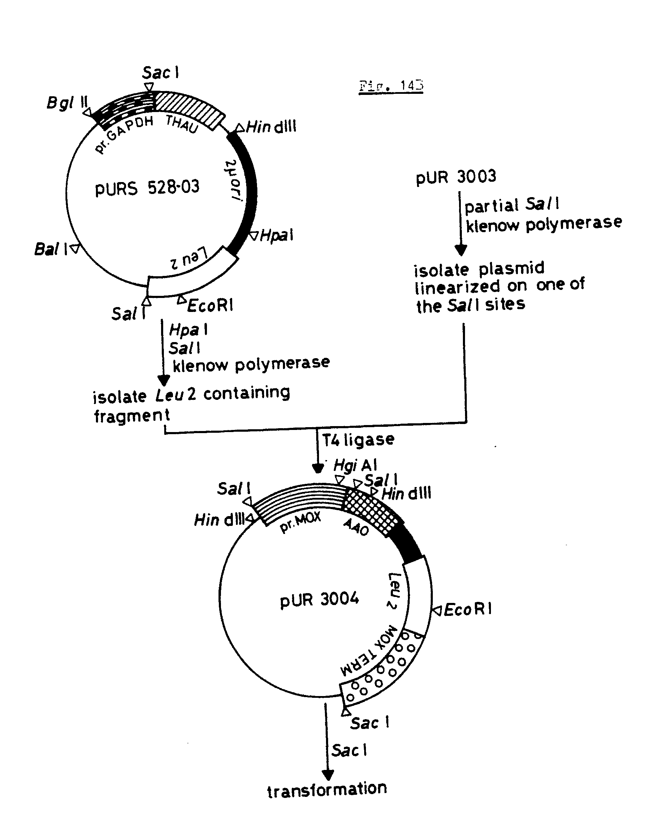

For sequence analysis, several fragments were subcloned in M13mp8/M13mp9 or M13mp18/M13mp19 respectively in two orientations, as indicated in Fig. 10. Clones that were smaller than 0.5 kb were sequenced directly from both sides. The larger clones were cut at the unique restriction sites situated in the middle of the cloned fragment, to allow generation of exonuclease Bal31 digested subclones as described in materials and methods. Using specific oligonucleotide primers, sequences around the restriction sites used for subcloning and sequences that did not allow an unequivocal sequence determination were sequenced once more, using the 5.5 kb BamHI/SacI subclone that covers the whole sequence. The complete nucleotide sequence is given in Fig. 11A and 11B.

The sequence contains an open reading frame of 2046 nucleotides that can code for a protein of 664 amino acids. The last codon of the open reading frame codes for Phe, which is in agreement with the carboxy terminus of purified MOX. The amino acid composition derived from the DNA sequence encoding this protein, and the amino acid composition of purified MOX are virtually identical (Table III). The only important differences involve the serine and threonine residues, which are notoriously difficult to determine.

The calculated molecular weight of the protein is 74 050 Dalton, which agrees well with the molecular weight of 74 kd of MOX, as determined on polyacrylamide/SDS gels.

Codon usage

In Table IV the codon usage for MOX is given. A bias towards the use of a selective number of codons is evident.

Example 2
CONSTRUCTION OF A PLASMID, pUR 3105, BY WHICH THE GENE CODING FOR NEOMYCIN PHOSPHOTRANSFERASE, THAT CONFERS RESISTANCE AGAINST THE ANTIBIOTIC G 418, IS INTEGRATED INTO THE CHROMOSOMAL MOX GENE UNDER REGIE OF THE MOX REGULON.

H. polymorpha cells, transformed with either the plasmids YEP 13, YRP 17, pHARS 1 or pHARS 2, were unstable and lost their leu$^+$ or ura$^+$ phenotype already after 10 generations upon growth under non-selective conditions. In order to obtain stable transformants and to test the MOX promoter, a plasmid pUR 3105 is constructed in which the neomycin phosphotransferase gene (NEO$^R$) is brought under direct control of the MOX regulon. The construction is made in such a way that the first ATG of the NEO$^R$ gene is coupled to 1.5 kb of the MOX regulon. The cloning of such a large regulon fragment is necessary as shorter fragments, that do not contain the -1000 region of the regulon, were less efficient.

The NEO$^R$ gene was isolated as a 1.1 kb XmaIII-SalI fragment from the transposon Tn5, situated from 35 bp downstream of the first ATG up to 240 bp downstream of the TGA translational stop codon. To avoid a complex ligation mixture, first pUR 3101 is constructed (Fig. 12A), which is a fusion of the far upstream SalI-XmaIII (position -1510 to position -1128) fragment of the MOX regulon, and the NEO$^R$ gene, subcloned on M13mp9. Another plasmid is constructed, pUR 3102, in which the 1.5 kb SalI-HgiAI fragment of the MOX gene, that covers nearly the whole MOX regulon, is ligated to a MOX-NEO$^R$ adapter (Fig. 12B) sequence and cloned in M13-mp9. The 1.2 kb XmaIII fragment of this plasmid is cloned into the XmaIII site of pUR 3101, resulting in pUR 3103, which is the exact fusion of the MOX regulon and the NEO$^R$ gene (Fig. 12C). The orientation is checked by cleavage with HgiAI and SalI. From the lambda-MOX-4 clone, a SalI-SacI fragment is subcloned that reaches from the SalI site, still in the structural MOX gene (position 894), up to the SacI site, far downstream of the structural MOX gene (position 3259) (see Fig. 10). This M13mp19 subclone is called pUR 3104. The plasmid pUR 3105 is obtained by the direct ligation of the 2.7 kb SalI fragment from pUR 3103 into the SalI site of pUR 3104. The orientation is tested by cleavage with SmaI and SacI.

After cleavage of this plasmid with HindIII and SacI and the transformation of this cleaved plasmid to H. polymorpha, G 418-resistant colonies are found that do not lose their resistance upon growth under non-selective conditions for a large number of generations.

Example 3
THE CONSTRUCTION OF pUR 3004, BY WHICH THE GENE CODING FOR D-AMINO ACID OXIDASE IS TRANSFERRED TO THE CHROMOSOME OF H. POLYMORPHA UNDER REGIE OF THE MOX-REGULON

D-amino acid oxidase (AAO) is an example of an oxido-reductase for the production of which the methylotrophic H. polymorpha is extremely suited. It might be expected that the enzyme, being an oxidase like MOX, is translocated to the peroxisomes of the yeast that are induced during growth on methanol or a mixture of methanol and a fermentable sugar as carbon source and D-amino acids as the sole nitrogen source. Under these conditions the cell will be protected from the H$_2$O$_2$ produced. Alternatively, AAO can be produced without the production of H$_2$O$_2$, when it is placed under regie of the MOX- or DAS-regulon. The AAO production will be induced by the presence of methanol in the medium.

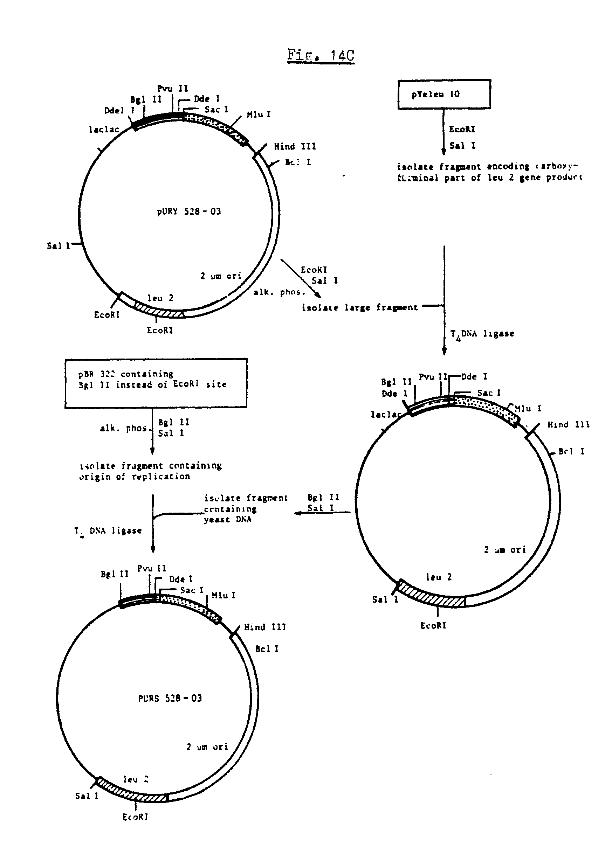

The amino acid sequence of the AAO enzyme has been published (Ronchi et al., 1981) and the complete gene is synthesised, using the phosphite technique (Matteuci and Caruthers, 1981). The gene is constructed in such a way that the optimal codon use for H. polymorpha, as derived from the sequence of the MOX gene, is used. Moreover, several unique restriction sites are introduced without changing the amino acid sequence, to facilitate subcloning during the synthesis. The DNA sequence is shown in Fig. 13. The gene is synthesised in oligonucleotides of about 50 nucleotides in length. Oligonucleotides are purified on 16% polyacrylamide gels. The oligonucleotides that form a subclone are added together in ligase buffer (Maniatis et al., 1982) and heated to 70°C in a waterbath. The waterbath is slowly cooled to 16°C and T4-ligase is added. After two hours of ligation, the DNA is separated on a 1.5% agarose gel and the fragment, having the expected length, is isolated from the gel. It is subcloned in an M13mp18 vector cleaved at the respective restriction sites situated at the end of the fragment. The gene is subcloned in this way in 4 subclones, respectively Sall-HindIII (position 39-346), HindIII-XmaI (position 346-589), XmaI-KpnI (position 589-721) and KpnI-Sall (position 721-1044). The Sall-HindIII and HindIII-XmaI subclones and the XmaI-KpnI and Kpn-I-Sall subclones are ligated together as two Sall-XmaI subclones in Sall-XmaI cleaved M13mp18. These two subclones are ligated into a Sall cleaved M13mp8, resulting in pUR 3001 (Figs 13, 14A). The whole sequence is confirmed by the determination of the nucleotide sequence using the modified Sanger dideoxy sequencing technique (Biggin et al., 1983).

The construction of the integrative plasmid, containing the AAO gene is shown in Fig. 14A,B. The nearly complete AAO gene is placed upstream of the MOX termination region, by insertion of the AAO gene-containing Sall fragment of pUR 3001, in the unique Sall site of pUR 3104 (see also Fig. 14A), resulting in pUR 3002. The orientation is checked by cleavage with HindIII. The MOX promoter region is isolated as a 1.4 kb Sall-HgiAI fragment from pUR 3102 (Fig. 14A). This fragment is subsequently placed upstream of the AAO gene in pUR 3002, by ligation to partially Sall-digested pUR 3002 in the presence of the HgiAI-Sall MOX-AAO adapter, shown in Fig. 14A. The orientation of the resulting plasmid pUR 3003 is checked again by cleavage with HindIII. This plasmid is integrated into the MOX gene after cleavage with SacI and transformation to H. polymorpha cells. Transformants are selected by their ability to grow on D-amino acids as nitrogen source in the presence of methanol as inducer.

As the selection of cells containing the AAO gene is not simple, another selection marker is introduced. To this end, the S. cerevisiae LEU2 gene is integrated in between the structural AAO gene and the MOX terminater. For this construction, the plasmid pURS 528-03 is used. This plasmid is derived from pURY 528-03 described in European patent application 0096910. The construction is shown in Fig. 14C. The deleted carboxy terminal LEU2 gene sequence of pURY 528-03 was replaced by the complete carboxy terminal LEU2 gene sequence from pYeleu 10 (Ratzkin and Carbon, 1977) and the E. coli lac-lac regulon was eliminated. Subsequently the HpaI-Sall fragment of pURS 528-03 containing the LEU2 gene, is blunt end inserted in the Sall site of pUR 3003, situated in between the AAO structural gene and the MOX terminater. The orientation of the resulting plasmid pUR 3004 can be checked by cleavage with Sall and SacI. pUR 3004 integrates in the chromosomal MOX gene of H. polymorpha after transformation of the SacI-cleaved plasmid to a H. polymorpha leu⁻ mutant. Selected leu⁺ transformants are integrated in the chromosomal MOX gene, together with the AAO gene.

Example 4
THE CONSTRUCTION OF pUR 3204, pUR 3205, pUR 3210 and pUR 3211, BY WHICH THE SMALL PEPTIDE HORMONE, THE HUMAN GROWTH HORMONE RELEASING FACTOR, IS EXPRESSED UNDER REGIE OF THE MOX-REGULON, EITHER BY INTEGRATION INTO THE CHROMOSOMAL MOX GENE (pUR 3203, pUR 3204), OR BY INTEGRATION INTO A HARS1-CONTAINING PLASMID (pUR 3205) OR BY FUSION TO THE MOX STRUCTURAL GENE (pUR 3209, pUR 3210 and pUR 3211).

Human growth hormone releasing factor (HGRF) is a small, 44 amino acids long, peptide, that activates the secretion of human growth hormone from the pituitary glands. HGRF can be used in the diagnosis and treatment of pituitary dwarfism in man. Since HGRF has been shown to induce growth hormone stimulation in numerous species, HGRF might be used in the vetinary field too, by stimulating growth of animals and increase of milk production (Coudé et al., 1984). It is difficult to obtain HGRF from human sources, but it could very well be produced by biotechnological processes, once the gene has been cloned and transferred to an appropriate host organism. Also, as a general example of the production of a peptide hormone by H. polymorpha, the gene for HGRF is synthesised in the optimal codon use of H. polymorpha and brought to expression in several ways.

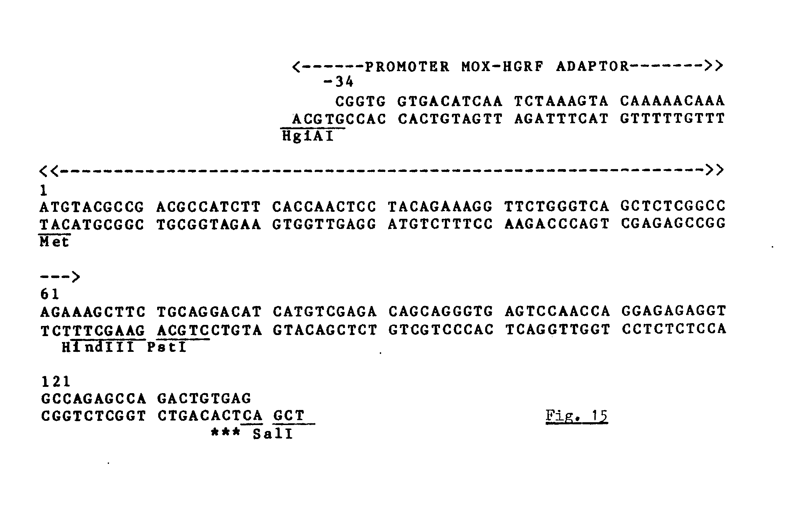

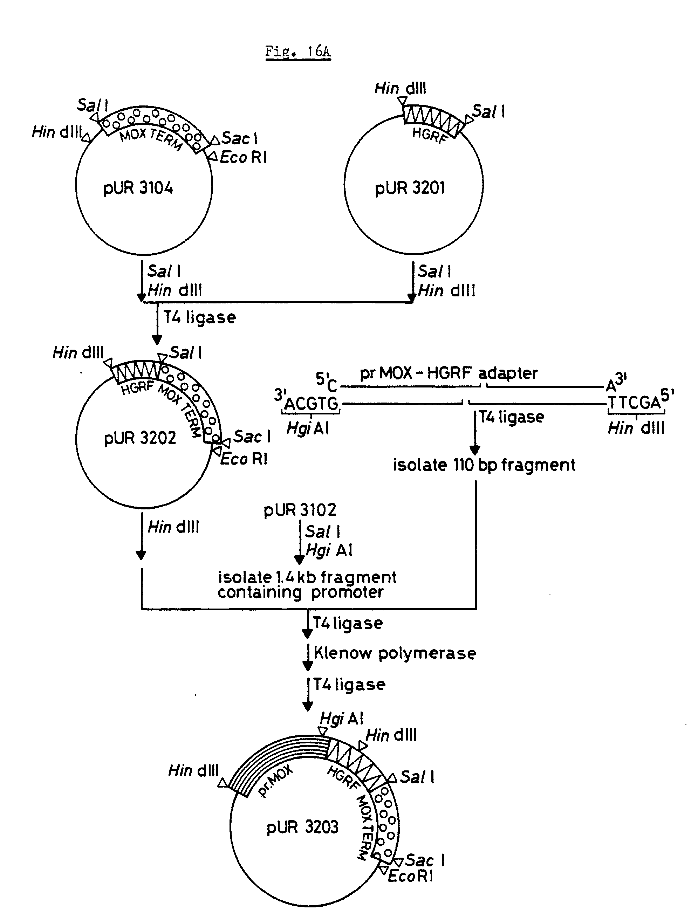

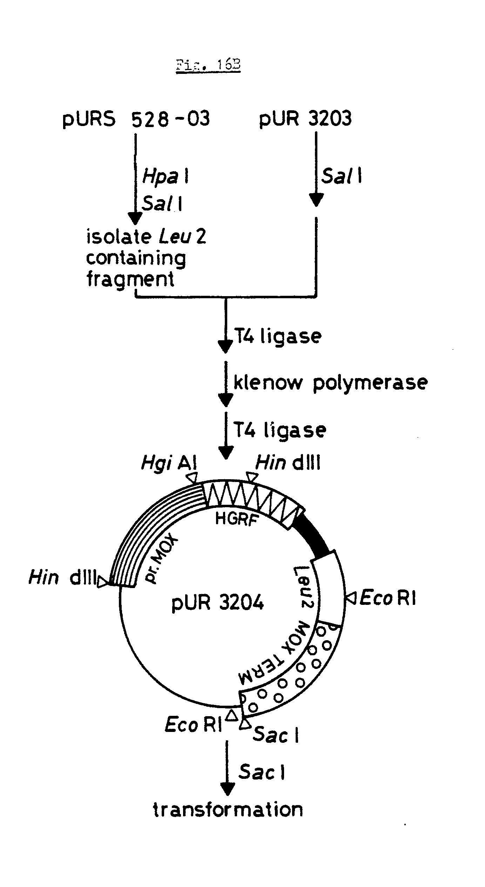

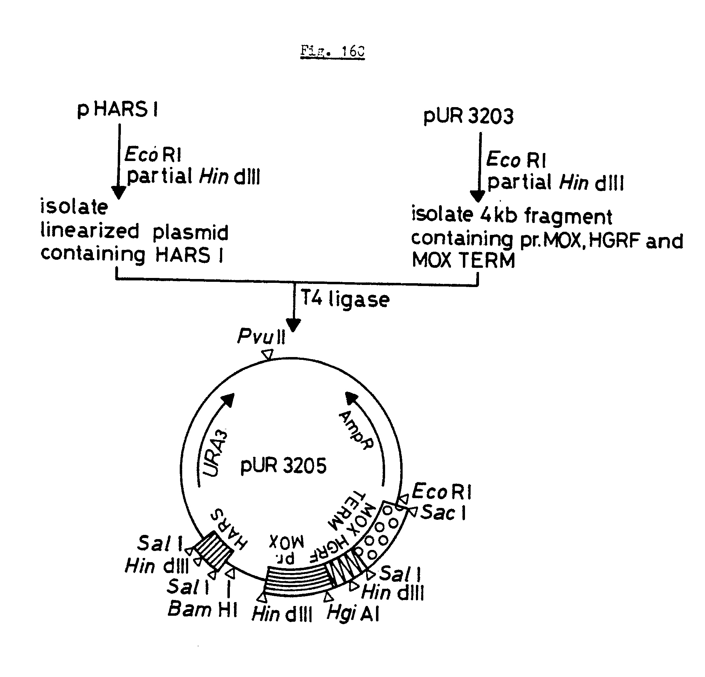

For the construction of pUR 3204 and pUR 3205, the gene fragment that codes for the carboxy terminal part of the protein is synthesised in DNA oligomers of about 50 nucleotides in length and subcloned as a HindIII-SalI fragment in HindIII-SalI cleaved M13mp18, resulting in pUR 3201 (Figs 15, 16A). This HindIII-SalI fragment is subsequently inserted upstream of the MOX terminater in HindIII-SalI cleaved pUR 3104 (Fig. 16A), resulting in pUR 3202. The MOX promoter is inserted in front of the HGRF gene, by insertion of the SalI-HgiAI MOX-promoter fragment from pUR 3102 (Fig. 16A) in HindIII cleaved pUR 3202, using a HgiAI-HindIII adapter between the MOX-promoter and the HGRF gene (Figs 15, 16A). The orientation of the resulting plasmid pUR 3203 is checked by cleavage with SalI and HgiAI. pUR 3203 integrates into the chromosomal MOX gene of H. polymorpha after transformation of the SacI cleaved plasmid. Transformants are selected on immunological activity. pUR 3203 is cleaved with SalI, to insert the SalI-HpaI fragment of pURS 528-03 (Fig. 16B) that contains the LEU2 gene. The orientation of this gene in pUR 3204 is checked by cleavage with HindIII and EcoRI. pUR 3204 integrates into the chromosomal MOX gene of H. polymorpha after transformation of the SacI cleaved plasmid (Fig. 16B) to a leu⁻ H. polymorpha mutant. Selection on on leu⁺ transformants. A plasmid, called pUR 3205, that replicates autonomously in H. polymorpha and contains the HGRF gene, is obtained by insertion of the EcoRI, partially HindIII cleaved 4 kb long fragment of pUR 3203, containing the HGRF gene inserted in between the MOX-promoter and terminater, into partially HindIII-EcoRI cleaved pHARS1 (Figs 2, 16C). The construction of pUR 3205 is checked by cleavage with HindIII.

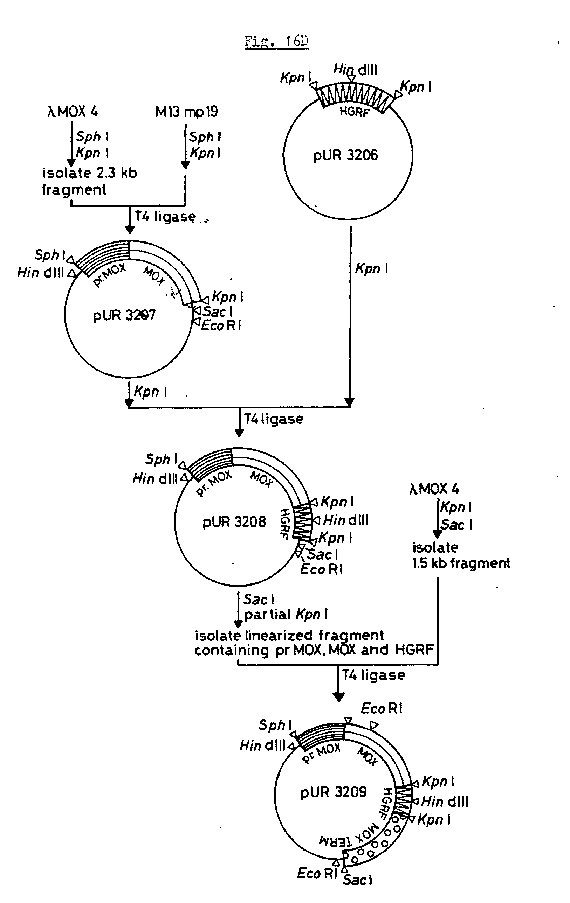

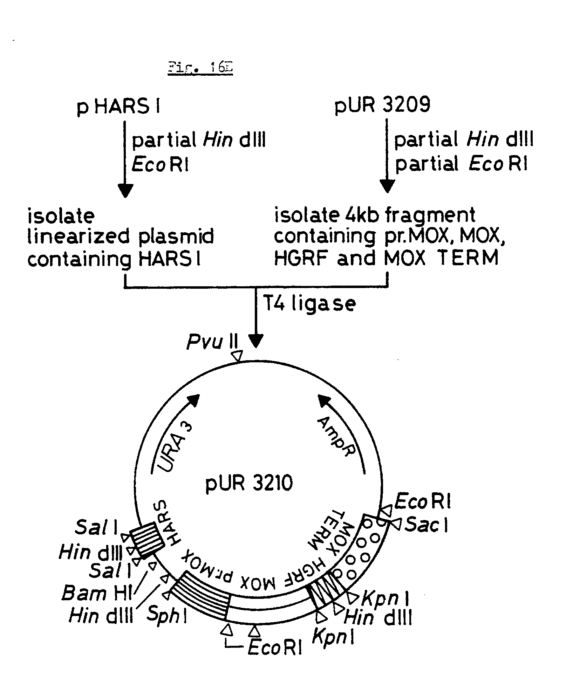

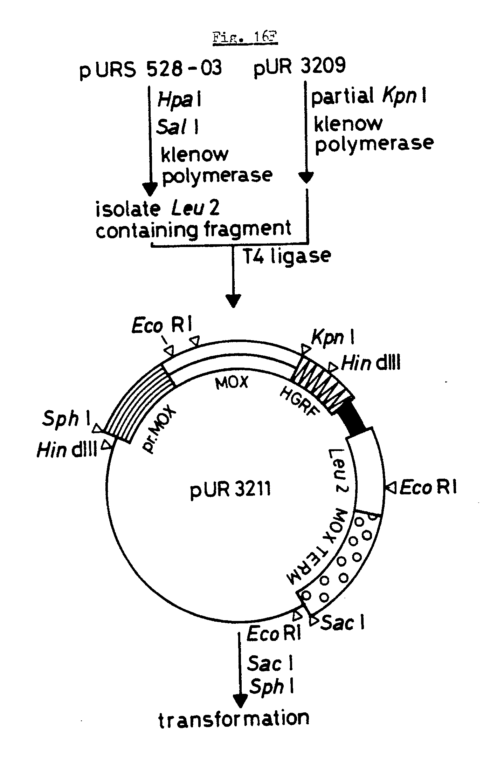

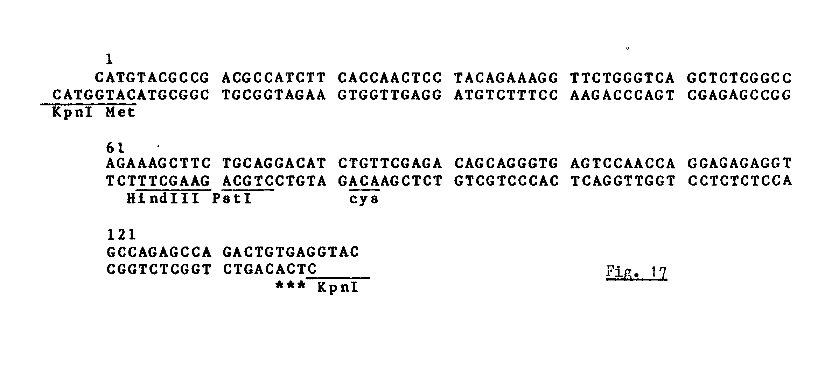

The production of small peptides as HGRF by microorganisms is often unstable as a result of enzymic degradation (Itakura et al., 1977). Fusion to a protein like MOX, and subsequent transport to the peroxisomes, could prevent degradation. Therefore, we decided to insert the HGRF gene into the unique KpnI site at position 1775 (amino acid 591, Figs 10, 11) of the MOX structural gene. The HGRF gene is synthesised again in DNA oligomers of 50 nucleotides in length, but now as two KpnI-HindIII subclones that are cloned as a complete HGRF structural gene in M13mp19, cleaved with KpnI (plasmid pUR 3206, Figs 17, 16D). Moreover, the ATG triplet coding for the internal methionine of HGRF at position 27 (Coudé et al., 1984) (position 82 of the DNA sequence) is converted into a TGT triplet coding for cysteine. This does not alter the HGRF activity essentially, and facilitates the cleavage of HGRF from the fusion protein by CNBr cleavage (Itakura et al., 1977). From phage lambda MOX-4 (Fig. 10 SphI (position -491)-KpnI fragment is isolated and in serted into SphI-KpnI cleaved M13mp19. This results in pUR 3207. pUR 3206 is cleaved with KpnI and the HGRF gene is inserted into the KpnI site of pUR 3207, resulting in pUR 3208. The orientation is checked by direct sequence analysis on the single-stranded DNA of pUR 3208. Subsequently the downstream part of the MOX gene, from the unique KpnI site up to the SacI site, is isolated as a 1.5 kb fragment from phage lambda MOX-4 and inserted into SacI - partially KpnI cleaved pUR 3208. The orientation of the resulting plasmid pUR 3209 is checked by digestion with KpnI. pUR 3209 integrates into the chromosomal MOX gene of H. polymorpha after transformation of the SacI, SphI cleaved plasmid. Selection on immunological activity.

This MOX-HGRF fusion gene is inserted into pHARS1 by isolation of the whole fusion gene from partially HindIII, partially EcoRI cleaved pUR 3209, into EcoRI partially HindIII cleaved pHARS1. This results in pUR 3210, which replicates in H. polymorpha after transformation (Fig. 16E). Alternatively, the LEU2-containing SalI-HpaI fragment of pURS 528-03 is inserted into the blunt-ended KpnI site of the HGRF gene, located at the carboxy terminus of the encoded protein, after partial KpnI cleavage of pUR 3209. The resulting plasmid pUR 3211 integrates into the chromosomal MOX gene of H. polymorpha, after transformation of the SacI, SphI cleaved plasmid (Fig. 16F).

Discussion

From the length of the open reading frame, from the similarity in the amino acid composition of purified MOX and the DNA derived protein sequence and from the identical 30 N-terminal amino acids, it is concluded that the complete gene for MOX from the yeast Hansenula polymorpha has been cloned. Its calculated molecular weight agrees well with the molecular weight determined on SDS polyacrylamide gels. Apart from the coding sequence, more than 1200 bp has been sequenced from both the 5'- and the 3'-non-coding regions, reaching from the SalI site upstream of the coding sequence, up to the SacI site downstream. The gene appears not to be interrupted with intervening sequences.

The protein is not transcribed in the form of a precursor. Based on the determination of the molecular weight, N-terminal signal sequences could not be detected in earlier studies of Roa and Blobel (1983) or Roggenkamp et al. (1984) as well. In similar studies, it was suggested that also the rat liver peroxisomal enzymes uricase (Goldman and Blobel, 1978) and catalase (Goldman and Blobel, 1978; Robbi and Lazarow, 1978) do not contain a cleavable N-terminal signal peptide. However, as discussed by these authors, proteolytic degradation could possibly explain the lack of the detection of such a signal sequence.

Our sequence results definitely prove that for translocation of this protein to the peroxisome, a cleavable N-terminal signal sequence is not required. Such a translocation signal may well be situated in the internal sequence of the mature protein, as is the case for ovalbumine (Lingappa et al., 1979). Inspection of the protein sequence reveals the amino acid sequence Gly X Gly Y Z Gly (amino acids 13-18), which is characteristicfor FAD-(flavin adenine dinucleotide)-containing enzymes (Ronchi et al., 1981).

The isolation of the MOX gene described above gives a way how to determine the DNA sequence coding for MOX and the amino acid sequence of the MOX enzyme.

Similarly, the DNA sequences and amino acid sequences belonging to other oxidase-enzymes can be isolated and determined. The knowledge of the MOX gene sequence can be used to facilitate the isolation of genes coding for alcohol oxidases or even other oxidases. By comparing the properties and the structure of enzymes one can probably establish structure function and activity relationships. One can also apply methods as site-directed mutagenesis, or shortening or lengthening of the protein coding sequences, modifying the corresponding polypeptides, to select oxidase-enzymes with improved properties, e.g. with increased alkali stability, improved production, or oxidase-enzymes which need a substrate which is more compatible with detergent products.

Besides the isolation and characterization of the structural gene for MOX from the yeast H. polymorpha, also the isolation and characterization of the structural gene for DHAS from the yeast H. polymorpha has been carried out in a similar way.

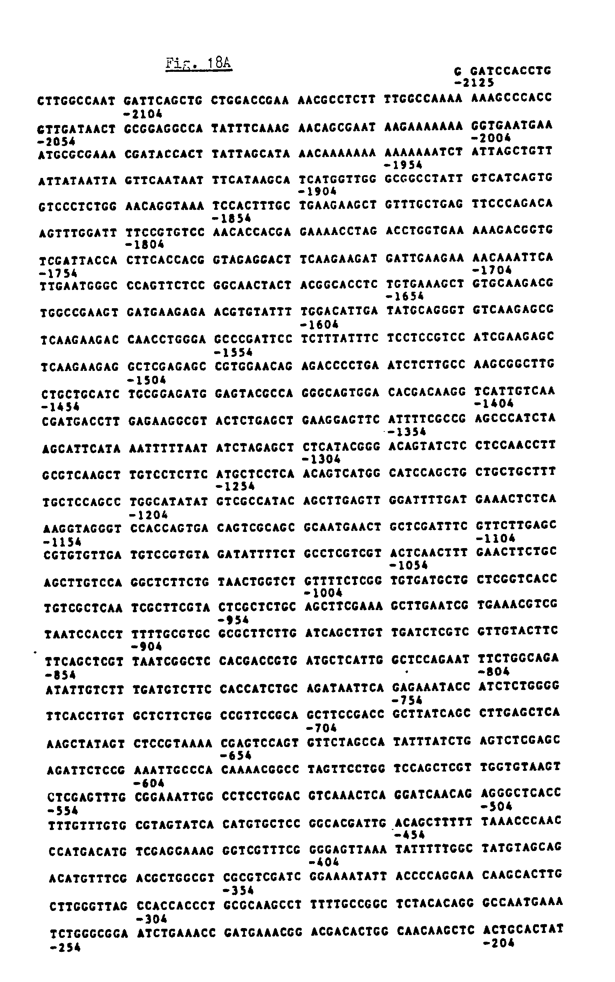

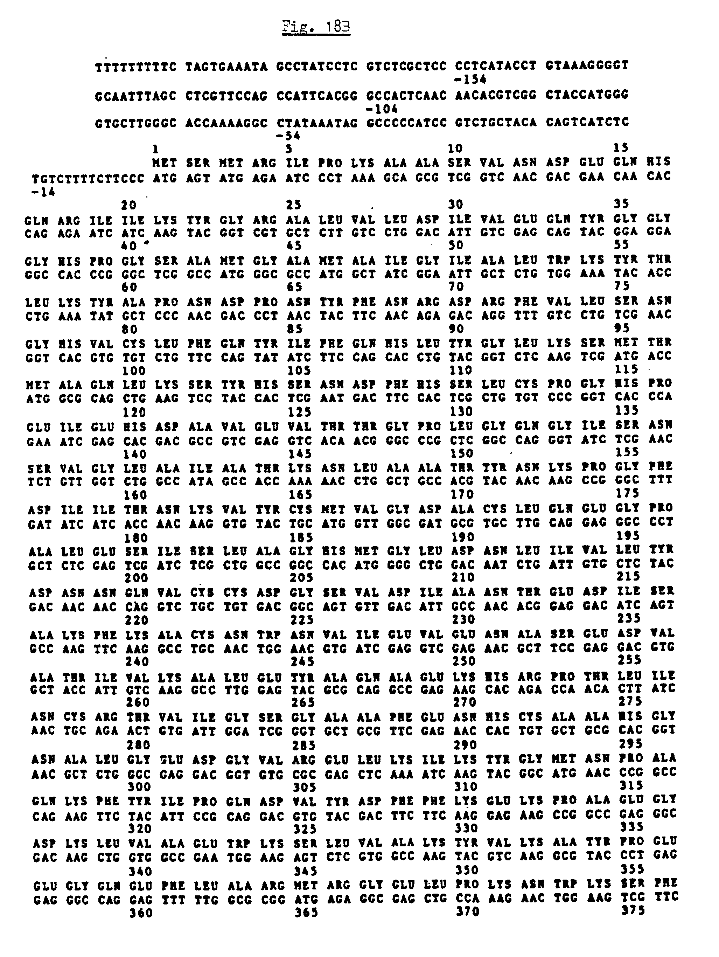

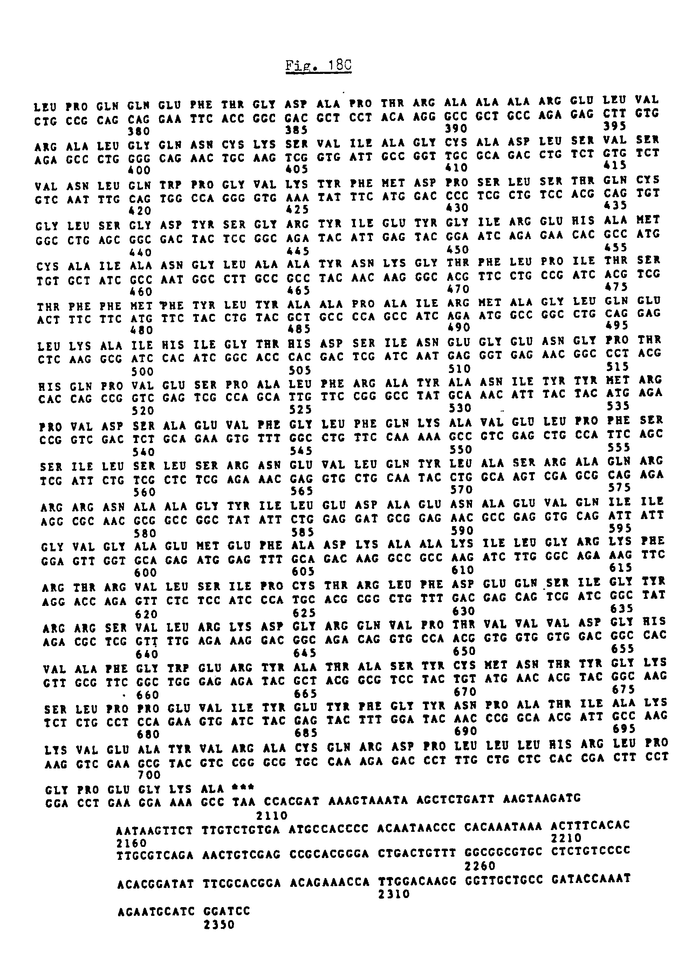

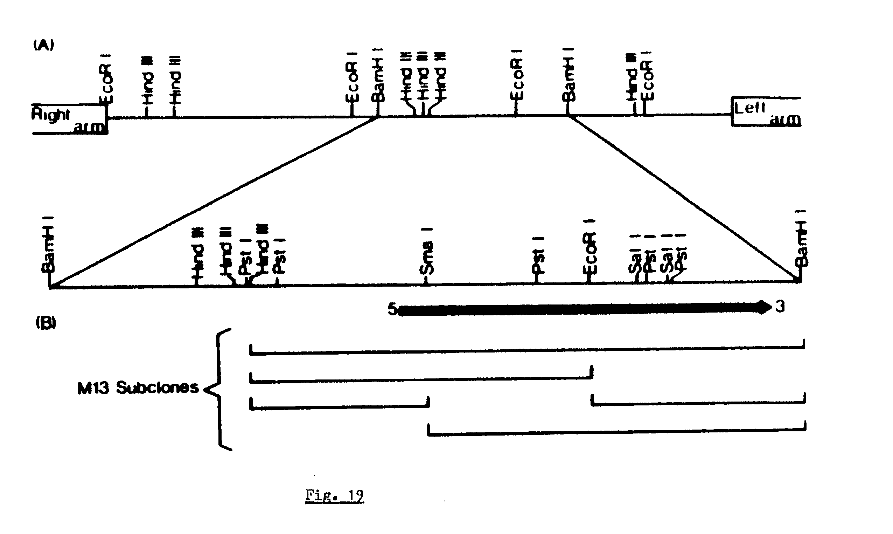

The DNA sequence of DAS is given in Fig. 18A-18C. A restriction map is given in Fig. 19. The amino acid composition calculated from the DNA sequence of DAS appeared to be in agreement with the amino acid composition determined after hydrolysis of purified DHAS. The DHAS enzyme catalyses the synthesis of dihydroxyacetone from formaldehyde and xylulose monophosphate. This reaction plays a crucial role in the methanol-assimilationprocess (cf. Veenhuis et al., 1983).

As described before, the synthesis of MOX and DHAS is subject to glucose repression. It has now been found that higher levels of MOX are reached when using glucose/methanol mixtures as substrates instead of 0.5% (v/v) methanol. Under the former conditions up to 30% of the cellular protein consists of MOX, compared with up to 20% under the latter conditions.

It was considered that in the regulons of MOX and DAS sequences must exist that play a decisive role in the regulation of repression/derepression by glucose or of the induction by methanol. Some homology therefore might be expected.

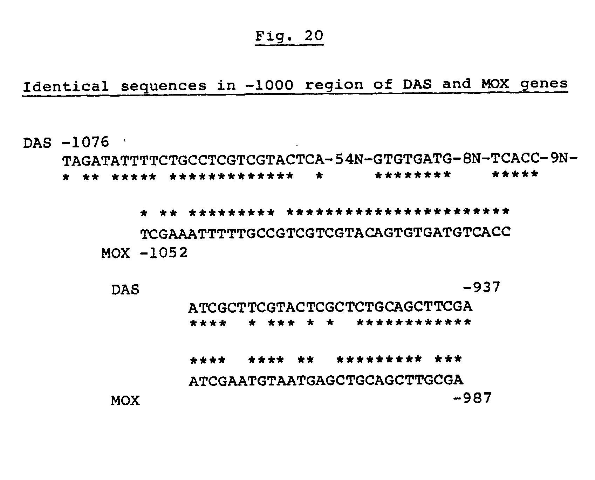

A striking homology of the "TATA-boxes" has been found, both having the sequence CTATAAATA. No other homologies in the near upstream region of the MOX and DAS regulons have been found. Unexpectedly, a detailed study of both regulons has shown a remarkable homology of the regulons for MOX and DAS in the region about 1000 bp upstream of the translation initiation codon. A practically complete consecutive region of 65 bp in the regulon of MOX is homologous to a 139 bp region in the DAS regulon, interspersed by several non-homologous regions (see Fig. 20). A similar homology is not found in any other region of both genes, that are over 4 kb in length including their upstream and downstream sequences. It is suggested that these homologous sequences play a role in the regulation of both genes by glucose and methanol. Transformation studies with vectors containing as regulon the first 500 bp upstream of the ATG of the structural gene of MOX, showed that this shortened MOX-regulon gave rise to a relatively low expression of the indicator gene beta-lactamase. Indicator genes are genes which provide the yeast with properties that can be scored easily, e.g. the gene for neomycin phosphotransferase giving resistance to the antibiotic G 418 (cf. Watson et al., 1983) or an auxotrophic marker such as leucin.

The fact that the far upstream homologous regions in the MOX and DAS genes have different interruptions and the fact that DAS is repressed at 0.1% glucose and MOX is not, suggest that these homologous regions are of importance to the repression-derepression by glucose and/or the induction of the expression in the presence of methanol. This assumption has been found correct indeed, and the presence or absence of these homologous regions can therefore be important for specific applications. For example, if the -1052 to -987 region of the MOX gene or the -1076 to -937 region of the DAS gene is important for the induction of MOX or DAS by methanol, the presence of these regions is required for the expression of MOX or DAS and/or for the induction of other enzymes by methanol. Another example might be the removal of the regions to avoid repression by glucose, which is needed for the expression of genes coding for proteins other than MOX and DHAS under influence of the MOX and/or DAS regulatory regions with glucose as a carbon source.

Inducible gene expression as shown in this specification was known in the art.

J.T. Lis c.s. describe in Cell 35 (December 1983) 403-410 the use of a hybrid gene including 194 bp of the sequence upstream of the start of the transcript of the *Drosophila* heat shock gene fused to the *E. coli* β-galactosidase gene in a *Drosophila* germline. The β-galactosidase activity in the transformants is inducible by heat shock and shows a widespread distribution throughout the tissues of larvae and adults.

R.L. Brinster c.s. describe in Nature 296 (4 March 1982) 39-42 the use of a plasmid with the promoter/regulatory region of the mouse metallothionein-I gene fused to the structural gene of herpesvirus thymidine kinase. When mouse eggs were microinjected and incubated with cadmium (a natural inducer of metallothionein gene transcription) thymidine kinase activity increased about 10-fold compared with control eggs not exposed to cadmium. The minimum sequence required for cadmium regulation lies within 90 nucleotides of the transcription start site.

However, the present invention provides an induction in more practical systems enabling a high yield of the polypeptide to be produced.

Thus one aspect of the present invention relates to the isolation and complete characterization of the structural genes coding for MOX and DHAS from the yeast H. polymorpha. It further relates to the isolation and complete characterization of the DNA sequences that regulate the biosynthesis of MOX and DHAS in H. polymorpha, notably the regulons and terminaters.

Moreover, it relates to combinations of genes coding for alcohol oxidase or other oxidases originating from H. polymorpha strains other than H. polymorpha CBS 4732, or Hansenula species other than H. polymorpha, or yeast genera other than Hansenula, or moulds, or higher eukaryotes, with the powerful regulon and terminater of the MOX gene from H. polymorpha CBS 4732. These combinations may be located on vectors carrying amongst others an autonomously replicating sequence originating from H. polymorpha or related species or minichromosomes containing centromers, and optionally selection marker- (s) and telomers. These combinations may also be integrated in the chromosomal DNA of H. polymorpha.

Furthermore it relates to combinations of the powerful regulon or parts of it and terminaters of the MOX and/or DAS and - by site-directed mutagenesis or other methods - changed structural genes coding for alcohol oxidase or another oxidase. These changed structural genes may be located on episomal vectors, in minichromosomes or integrated in the chromosomes of H. polymorpha, H. wingeii, H. anomala, and S. cerevisiae or in other yeasts.

Besides this, the present invention relates to combinations of the regulon and terminater of the MOX and/or DAS gene of H. polymorpha with structural genes coding for other proteins than oxidases.

A very important and preferred embodiment of the invention is a process for preparing a polypeptide, such as a protein or an enzyme, by culturing a microorganism under suitable conditions, optionally concentrating the polypeptide and collecting same in a manner known per se, characterized in that a microorganism is used that has been obtained by recombinant DNA technology and caries a structural gene coding for the polypeptide concerned, the expression of which is under the control of a regulon, comprising a promoter and at least either the -1052 to -987 region of the MOX gene of Hansenula polymorpha CBS 4732, or the -1076 to -937 region of the DAS gene of Hansenula polymorpha CBS 4732, or a corresponding region of other methylotrophic moulds or yeasts, or an effective modification of any of these regions.

Surprisingly, it has been observed by the present inventors that the regions concerned, which are shown in Fig. 20 and are referred to herein as the -1000 regions of the MOX and DAS genes, are of crucial importance for the expression of the structural gene concerned. Experiments performed with recombinants containing the MOX regulon from which this region was eliminated showed a low level of expression. Therefore, use of a regulon comprising such -1000 region, or an effective modification thereof, i.e. any modification which does not result in a significant mutilation of the function of said region, makes it possible to realize production of a relatively high amount of the desired polypeptide.

A preferred embodiment of this process according to the invention is characterized in that the structural gene concerned has been provided with one or more DNA sequences coding for amino acid sequences involved in the translocation of the gene product into the peroxisomes or equivalent microbodies of the microbial host. Translocation of the produced polypeptide into the peroxisomes or equivalent microbodies improves their stability, which results in a higher yield. For certain kinds of polypeptides, in particular oxidases, such translocation is imperative for survival of the microbial host, i.e. to protect the host against the toxic effects of the hydrogen peroxide produced when the microbial host cells are growing on the substrate of the oxidase. If the oxidase concerned does not contain addressing signals which are functional in the microbial host used in the production process, one should provide the structural gene with sequences coding for host specific addressing signals, for example by adding such sequences or by substituting these for the original addressing sequences of the gene. production of a fused polypeptide, in which the fusion partner carries suitable addressing signals, is another possibility. In case methylotrophic yeasts are used in the production process, it is preferred that the DNA sequences consist of the MOX gene or thos parts thereof which are responsible for MOX translocation into the peroxisomes or microbodies.

Finally, this aspect of the present invention is related to the synthesis of MOX originating from H. polymorpha in other yeasts.

Some microorganisms with the potential of producing alcohol oxidases are summarized below.

Yeasts producing alcohol oxidases
(Taxonomic division according to Lee and Komagata, 1980)

| | |
|---|---|
| Group 1 | Candida boidinii |
| Group 2a | Hansenula philodendra |
| | Pichia lindnerii |
| | Torulopsis nemodendra |
| | Torulopsis pinus |
| | Torulopsis sonorensis |
| Group 2b | Candida cariosilignicola |
| | Hansenula glucozyma |
| | Hansenula henricii |
| | Hansenula minuta |
| | Hansenula nonfermentans |
| | Hansenula polymorpha |
| | Hansenula wickerhamii |
| | Pichia pinus |
| | Pichia trehalophila |
| Group 2c | Candida succiphila |
| | Torulopsis nitratophila |
| Group 3 | Pichia cellobiosa |
| Group 4 | Hansenula capsulata |
| | Pichia pastoris |
| | Torulopsis molischiana |

Moulds producing alcohol oxidases: Lenzites trabea
Polyporus versicolor
Polyporus obtusus
Poria contigua

Among the oxidases other than alcohol oxidases, the most interesting are:
- glycerol oxidase,
- aldehyde oxidase,
- amine oxidase,
- aryl-alcohol oxidase,
- amino acid oxidase,
- glucose oxidase,
- galactose oxidase,
- sorbose oxidase,
- uric acid oxidase,
- chloroperoxidase, and
- xanthine oxidase.

Combinations of the powerful regulons and terminaters of the MOX and DAS genes from H. polymorpha and structural genes for oxidases may be combined with one or more DNA sequences that enable replication of the structural gene in a particular host organism or group of host organisms, for example autonomously replicating sequences or centromers (and telomers) originating from H. polymorpha, to suitable vectors that may be transferred into H. polymorpha and related yeasts or other microorganisms.

H. polymorpha mutants LEU-1 and LR9, mentioned on page 12 of this specification, were deposited at the Centraalbureau voor Schimmelcultures at Delft on 15th July, 1985, under numbers CBS 7171 and CBS 7172, respectively.

The above description is followed by a list of references, claims, Tables, Legends to Figures and Figures.

References

1. GB-PS 1 225 713 (Colgate-Palmolive Company; publ. 24th March 1971; priority date 19th April 1968).
2. DE-PA 2 557 623 (Henkel & Cie GmbH; publ. 30th June 1977; priority date 20th December 1975).
3. GB-PA 2 101 167 (Unilever PLC; publ. 12th January 1983; priority date 7th July 1981).

4. van Dijken, J.P., Otto, R. and Harder, W. (1976), Arch.Microb. 111, 137-144.

5. Veenhuis, M., van Dijken, J.P. and Harder, W. (1983) in Advances in Microbial Physiology, Rose, A.H., Gareth Morris, J. and Tempest, D.W., Eds, Vol. 24, pp 1-82, Academic Press, New York.

6. Roggenkamp, R., Janowicz, z., Stanikowski, B. and Hollenberg, C.P. (1984), Mol.Gen.Genet. 194, 489-493.

7. Sahm, H. (1977) in Advances in Microbiol. Engineering, Ghose, T.K., Fiechter, A. and Blakebrough, N., Eds, Vol. 6, pp 77-103, Springer-Verlag, Berlin.

8. Bystrykh, L.V., Sokolov, A.P. and Trotsenko, Y.A. (1981), FEBS Letters 132, 324-328.

9. Roa, M. and Blobel, G. (1983), Proc.Natl.Acad.Sci. USA, 80, 6872-6876.

10. Veenhuis, M., van Dijken, J.P., Pilon, S.A.F. and Harder, W. (1978), Arch.Microbiol. 117, 1953-163.

11. Loenen, W.A.M. and Brammar, W.J. (1980), Gene 20, 249-259.

12. Hohn, B. (1979) in Methods in Enzymology, Wu, R., Ed., Vol. 68, pp 299-309, Academic Press, New York.

13. Edens, L., Heslinga, L., Klok, R., Ledeboer, A.M., Maat, J., Toonen, M.Y., Visser, C. and Verrips, C.T. (1982), Gene 18, 1-12.

14. Pelham, H.R.B. and Jackson, R.J. (1976), Eur.J. Biochem. 67, 247-257.

15. Valerio, D., Duyvensteijn, M.G.C., Meera Khan, P., Geurts van Kessel, A., de Waard, A. and van der Eb, A.J. (1983), Gene 25, 231-240.

16. Ledeboer, A.M., Verrips, C.T. and Dekker, B.M.M. (1984), Gene 30, 23-32.

17. Maniatis, T., Fritsch, E.T. and Sambrook, J. (1982), Molecular Cloning, p 278, Cold Spring Harbor Laboratory Publ., New York.

18. Benton, W.D. and Davis, R.W. (1977), Science 196, 180-182.

19. Ambler, R.P. (1972), Methods in Enzymology, Vol. 25, pp 262-272, Academic Press, New York.

20. Matteucci, M.D. and Caruthers, M.H. (1981), J.Am. Chem.Soc. 103, 3185-3191.

21. Wallace, R.B., Johnson, M.J., Hirose, T., Miyake, T., Kawashima, E.H. and Itakura, K. (1981), Nucl. Acids Res. 9, 879-894.

22. Biggin, M.D., Gibson, T.J. and Hong, G.F. (1983), Proc.Natl.Acad.Sci.USA 80, 3963-3965.

23. Gleeson, M.A., Waites, M.J. and Sudbery, P.E. (1984), in: Microbial growth on $C_1$ compounds, Eds. Crawford, R.L. and Hanson, R.S., Publ. A.S.M., Washington, 228-235.

24. Fink, G.D. (1970), Methods in Enzymology, Tabor, H. and Tabor, C.W., Eds., Vol. 17, pp 59-78, Academic Press, New York.

25. Boeke, J.D., LaCroute, F. and Fink, G.D. (1984), Mol. Gen.Genet. 197, 345-346.

26. Jones, E.W. and Fink, G.D. (1982), Cold Spring Harbour Monogr.Ser., 11B, 181-299.

27. Lieberman, I., Kornberg, A. and Simms, E.S. (1955), J.Biol.Chem. 215, 403-415.

28. Stinchcomb, D.T., Thomas, M., Kelly, J., Selker, E. and Davis, R.W. (1980), Proc.Natl.Acad.Sci.USA 77, 4559-4563.

29. Stinchcomb, D.T., Mann, C. and Davis, R.W. (1982), J.Mol.Biol. 158. 157-179.

30. Struhl, K., Stinchcomb, D.T., Scherer, S. and Davis, R.W. (1979), Proc.Natl.Acad.Sci.USA 76. 1035-1039.

31. Carlson, M. and Botstein, D. (1982), Cell 28, 145-154.

32. Beggs, J.D. (1978), Nature 275, 104-109.

33. Broach, J.R., Strathern, J.N. and Hicks, J.B. (1979), Gene 8, 121-133.

34. Das, S., Kellerman, E. and Hollenberg, C.P. (1984), J.Bacteriol. 158, 1165-1167.

35. Ito, H., Fukuda, Y., Murata, K. and Kimura, A. (1983), J.Bacteriol. 153, 163-168.

36. Klebe, R.J., Harriss, J.V., Sharp, Z.D. and Douglas, M.G. (1983), Gene 25, 333-341.

37. Hollenberg, C.P. (1982), Curr.Top.Microbiol.Immunol. 96, 119-144.

38. Kato, N., Omori, Y., Tani, Y. and Ogata, K. (1976), Eur.J.Biochem. 64, 341-350.

39. Schütte, H., Flossdorf, J., Sahm, H. and Kula, M.R., (1976), Eur.J.Biochem. 62, 151-160.

40. Ronchi, S., Minchiotti, L., Galliano, M., Curti, B., Swenson, R.P., Williams, C.H. and Massey, V. (1981), J.Biol.Chem. 257, 8824-8830.

41. Ratzkin, B. and Carbon, J. (1977), Proc.Natl.Acad. Sci.USA 74, 487-491.

42. Coudé, F.X., Diaz, J., Morre, M., Roskam, W. and Roncucci, R. (1984), Trends in Biotechnology 2, 83-88.

43. Itakura, K., Hirose, T., Crea, R., Riggs, A.D., Heyneker, H.L., Bolivar, F. and Boyer, H.W. (1977), Science 198. 1056-1063.

44. Goldman, B.M. and Blobel, G. (1978), Proc.Natl. Acad.Sci.USA 75, 5066-5070.

45. Robbi, M. and Lazarow, P.B. (1978), Proc.Natl.Acad. Sci.USA 75, 4344-4348.

46. Lingappa, V.R., Lingappa, J.R. and Blobel, G. (1979), Nature 281, 117-121.

47. Watson, J.D., Tooze, J. and Kurtz, D.T. (1983), Recombinant DNA, A Short Course, page 178, published by W.H. Freeman and Company, New York.
48. Lee, J.D. and Komagata, K. (1980), J.Gen.Appl. Microbiol. 26, 133-158.

## TABLE I

Activities of orotidine 5'-phosphate decarboxylase and orotidine 5'-phosphate pyrophosphorylase in H. polymorpha mutants requiring uracil for growth.

| Strain/ Genotype | Reversion rate | Activity (%)[a] | |
| --- | --- | --- | --- |
| | | Orotidine 5'-phosphate decarboxylase | Orotidine 5-phosphate pyrophosphorylase |
| Wild type | – | 100 | 100 |
| LR 9/odcl | $< 2 \times 10^9$ | $< 1$ | 106 |
| MR 7/odcl | $6 \times 10^7$ | $< 1$ | 71 |
| NM 8/odcl | $3 \times 10^8$ | $< 1$ | 105 |
| CLK 55/oppl | n.e.[b] | 90 | $< 1$ |
| CLK 68/oppl | n.e. | 82 | $< 1$ |
| YNN 27/ura3 | n.e. | 0 | n.e. |

Strains were grown in YEPD until late exponential phase. Extraction of cells was performed with glass beads using a Braun homogenizer. Protein was estimated by the optical density at 280 nm.

a) Expressed as the percentage of wild type activity.

b) Not estimated.

## TABLE II

Transformation of uracil-requiring mutants of H. poly-morpha

| Strain | Plasmid | Transformation frequency[a] | Stability[b] (%) | Status of transformed DNA |
|--------|---------|------------------|-----------|----------------------|
| LR 9 | YRP17 | $2.2 \times 10^2$ | $<1$ | Autonomous replication |
| LR 9 | pHARS1 | $1.5 \times 10^3$ | 2 | Autonomous replication |
| LR 9 | pHARS2 | $4.6 \times 10^2$ | 1.5 | Autonomous replication |
| LR 9 | YIP5 | $3\ (38)^{c}$ | 105 | Integration |
| LR 9 | pRB58 | 0 | – | – |
| LR 9 | pHH85 | 0 | – | – |
| YNN 27 | YIP5 | 0 | – | – |

a) Expressed as total number per /ug of DNA. Intact cells treated with polyethyleneglycol were used for transformation as described in Materials and Methods.

b) Expressed as the percentage of remaining uracil prototrophs after growth on YEPD for ten generations.

c) Number in parentheses indicates the amount of mini-colonies containing free plasmid YIP5.

## TABLE III

### Amino acid composition of MOX

| Amino Acid | DNA sequence | Hydrolysate [a] |
|---|---|---|
| PHE | 31 | 32 |
| LEU | 47 | 49 |
| ILE | 34 | 34 |
| MET | 12 | 11 |
| VAL | 42 | 43 |
| SER | 43 | 33 [a] |
| PRO | 43 | 42 |
| THR | 44 | 38 |
| ALA | 47 | 50 |
| TYR | 27 | 27 |
| HIS | 19 | 21 |
| GLN | 13 | ] 51 |
| GLU | 36 | |
| ASN | 32 | ] 84 |
| ASP | 50 | |
| LYS | 35 | 38 |
| CYS | 13 | 12 |
| TRP | 10 | – [b] |
| ARG | 36 | 36 |
| GLY | 50 | 53 |

a) Hydrolysis was performed for 24 h.

b) Not determined.

## TABLE IV

### Comparison of preferred codon usage in S. cerevisiae, H. polymorpha and E. coli

| | Saccharomyces | Hansenula MOX | E. coli |
|---|---|---|---|
| ALA | GCU, GCC | GCC | GCC not used, no clear pref. |
| SER | UCU, UCC | UCC, UCG | UCU, UCC |
| THR | ACU, ACC | ACC | ACU, ACC |
| VAL | GUU, GUC | GUA not used, no clear pref. | GUU, GUA |
| ILE | AUU, AUC | AUC, AUU | AUC |
| ASP | GAC | GAC | GAC |
| PHE | UUC | UUC | UUC |
| TYR | UAC | UAC | UAC |
| CYS | UGU | no clear pref. | no clear pref. |
| ASN | AAC | AAC | AAC |
| HIS | CAC | CAC | CAC |
| GLU | GAA | GAG | GAA |
| GLY | GGU | GGC practically not used, no clear pref. | GGU, GGC |
| GLN | CAA | CAG | CAG |
| LYS | AAG | AAG | AAA |
| PRO | CCA | CCU, CCA | CCG |
| LEU | UUG | CUG, CUC | CUG |
| ARG | AGA | AGA | CGU |

Legends to Figures

Fig. 1.      The exonuclease Bal31 digestion strategy used in sequencing specific MOX subclones. The fragment X-Y̅ subcloned in M13mp-8 or -9, -18 or -19 is cut at the unique restriction site Z. The DNA molecule is subjected to a time-dependent exonuclease Bal31 digestion. The DNA fragment situated near the M13 sequencing primer is removed using restriction enzyme Y; ends are made blunt end by incubation with T₄-DNA polymerase and then ligated intramolecularly. phage plaques are picked up after transformation and the fragment is sequenced from site Z in the direction of site X.

Using the M13 derivative with a reversed multiple cloning site, the fragment is sequenced from site Z in the direction of site X.

Fig. 2.   Alignment of pHARS plasmids derived by insertion of HARS fragments into the single SalI site of YIp5.

Fig. 3.   The complete nucleotide sequence of the HARS-1 fragment.

Fig. 4.   Estimation of copy number by Southern hybridization of H. polymorpha transformants. An aliquot of 8 and 16 ,ul of each probe was electrophoresed. Lane 1, phage lambda DNA digested with HindIII and EcoRI. Lanes 2,3 transformant of K. lactis containing two copies of integrated plasmid, digested with HindIII (M. Reynen, K. Breunig and C.P. Hollenberg, unpublished); lanes 4-7, YNN 27, transformed with pRB58 (4-5) and YRP17 (6-7) digested with EcoRI respectively; lanes 8,9, LR9 transformed with YRP17 digested with EcoRI; lanes 10,11, LR9 transformed with pHARS2 digested with HindIII; lanes 12,13, LR9 transformed with pHARS1 digested with EcoRI.

Fig. 5.   Autoradiogram of Southern blots of DNA from H. polymorpha mutant LR9 transformed by integration of plasmid YIp5. Lane 1, phage lambda DNA, digested both with HindIII and EcoRI; lane 2, pHARS-1, undigested; lanes 3-5 and lanes 6,7 show DNA from 2 different transformants. Lane 3, undigested; lane 4, digested with EcoRI; lane 5, digested with PvuII; lane 6, digested with EcoRI; lane 7, digested with PvuII; lane 8, plasmid YIp5, digested with EcoRI. Nick-translated YIp5 was used as a hybridization probe.

Fig. 6    Electrophoresis of $^{32}$p-labelled RNA from Hansenula polymorpha, purified once (lane A) or twice (lane B) on oligo(dT)cellulose. Electrophoresis was performed on a denaturing 7 M urea 2.5% polyacrylamide gel. The position of the yeast rRNA's and their respective molecular weights are indicated by 18S and 25S. The 2.3 kb band, that can be seen in lane B, was converted into a cDNA probe which was subsequently used to isolate MOX and DHAS clones from the Hansenula polymorpha clone bank.

Fig. 7    $^{35}$S-labelled proteins obtained after in vitro translation of methanol derepressed, Hansenula polymorpha mRNA with a rabbit reticulocyte lysate. Either 2 microliters of the total lysate (lane A) or an immuno-precipitate of the remaining 18 microliters using a MOX specific antiserum (lane B) were separated on an 11.5% SDS-polyacrylamide gel. A mixture of proteins with known molecular weights was used as markers.

Fig. 8.   The N-terminal sequence of purified MOX, as determined on a Beckman sequenator. The two probes that could be derived from the sequence Pro-Asp-Gln-Phe-Asp, using Saccharomyces preferred codons, are indicated.

Fig. 9.   Hybridization of a DBM blot of HindIII/SalI cut MOX clones. The DNA was separated on a 1.5% agarose gel (Fig. 9A) and the blot was hybridized to a mixture of both MOX-derived synthetic DNA probes (Fig. 8). Only one band of clones 1, 4 and 5 hybridize (Fig. 9B), indicated by an arrow in Fig. 9A. Lane M: molecular weight markers as indicated. Lane A, B, C and D: clones 1, 3, 4 and 5, respectively. Lane E: lambda L47.1.

Fig. 10.  Restriction map for MOX clone 4. Only relevant restriction sites are indicated that have been used for subcloning and sequencing of the MOX gene. The open reading frame, containing the structural MOX sequence, and the M13 subclones made are depicted.Restriction sites used are: B = BamHI, E$_I$ = EcoRI, E$_V$ = EcoRV, p = PstI, Sl = SalI, Sc = SacI, St = StuI, H = HindIII, Sp = SphI, K = KpnI, Hg = HgiAI and X = XmaI.

Fig. 11A, B.   The nucleotide sequence of the MOX structural gene and its 5'- and 3'-flanking sequence.

Fig. 12A,C.   The construction of plasmid pUR 3105 by which the neomycin phosphotransferase gene integrates into the chromosomal MOX gene of H. polymorpha.

Fig. 12B.   Promoter MOX-neomycin phosphotransferase adapter fragments.

Fig. 13.   The DNA sequence of the AAO gene, derived from the published amino acid sequence. The gene is synthesised in the optimal codon use for H. polymorpha in oligonucleotides of about 50 nucleotides long. Restriction sites, used for subcloning are indicated. The HgiAI-SalI fragment forms the adapter between the structural AAO gene and the MOX promoter. The translational start codon (met) and stop codon (***) are indicated. The structural sequence is numbered from 1 to 1044, while the MOX promoter is numbered from -34 to -1.

| | |
|---|---|
| Fig. 14A. | The construction of pUR 3003, by which the AAO gene integrates into the chromosomal MOX gene of H. polymorpha. Selection on activity of the AAO gene. |
| Fig. 14B. | The construction of pUR 3004, by which the AAO gene integrates into the chromosomal MOX gene of a H. polymorpha leu⁻ derivative. Selection on leu⁺. |
| Fig. 14C. | The construction of pURS 528-03. Owing to the removal of the pCR1 sequence and the double lac UV5 promoter, this plasmid is about 2.2 kb shorter than pURY 528-03. |
| Fig. 15. | The DNA sequence of the HGRF gene, derived from the published amino acid sequence. The gene is synthesised in the optimal codon use for H. polymorpha in oligonucleotides of about 50 nucleotides long. HgiAI, HindIII and SalI sites are used for subcloning. The HgiAI-HindIII fragment forms the adapter between the structural HGRF gene and the MOX promoter. The translational start codon (met) and stop codon (***) are indicated. The structural sequence is numbered from 1 to 140, while the MOX promoter is numbered from -34 to -1. |
| Fig. 16A. | The construction of pUR 3203, by which the gene coding for HGRF integrates into the chromosomal MOX gene of H. polymorpha. Selection on immunological activity of HGRF. |
| Fig. 16B. | The construction of pUR 3204, by which the gene coding for HGRF integrates into the chromosomal MOX gene of a H. polymorpha leu⁻ derivative. Selection on leu⁺. |
| Fig. 16C. | The construction of pUR 3205, by which the gene coding for HGRF is inserted into a HARS-1-containing plasmid, which replicates autonomously in H. polymorpha. Selection by transformation of a ura⁻ mutant. |
| Fig. 16D. | The construction of pUR 3209, by which the gene coding for HGRF integrates into the chromosomal MOX gene of H. polymorpha, fused to the structural MOX gene. HGRF is cleaved from the fusion protein by CNBr cleavage. Selection on immunological activity of HGRF. |
| Fig. 16E. | The construction of pUR 3210, by which the gene coding for HGRF is inserted into a HARS-1-containing plasmid, fused to the structural MOX gene. Selection as in Fig. 16C. |
| Fig. 16F. | The construction of pUR 3211, by which the gene coding for HGRF integrates into the chromosomal MOX gene of a H. polymorpha leu⁻ derivative, fused to the structural MOX gene. Selection on leu⁺. |
| Fig. 17. | The DNA sequence of the HGRF gene, derived from the published amino acid sequence. The gene is synthesised as mentioned in Fig. 15, but constructed in such a way that it could be inserted into the unique KpnI site of the structural MOX gene. Therefore it was equipped with KpnI sites on both sides of the gene, and KpnI-HindIII fragments were used for subcloning. Synthesis will be as a fusion product to the MOX enzyme. The internal met (ATG) at position 82 is converted into a cys (TGT). Translational start (met) and stop (***) codons are indicated. |
| Fig. 18A,B,C. | The nucleotide sequence of the DAS structural gene and its 5'- and 3'-flanking sequence. |
| Fig. 19. | Restriction map for the DAS-lambda clone. Only relevant restriction sites are indicated that have been used for subcloning and sequencing of the MOX gene. The open reading frame, containing the structural DAS sequence, and the M13 subclones made, are depicted. |
| Fig. 20. | Identical sequences in -1000 region of DAS and MOX genes. |

## Claims

1. Process for preparing a polypeptide by culturing a microorganism under such conditions that the microorganism produces the polypeptide, optionally concentrating the polypeptide and collecting same in a manner known *per se*, characterized in that a transformed microorganism is used that carries a structural gene coding for the polypeptide concerned and under the control of a regulon comprising a promoter and at least either the -1052 to -987 region of the MOX gene of *Hansenula polymorpha* CBS 4732 given in Fig. 11A, or the -1076 to -937 region of the DAS gene of *Hansenula polymorpha* CBS 4732 given in Fig. 18A, or a corresponding region of other methylotrophic moulds or yeasts, or a modification of any of these regions that does not impair the regulon function, while the structural gene coding for the polypeptide and the regulon are introduced into the microorganism by means of a vector.

2. Process according to claim 1, characterized in that the promoter is derived from the yeast *Hansenula polymorpha.*

3. Process according to claim 1 or 2, characterized in that the microorganism is a mould or yeast.

4. Process according to claim 3, characterized in that a mould or yeast is selected from the group consisting of the genera *Aspergillus, Candida, Geotrichum, Hansenula, Lenzites, Nadsonia, Pichia, Poria, Polyporus, Saccharomyces, Sporobolomyces, Torulopsis, Trichospora* and *Zendera.*

5. Process according to claim 4, characterized in that the mould or yeast is selected from the species *Aspergillus japonicus, Aspergillus niger, Aspergillus oryzae, Candida boidinii, Hansenula anomala, Hansenula polymorpha, Hansenula wingeii, Kloeckera* sp. 2201 and *Pichia pastoris.*

6. Process according to claim 5, characterized in that the microorganism is the yeast species *Hansenula polymorpha.*

7. Process according to any of claims 1-6, characterized in that the structural gene concerned has been provided with one or more DNA sequences which translocate the gene product into the peroxisomes or equivalent microbodies of the microbial host.

8. Process according to claim 7, characterized in that the DNA sequences concerned consist of the MOX gene or those parts thereof which are responsible for MOX translocation into the peroxisomes or microbodies.

9. Transformed microorganism suitable for use in a process as claimed in one of claims 1-8, comprising a structural gene coding for the polypeptide to be prepared, the expression of the gene being under control of a regulon comprising a promoter and at least either the -1052 to -987 region of the MOX gene given in Fig. 11A or the -1076 to -937 region of the DAS gene given in Fig. 18A of *Hensenula polymorpha* CBS 4732, or a corresponding region of other methylotrophic moulds or yeasts, or an effective modification of any of these regions.

10. Combination of DNA sequences suitable for the transformation of a microbial host to produce a polypeptide and containing a regulon, a structural gene encoding the polypeptide and optionally a terminator, characterized in that a regulon is used selected from the group consisting of at least part of the regulon -1 to about -1500 of the MOX gene given in Fig. 11A or at least part of the regulon of -1 to about -2125 of the DAS gene given in Fig. 18A and modifications thereof that do not impair the regulon function, and optionally a terminator is used selected from the group consisting of at least part of the terminator 1993 to about 3260 of the MOX gene given in Fig. 11B or at least part of the terminator of 2110 to about 2350 of the DAS gene given in Fig. 18B and modifications thereof that do not impair the terminator function.

11. Combination of DNA sequences according to claim 10, characterized in that the regulon comprises a promoter and at least either the -1052 to -987 region of the MOX gene or the -1076 to -937 region of the DAS gene of *Hansenula polymorpha* CBS 4732, or a corresponding region of other methylotrophic moulds or yeasts, or an effective modification of any of these regions.

12. Combination of DNA sequences according to claim 10 or 11, characterized in that it is suitable for transformation of a *Hansenula* yeast, in particular a *Hansenula polymorpha.*

13. Combination of DNA sequences according to claim 10 or 11, characterized in that it is suitable for transformation of a *Saccharomyces* yeast, in particular *Saccharomyces cerevisiae.*

14. Combination of DNA sequences according to claim 10, characterized in that the structural gene coding for said polypeptide contains DNA sequences derived from the structural gene coding for MOX (Fig. 11A + 11B), which modify said polypeptide, without impairing its functions, in such a way that said

23

polypeptide is translocated into the peroxisomes or equivalent microbodies of said microbial host.

Claims for the following Contracting State: AT

1. Process for preparing a polypeptide by culturing a microorganism under such conditions that the microorganism produces the polypeptide, optionally concentrating the polypeptide and collecting same in a manner known *per se,* characterized in that a transformed microorganism is used that carries a structural gene coding for the polypeptide concerned and under the control of a regulon comprising a promoter and at least either the -1052 to -987 region of the MOX gene of *Hansenula polymorpha* CBS 4732 given in Fig. 11A, or the -1076 to -937 region of the DAS gene of *Hansenula polymorpha* CBS 4732 given in Fig. 18A, or a corresponding region of other methylotrophic moulds or yeasts, or a modification of any of these regions that does not impair the regulon function, while the structural gene coding for the polypeptide and the regulon are introduced into the microorganism by means of a vector.

2. Process according to claim 1, characterized in that the promoter is derived from the yeast *Hansenula polymorpha.*

3. Process according to claim 1 or 2, characterized in that the microorganism is a mould or yeast.

4. Process according to claim 3, characterized in that a mould or yeast is selected from the group consisting of the genera *Aspergillus, Candida, Geotrichum, Hansenula, Lenzites, Nadsonia, Pichia, Poria, Polyporus, Saccharomyces, Sporobolomyces, Torulopsis, Trichospora* and *Zendera.*

5. Process according to claim 4, characterized in that the mould or yeast is selected from the species *Aspergillus japonicus, Aspergillus niger, Aspergillus oryzae, Candida boidinii, Hansenula anomala, Hansenula polymorpha, Hansenula wingeii, Kloeckera* sp. 2201 and *Pichia pastoris.*

6. Process according to claim 5, characterized in that the microorganism is the yeast species *Hansenula polymorpha.*

7. Process according to any of claims 1-6, characterized in that the structural gene concerned has been provided with one or more DNA sequences which translocate the gene product into the peroxisomes or equivalent microbodies of the microbial host.

8. Process according to claim 7, characterized in that the DNA sequences concerned consist of the MOX gene or those parts thereof which are responsible for MOX translocation into the peroxisomes or microbodies.

9. Process for producing a transformed microorganism suitable for use in a process as claimed in any one of claims 1-8, in which a structural gene coding for the polypeptide to be prepared is introduced into the microorganism, whereby the expression of the gene is put under control of a regulon comprising a promoter and at least either the -1052 to -987 region of the MOX gene given in Fig. 11A or the -1076 to -937 region of the DAS gene given in Fig. 18A of *Hansenula polymorpha* CBS 4732, or a corresponding region of other methylotrophic moulds or yeasts, or an effective modification of any of these regions.

10. Process for preparing a combination of DNA sequences containing a regulon capable of transforming a microbial host to produce a polypeptide, in which a structural gene encoding the polypeptide is placed in the 5' to 3' direction downstream of the regulon and upstream of an optional terminator, characterized in that the regulon is selected from the group consisting of at least part of the regulon -1 to about -1500 of the MOX gene given in Fig. 11A, at least part of the regulon of -1 to about -2125 of the DAS gene given in Fig. 18A, and modifications thereof that do not impair the regulon function, and optionally the terminator is selected from the group consisting of at least part of the terminator 1993 to about 3260 of the MOX gene given in Fig. 11B, at least part of the terminator of 2110 to about 2350 of the DAS gene given in Fig. 18B, and modifications thereof that do not impair the terminator function.

11. Process according to claim 10, characterized in that the regulon comprises a promoter and at least either the -1052 to -987 region of the MOX gene or the -1076 to -937 region of the DAS gene of *Hansenula polymorpha* CBS 4732, or a corresponding region of other methylotrophic moulds or yeasts, or an effective modification of any of these regions.

12. Process according to claim 10 or 11, characterized in that the combination of DNA sequences is suitable for transformation of a *Hansenula* yeast, in particular a *Hansenula polymorpha*.

13. Process according to claim 10 or 11, characterized in that the combination of DNA sequences is suitable for transformation of a *Saccharomyces* yeast, in particular *Saccharomyces cerevisiae*.

14. Process according to claim 10, characterized in that the structural gene coding for said polypeptide contains DNA sequences derived from the structural gene coding for MOX (Fig. 11A + 11B), which modify said polypeptide, without impairing its functions, in such a way that said polypeptide is translocated into the peroxisomes or equivalent microbodies of said microbial host.


**Revendications**

1. Procédé de préparation d'un polypeptide par culture d'un microorganisme dans des conditions telles que le microorganisme produit le polypeptide, éventuellement concentration du polypeptide et collecte de ce polypeptide de façon connue, caractérisé en ce qu'on utilise un microorganisme transformé qui porte un gène de structure codant pour le polypeptide concerné et sous le contrôle d'un régulon comprenant un promoteur et au moins soit la région -1052 à -987 du gène MOX de Hansenula polymorpha CBS 4732 donné dans la figure 11A, soit la région -1076 à -937 du gène DAS d'Hansenula polymorpha donné dans la figure 18A, soit une région correspondante d'autres moisissures ou levures méthylotrophes, soit une modification de l'une quelconque de ces régions qui n'affecte pas négativement la fonction du régulon, tandis que le gène de structure codant pour le polypeptide et le régulon sont introduits dans le microorganisme au moyen d'un vecteur.

2. Procédé selon la revendication 1, caractérisé en ce que le promoteur est dérivé de la levure Hansenula Polymorpha.

3. Procédé selon la revendication 1 ou 2, caractérisé en ce que le microorganisme est une moisissure ou une levure.

4. Procédé selon la revendication 3, caractérisé en ce que la moisissure ou levure est choisie dans le groupe constitué par les genres Aspergillus, Candida, Geotrichum, Hansenula, Lenzites, Nadsonia, Pichia, Poria, Polyporus, Saccharomyces, Sporobolomyces, Torulopsis, Trichospora et Zendera.

5. Procédé selon la revendication 4, caractérisé en ce que la moisissure ou levure est choisie parmi les espèces Aspergillus japonicus, Aspergillus niger, Aspergillus oryzae, Candida boidinii, Hansenula anomala, Hansenula polymorpha, Hansenula wingeii, Kloeckera sp. 2201 et Pichia pastoris.

6. Procédé selon la revendication 5, caractérisé en ce que le microorganisme est la levure de l'espèce Hansenula polymorpha.

7. Procédé selon l'une quelconque des revendications 1-6, caractérisé en ce que le gène de structure en question a été muni d'une ou plusieurs séquences d'ADN qui transloquent le produit de gène dans les peroxysomes ou microcorpuscules équivalents de l'hôte microbien.

8. Procédé selon la revendication 7, caractérisé en ce que les séquences d'ADN en question se composent du gène MOX ou des parties de ce gène qui sont responsables de la translocation de MOX dans les peroxysomes ou microcorpuscules.

9. Procédé de préparation d'un microorganisme transformé pour utilisation dans un procédé tel que revendiqué dans l'une quelconque des revendications 1-8, dans lequel on introduit un gène de structure codant pour le polypeptide à préparer dans le microorganisme, ce qui fait que l'expression du gène est mise sous le contrôle d'un régulon comprenant un promoteur et au moins soit la région de -1052 à -987 du gène MOX donnée dans la figure 11A, soit la région de -1076 à -937 du gène DAS donnée dans la figure 18A d'Hansenula polymorpha CBS 4732, soit une région correspondante d'autres moisissures ou levures méthylotrophes, soit une modification efficace de l'une quelconque de ces régions.

10. Procédé de préparation d'une combinaison de séquences d'ADN contenant un régulon capable de transformer un hôte microbien pour produire un polypeptide, dans lequel on met un gène de structure encodant le polypeptide dans la direction de 5' à 3' en aval du régulon et en amont d'un terminateur optionnel, caractérisé en ce que le régulon est choisi dans le groupe constitué par au moins une partie du régulon -1 à environ -1500 du gène MOX donné dans la figure 11A, au moins une partie du régulon de -1 à environ -2125 du gène DAS donné dans la figure 18A, et leurs modifications qui n'altèrent pas la fonction du régulon, et éventuellement le terminateur est choisi dans le groupe constitué par au moins une partie du terminateur de 1993 à environ 3260 du gène MOX donné dans la Fig. 11B, au moins une partie du terminateur de 2110 à environ 2350 du gène DAS donné dans la figure 18B, et leurs modifications qui n'affecte pas négativement la fonction du terminateur.

11. Procédé selon la revendication 10, caractérisé en ce que le régulon comprend un promoteur et au moins soit la région de -1052 à -987 du gène MOX, soit la région de -1076 à -937 du gène DAS de Hansenula polymorpha CBS 4732, soit une région correspondante d'autres moisissures ou levures méthylotrophes, soit une modification efficace de l'une quelconque de ces régions.

12. Procédé selon la revendication 10 ou 11, caractérisé en ce que la combinaison de séquences d'ADN convient pour la transformation d'une levure Hansenula, en particulier une Hansenula polymorpha.

13. Procédé selon la revendication 10 ou 11, caractérisé en ce que la combinaison de séquences d'ADN convient pour la transformation d'une levure Saccharomyces, en particulier de Saccharomyces cerevisiae.

14. Procédé selon la revendication 10, caractérisé en ce que le gène de structure codant pour ledit polypeptide contient des séquences d'ADN dérivées du gène de structure codant pour MOX (Fig. 11A + 11B), qui modifient ledit polypeptide, sans affecter négativement ses fonctions, de manière telle que ledit polypeptide est transloqué dans les peroxysomes ou les microcorpuscules équivalents dudit hôte microbien.

Revendications pour l'Etat contractant suivant: AT

1. Procédé de préparation d'un polypeptide par culture d'un microorganisme dans des conditions telles que le microorganisme produit le polypeptide, éventuellement concentration du polypeptide et collecte de ce polypeptide de façon connue, caractérisé en ce qu'on utilise un microorganisme transformé qui porte un gène de structure codant pour le polypeptide concerné et sous le contrôle d'un régulon comprenant un promoteur et au moins soit la région -1052 à -987 du gène MOX de Hansenula polymorpha CBS 4732 donné dans la figure 11A, soit la région -1076 à -937 du gène DAS d'Hansenula polymorpha donné dans la figure 18A, soit une région correspondante d'autres moisissures ou levures méthylotrophes, soit une modification de l'une quelconque de ces régions qui n'affecte pas négativement la fonction du régulon, tandis que le gène de structure codant pour le polypeptide et le régulon sont introduits dans le microorganisme au moyen d'un vecteur.

2. Procédé selon la revendication 1, caractérisé en ce que le promoteur est dérivé de la levure Hansenula polymorpha.

3. Procédé selon la revendication 1 ou 2, caractérisé en ce que le microorganisme est une moisissure ou une levure.

4. Procédé selon la revendication 3, caractérisé en ce que la moisissure ou levure est choisie dans le groupe constitué par les genres Aspergillus, Candida, Geotrichum, Hansenula, Lenzites, Nadsonia, Pichia, Poria, Polyporus, Saccharomyces, Sporobolomyces, Torulopsis, Trichospora et Zendera.

5. Procédé selon la revendication 4, caractérisé en ce que la moisissure ou levure est choisie parmi les espèces Aspergillus japonicus, Aspergillus niger, Aspergillus oryzae, Candida boidinii, Hansenula anomala, Hansenula polymorpha, Hansenula wingeii, Kloeckera sp. 2201 et Pichia pastoris.

6. Procédé selon la revendication 5, caractérisé en ce que le microorganisme est la levure de l'espèce Hansenula polymorpha.

7. Procédé selon l'une quelconque des revendications 1-6, caractérisé en ce que le gène de structure en question a été muni d'une ou plusieurs séquences d'ADN qui transloquent le produit de gène dans les peroxysomes ou microcorpuscules équivalents de l'hôte microbien.

8. Procédé selon la revendication 7, caractérisé en ce que les séquences d'ADN en question se composent du gène MOX ou des parties de ce gène qui sont responsables de la translocation de MOX dans les peroxysomes ou microcorpuscules.

9. Microorganisme transformé approprié pour utilisation dans un procédé tel que revendiqué dans l'une des revendications 1-8, comprenant un gène de structure codant pour le polypeptide à préparer, l'expression du gène étant sous le contrôle d'un régulon comprenant un promoteur et au moins soit la région -1052 à -987 du gène MOX donnée dans la figure 11A, soit la région - 1076 à -937 du gène DAS donnée dans la figure 18A d'Hansenula polymorpha CBS 4732, soit une région correspondante d'autres moisissures ou levures méthylotrophes, soit une modification efficace de l'une quelconque de ces régions.

10. Combinaison de séquences d'ADN appropriées pour la transformation d'un hôte microbien afin de produire un polypeptide et contenant un régulon, un gène de structure encodant le polypeptide et éventuellement un terminateur, caractérisée en ce qu'on utilise un régulon choisi dans le groupe constitué par au moins une partie du régulon -1 à environ -1500 du gène MOX donné dans la figure 11A ou au moins une partie du régulon de -1 à environ -2125 du gène DAS donné dans la figure 18A et de leurs modifications qui ne portent pas atteinte à la fonction du régulon, et éventuellement en ce qu'on utilise un terminateur choisi dans le groupe constitué par au moins une partie du terminateur 1993 à environ 3260 du gène MOX donné dans la Fig. 11B ou au moins une partie du terminateur de 2110 à environ 2350 du gène DAS donné dans la Fig 18B et leurs modifications qui n'affecte négativement la fonction du terminateur.

11. Combinaison de séquences d'ADN selon la revendication 10, caractérisée en ce que le régulon comprend un promoteur et au moins soit la région de -1052 à -987 du gène MOX, soit la région de -1076 à - 937 du gène DAS d'Hansenula polymorpha CBS 4732, soit une région correspondante d'autres moisissures ou levures méthylotrophes, soit une modification efficace de l'une quelconque de ces régions.

12. Combinaison de séquences d'ADN selon la revendication 10 ou 11, caractérisée en ce qu'elle convient pour la transformation d'une levure Hansenula, en particulier d'une Hansenula polymorpha.

13. Combinaison de séquences d'ADN selon la revendication 10 ou 11, caractérisée en ce qu'elle convient pour la transformation d'une levure Saccharomyces, en particulier de Saccharomyces cerevisiae.

14. Combinaison de séquences d'ADN selon la revendication 10, caractérisée en ce que le gène de structure codant pour ledit polypeptide contient des séquences d'ADN dérivées du gène de structure codant pour MOX (Fig. 11A + 11B), qui modifient ledit polypeptide, sans affecter négativement ses fonctions, de manière telle que ledit polypeptide est transloqué dans les peroxysomes ou microcorpuscules dudit hôte microbien.

## Ansprüche

1. Verfahren zur Herstellung eines Polypeptids durch Kultivieren eines Mikroorganismus unter solchen Bedingungen, daß der Mikroorganismus das Polypeptid produziert, fakultatives Konzentrieren des Polypeptids und Sammeln desselben in einer per se bekannten Weise, dadurch gekennzeichnet, daß ein transformierter Mikroorganismus verwendet wird, der ein Strukturgen trägt, das das betreffende Polypeptid kodiert und unter der Kontrolle eines Regulons steht, das einen Promotor und mindestens entweder die -1052 bis -987 Region des in Fig. 11A angegebenen MOX-Gens von Hansenula polymorpha CBS 4732 oder die -1076 bis -937 Region des in Fig. 18A angegebenen DAS-Gens von Hansenula polymorpha CBS 4732 oder eine entsprechende Region anderer methylotropher Schimmelpilze oder Hefen oder eine die Regulonfunktion nicht beeinträchtigende Modifikation irgendeiner dieser Regionen umfaßt, wobei das das Polypeptid kodierende Strukturgen und das Regulon mittels eines Vektors in den Mikroorganismus eingeführt werden.

2. Verfahren gemäß Anspruch 1, dadurch gekennzeichnet, daß der Promotor von der Hefe Hansenula polymorpha abgeleitet wird.

3. Verfahren gemäß Anspruch 1 oder 2, dadurch gekennzeichnet, daß der Mikroorganismus ein Schimmelpilz oder eine Hefe ist.

4. Verfahren gemäß Anspruch 3, dadurch gekennzeichnet, daß ein Schimmelpilz oder eine Hefe aus der Gruppe ausgewählt wird, die aus den Gattungen Aspergillus, Candida, Geotrichum, Hansenula, Lenzites, Nadsonia, Pichia, Poria, Polyporus, Saccharomyces, Sporobolomyces, Torulopsis, Trichospora und Zendera besteht.

5. Verfahren gemäß Anspruch 4, dadurch gekennzeichnet, daß der Schimmel oder die Hefe aus den Spezies Aspergillus japonicus, Aspergillus niger, Aspergillus oryzae, Candida boidinii, Hansenula anomala, Hansenula polymorpha, Hansenula wingii, Kloeckera sp. 2201 und Pichia pastoris ausgewählt wird.

6. Verfahren gemäß Anspruch 5, dadurch gekennzeichnet, daß der Mikroorganismus die Hefe-Spezies Hansenula polymorpha ist.

7. Verfahren gemäß irgendeinem der Ansprüche 1 bis 6, dadurch gekennzeichnet, daß das betreffende Strukturgen mit einer oder mehreren DNS-Sequenzen versehen worden ist, die das Genprodukt in die Peroxisomen oder äquivalente Mikrokörper des mikrobiellen Wirtes translozieren.

8. Verfahren gemäß Anspruch 7, dadurch gekennzeichnet, daß die betreffenden DNS-Sequenzen aus dem MOX-Gen oder solchen Teilen desselben bestehen, die für die MOX-Translokation in die Peroxisomen oder Mikrokörper verantwortlich sind.

9. Transformierter Mikroorganismus, der zur Verwendung in einem Verfahren gemäß einem der Ansprüche 1 bis 8 geeignet ist und ein das Polypeptid kodierendes Strukturgen umfaßt, wobei die Expression des Gens unter der Kontrolle eines Regulons steht, das einen Promotor und mindestens entweder die -1052 bis -987 Region des in Fig. 11A angegebenen MOX-Gens oder die -1076 bis -937 Region des in Fig. 18A angegebenen DAS-Gens von Hansenula polymorpha CBS 4732 oder eine entsprechende Region anderer methylotropher Schimmelpilze oder Hefen oder eine wirksame Modifikation irgendeiner dieser Regionen umfaßt.

10. Kombination von DNS-Sequenzen, die zur Transformation eines mikrobiellen Wirtes zwecks Produktion eines Polypeptids geeignet ist und ein Regulon, ein das Polypeptid kodierendes Strukturgen und fakultativ einen Terminator enthält, dadurch gekennzeichnet, daß ein Regulon verwendet wird, das aus der Gruppe ausgewählt wird, die aus mindestens einem Teil des Regulons von -1 bis etwa -1500 des in Fig. 11A angegebenen MOX-Gens oder mindestens einem Teil des Regulons von -1 bis etwa -2125 des in Fig. 18A angegebenen DAS-Gens und Modifikationen derselben, die die Regulonfunktion nicht beeinträchtigen, besteht und fakultativ ein Terminator verwendet wird, der aus der Gruppe ausgewählt wird, die mindestens aus einem Teil des Terminators von 1993 bis etwa 3260 des in Fig. 11B angegebenen MOX-Gens oder mindestens einem Teil des Terminators von 2110 bis etwa 2350 des in Fig. 18B angegebenen DAS-Gens und Modifikationen derselben, die die Terminatorfunktion nicht beeinträchtigen, besteht.

11. Kombination von DNS-Sequenzen gemäß Anspruch 10, dadurch gekennzeichnet, daß das Regulon einen Promotor und mindestens entweder die -1052 bis -987 Region des MOX-Gens oder die -1076 bis -937 Region des DAS-Gens von Hansenula polymorpha CBS 4732 oder eine entsprechende Region anderer methylotropher Schimmelpilze oder Hefen oder eine wirksame Modifikation irgendeiner dieser Regionen umfaßt.

12. Kombination von DNS-Sequenzen gemäß Anspruch 10 oder 11, dadurch gekennzeichnet, daß sie zur Transformation einer Hansenula-Hefe, insbesondere Hansenula polymorpha, geeignet ist.

13. Kombination von DNS-Sequenzen gemäß Anspruch 10 oder 11, dadurch gekennzeichnet, daß sie zur Transformation einer Saccharomyces-Hefe, insbesondere Saccharomaces cerevisiae, geeignet ist.

14. Kombination von DNS-Sequenzen gemäß Anspruch 10, dadurch gekennzeichnet, daß das dieses Polypeptid kodierende Strukturgen DNS-Sequenzen enthält, die von einem MOX (Fig. 11A + 11B) kodierenden Strukturgen abgeleitet sind, die das Polypeptid ohne Beeinträchtigung seiner Funktionen in solcher Weise modifizieren, daß das Polypeptid in die Peroxisomen oder äquivalente Mikrokörper des mikrobiellen Wertes transloziert werden.

Patentansprüche für folgenden Vertragsstaat: AT

1. Verfahren zur Herstellung eines Polypeptids durch Kultivieren eines Mikroorganismus unter solchen Bedingungen, daß der Mikroorganismus das Polypeptid produziert, fakultatives Konzentrieren des Polypeptids und Sammeln desselben in einer per se bekannten Weise, dadurch gekennzeichnet, daß ein transformierter Mikroorganismus verwendet wird, der ein Strukturgen trägt, das das betreffende Polypeptid kodiert und unter der Kontrolle eines Regulons steht, das einen Promotor und mindestens entweder die -1052 bis -987 Region des in Fig. 11A angegebenen MOX-Gens von Hansenula polymorpha CBS 4732 oder die -1076 bis -937 Region des in Fig. 18A angegebenen DAS-Gens von Hansenula polymorpha CBS 4732 oder eine entsprechende Region anderer methylotropher Schimmelpilze oder Hefen oder eine die Regulonfunktion nicht beeinträchtigende Modifikation irgendeiner dieser Regionen umfaßt, wobei das das Polypeptid kodierende Strukturgen und das Regulon mittels eines Vektors in den Mikroorganismus eingeführt werden.

2. Verfahren gemäß Anspruch 1, dadurch gekennzeichnet, daß der Promotor von der Hefe Hansenula polymorpha abgeleitet wird.

3. Verfahren gemäß Anspruch 1 oder 2, dadurch gekennzeichnet, daß der Mikroorganismus ein Schimmelpilz oder eine Hefe ist.

4. Verfahren gemäß Anspruch 3, dadurch gekennzeichnet, daß ein Schimmelpilz oder eine Hefe aus der Gruppe ausgewählt wird, die aus den Gattungen Aspergillus, Candida, Geotrichum, Hansenula, Lenzites, Nadsonia, Pichia, Poria, Polyporus, Saccharomyces, Sporobolomyces, Torulopsis, Trichospora und Zendera besteht.

5. Verfahren gemäß Anspruch 4, dadurch gekennzeichnet, daß der Schimmel oder die Hefe aus den Spezien Aspergillus japonicus, Aspergillus niger, Aspergillus oryzae, Candida boidinii, Hansenula anomala, Hansenula polymorpha, Hansenula wingii, Kloeckera sp. 2201 und Pichia pastoris ausgewählt wird.

6. Verfahren gemäß Anspruch 5, dadurch gekennzeichnet, daß der Mikroorganismus die Hefe-Spezies Hansenula polymorpha ist.

7. Verfahren gemäß irgendeinem der Ansprüche 1 bis 6, dadurch gekennzeichnet, daß das betreffende Strukturgen mit einer oder mehreren DNS-Sequenzen versehen worden ist, die das Genprodukt in die Peroxisomen oder äquivalente Mikrokörper des mikrobiellen Wirtes translozieren.

8. Verfahren gemäß Anspruch 7, dadurch gekennzeichnet, daß die betreffenden DNS-Sequenzen aus dem MOX-Gen oder solchen Teilen desselben bestehen, die für die MOX-Translokation in die Peroxisomen oder Mikrokörper verantwortlich sind.

9. Verfahren zur Herstellung eines transformierten Mikroorganismus, der zur Verwendung in einem Verfahren gemäß irgendeinem der Ansprüche 1 bis 8 geeignet ist, in welchem ein das herzustellende Polypeptid kodierendes Strukturgen in den Mikroorganismus eingeführt wird, wodurch die Expression des Gens unter die Kontrolle eines Regulons gebracht wird, das einen Promotor und mindestens entweder die -1052 bis -987 Region des in Fig. 11A angegebenen MOX-Gens oder die -1076 bis -937 Region des in Fig. 18A gegebenen DAS-Gens von Hansenula polymorpha CBS 4732 oder eine entsprechende Region anderer methylotropher Schimmelpilze oder Hefen oder eine wirksame Modifikation irgendeiner dieser Regionen umfaßt.

10. Verfahren zur Herstellung einer Kombination von DNS-Sequenzen, die ein Regulon enthalten, das zum Transformieren eines mikrobiellen Wirtes zwecks Produktion eines Polypeptids fähig ist, bei welchen ein das Polypeptid kodierendes Strukturgen in der 5'- oder 3'-Richtung unterhalb des Regulons und oberhalb eines fakultativen Terminators plaziert wird, dadurch gekennzeichnet, daß das Regulon aus der Gruppe ausgewählt wird, die aus mindestens einem Teil des Regulons von -1 bis etwa -1500 des in Fig. 11A angegebenen Mox-Gens, mindestens einem Teil des Regulons von -1 bis etwa -2125 des in Fig. 18A angegebenen DAS-Gens und Modifikationen derselben, die der Regulonfunktion nicht beeinträchtigen, besteht und der Terminator fakultativ aus der Gruppe ausgewählt wird, die aus mindestens einem Teil des Terminators von 1993 bis etwa 3260 des in Fig. 11B angegebenen MOX-Gens, mindestens einem Teil des Terminators von 2110 bis etwa 2350 des in Fig. 18B angegebenen DAS-Gens und Modifikationen derselben, die die Terminatorfunktion nicht beeinträchtigen, besteht.

11. Verfahren gemäß Anspruch 10, dadurch gekennzeichnet, daß das Regulon einen Promotor und mindestens entweder die -1052 bis -987 Region des MOX-Gens oder die -1076 bis -937 Region des DAS-Gens von Hansenula polymorpha CBS 4732 oder eine entsprechende Region anderer methylotropher Schimmelpilze oder Hefen oder eine wirksame Modifikation irgendeiner dieser Regionen umfaßt.

12. Verfahren gemäß Anspruch 10 oder 11, dadurch gekennzeichnet, daß die Kombination von DNS-Sequenzen zur Transformation einer Hansenula-Hefe, insbesondere Hansenula polymorpha, geeignet ist.

13. Verfahren gemäß Anspruch 10 oder 11, dadurch gekennzeichnet, daß die Kombination von DNS-Sequenzen zur Transformation einer Saccharomyces-Hefe, insbesondere Saccharomyces cerevisiae, geeignet ist.

14. Verfahren gemäß Anspruch 10, dadurch gekennzeichnet, daß das dieses Polypeptid kodierende Strukturgen DNS-Sequenzen enthält, die von dem MOX (Fig. 11A + 11B) kodierenden Strukturgen abgeleitet werden, die das Polypeptid ohne Beeinträchtigung seiner Funktionen in solcher Weise modifizieren, daß das Polypeptid in die Peroxisomen oder aquivalente Mikrokörper des mikrobiellen Wirtes transloziert wird.

Fig. 1

Fig. 2

Fig. 3 DNA sequence of the autonomous-replicating sequence HARS1
from the methylotrophic yeast Hansenula polymorpha. The
HARS1 represents a SalI fragment comprising 483 nucleo-
tides. The dideoxy-sequencing method was employed.

```
(G)TCGACTCCG CGACTCGGCG TTCACTTTCG AGCTATTCAT CAACGCCGGA ATACGTCAGA
AACAGCCGTG CCCCAGGGAC CAGAAAGCCT ACTGGTGAGT ATGTTCTTTC GTGTGATTTT
CCGAGGATGA GACGACGATA ACGAGCACAA CTCGGAGTCG GAGGACACGC TTATTGCGTT
GACGAGCCAC ATCAGCAGGC TGTCAAGACT GAGTATAGGC CACAGAGCTG ATTCTGCTCA
TACTCAAGAC GTTAGTAAAC TCCGTCTGCC ACAATGCTGA CAGAGTATTA TAATAATAGT
GAATTACGAA CAATGTAGTC AAAAAAATTT AGTAACAATA TGTCATGATG ACAGATTTGC
TGAAACCAGT GAACTCCAAT AAATCCAGCG GCTACCGCAT CCCAAGAGAA ACAGATCAGA
GGTCTAGGCT TGTTTCAGAG TACTACAAGC TTTCCAGAAC TTAGCAATTC TCAAACGCGG
TTTG(TCGAC)
  ↓
 483
```

Fig. 4

1 2 3 4 5 6 7 8 9 10 11 12 13

Fig. 5

Fig. 6

Fig. 7

Fig. 8

NH2-Ala-Ile-Pro-Asp-Glu-Phe-Asp-Ile-Ile-Val-Val-Gly-

CCA GAC GAA TTC GA
CCA GAT GAA TTC GA

-Gly-Gly- * -Thr-Gly-Cys-Cys-Ile-Ala-Gly- * -Leu-
-Ala-Asn-Leu-Asp-Asp-Gln-Asn-Leu

Fig. 9

Phage λ 47.1　　　　　　　Hansenula DNA　　　　　　　Phage λ 47.1

X　　　　Hg
$E_I$　H　　　　　B　Sl | St　Sp　$E_I E_{\underline{Y}}$　$E_I$　Sl　Kp　$E_{\underline{Y}}$　Sc　　　H　　$E_I$

P　P　P　5'　　　　　3'

open reading frame

M13 subclones

1 kb

Fig. 19

EP 0 173 378 B1

Fig. 11A

```
          CTCGACGCGG AGAACGATCT CCTCGAGCTC CTCCCGGATC AGCTTGTGGC CCGGTAATCG
                    -1501
          AACCAGGCCG ACGCGACGCT CCTTGCGGAC CACGGTGGCT GGGCAGCCCA GTTTGTGAAC
          -1451                                                           -1401
          GAGGTCGTTT AGAACGTCCT GCGCAAAGTC CAGTGTCAGA TCAATGTCCT CCTCGGACCA
                                                               -1351
          ATTCAGCATG TTCTCGAGCA GCCATCTGTC TTTGGAGTAG AAGCGTAATC TCTGCTCCTC
                                                    -1301
          CTTACTCTAC CGGAAGAGGT ACTTTGCCTC GCCGCCCATA ATGAACACGT TCTCTTTCTG
                                 -1251
          GTGGCCTGTG AGCAGCGGGG ACGTCTGGAC GGCGTCGATG AGGCCCTTGA GGCCGCTCGTA
                    -1201
          CTACTTGTTC CGTCGCTGTA GCCCGCCGCG CTGACCATAC CCACATAGAG GTCCTTGGCC
          -1151                                                           -1101
          ATTAGTTTGA TGAGGTGGGG CAGGATGGGC GACTCGGCAT CGAAATTTTT GCCGTCGTCG
                                                               -1051
          TACAGTGTGA TGTCACCATC GAATGTAATG ACCTGCAGCT TGCCGATCTCG GATGGTTTTG
                                                    -1001
          GAATGGAAGA ACCGCGACAT CTCCAACAGC TGGCCCGTGT TGAGAATGAG CCGGACGTCG
                    -951
          TTGAACGAGG GGGCCACAAG CCGGCCGTTTG CTGATGCCGC GGCGGCTCGTC CTCGATGTAC
                    -901
          AAGGCCTTTT CCAGAGGCAG TCTCGTGAAG AAGCTGCCAA CGCTCGGAAC CACCTGCACG
          -851                                                           -801
          AGCCGAGACA ATTCGGGGGT GCCGGCTTTG CTCATTTCAA TGTTGTCGTC GATGAGGAGT
                                                    -751
          TCGAGGTCGT GGAAGATTTC CGCGTAGCGG CGTTTTGCCT CAGAGTTTAC CATGAGGTCG
                                                    -701
          TCCACTGCAG AGATGCCGTT GCTCTTCACC GCGTACAGGA CGAACGGCGT GCCCAGCAGG
                                 -651
          CCCTTGATCC ATTCTATGAG GCCATCTCGA CGGTGTTCCT TGAGTGCGTA CTCCACTCTG
                    -601
          TAGCCACTGC ACATCTCGAG ACTGGGCTTG CTCTGCTGGA TGCACCAATT AATTGTTGCC
          -551                                                           -501
          GCATGCATCC TTCCACCGCA AGTTTTTAAA ACCCACTCGC TTTAGCCGTC GCGTAAAACT
                                                    -451
          TGTGAATCTG GCAACTGAGG GGGTTCTGCA CCCGCAACCG AACTTTTCGC TTCGAGGACG
                                 -401
          CAGCTGGATG GTGTCATGTG AGGCTCTGTT TGCTGGCGTA GCCTACAACG TGACCTTGCC
                    -351
          TAACCGGACG GCGCTACCCA CTGCTGTCTG TGCCTGCTAC CAGAAAATCA CCACAGCAGC
                    -301
          AGAGGGCCGA TGTGGCAACT GGTGCGGTGT CGGACAGGCT GTTTCTCCAC ACTGCAAATG
          -251                                                           -201
          CGGGTGAACC GGCCACAAAG TAAATTCTTA TGCTACCGTG CAGCGGACTCC GACATCCCCA
                                                    -151
          CTTTTTGCCC TACTTGATCA CAGATGGGCT CAGCGCTGCC GCTAACTGTA CCCAACCGTC
                                                    -101
          CCCACACGGT CCATCTATAA ATACTGCTGC CAGTGCACGG TGCTGACATC AATCTAAAGT
                                 -51
```

```
              1                5                     10             15
           MET ALA ILE PRO ASP GLU PHE ASP ILE ILE VAL VAL GLY GLY GLY SER THR
ACAAAAACAAA ATG GCC ATT CCT GAC GAA TTC GAT ATC ATT GTT GTT GGT GGA GGT TCC ACC
-11
              20               25                    30             35
           GLY CYS CYS ILE ALA GLY ARG LEU ALA ASN LEU ASP ASP GLN ASN LEU THR VAL ALA LEU
           GGC TGC TGC ATT GCG GGC AGA CTC GCA AAC CTC GAC GAC CAA AAC CTC ACA GTT GCC CTG
              40               45                    50             55
           ILE GLU GLY GLY GLU ASN ASN ILE ASN ASN PRO TRP VAL TYR LEU PRO GLY VAL TYR PRO
           ATC GAG GGT GGT GAG AAC AAC ATC AAC AAC CCT TGG GTC TAC CTT CCC GGA GTG TAT CCT
              60               65                    70             75
           ARG ASN MET ARG LEU ASP SER LYS THR ALA THR PHE TYR SER SER ARG PRO SER LYS ALA
           AGA AAC ATG AGA CTC GAC TCC AAG ACG GCC ACC TTC TAC TCG TCC AGA CCA TCG AAG GCT
              80               85                    90             95
           LEU ASN GLY ARG ARG ALA ILE VAL PRO CYS ALA ASN ILE LEU GLY GLY GLY SER SER ILE
           CTG AAC GGC AGA AGA GCG ATC GTT CCT TGC GCC AAC ATC CTT GGA GGC GGC TCG TCC ATC
              100              105                   110            115
           ASN PHE LEU MET TYR THR ARG ALA SER ALA SER ASP TYR ASP ASP TRP GLU SER GLU GLY
           AAC TTT CTG ATG TAC ACC AGA GCC TCT GCT TCC GAC TAC GAC GAC TGG GAG TCC GAG GGA
              120              125                   130            135
           TRP SER THR ASP GLU LEU LEU PRO LEU ILE LYS LYS ILE GLU THR TYR GLN ARG PRO CYS
           TGG AGC ACC GAC GAG TTG CTA CCT CTG ATC AAA AAA ATC GAA ACT TAC CAG CGT CCT TGC
              140              145                   150            155
           ASN ASN ARG ASP LEU HIS GLY PHE ASP GLY PRO ILE LYS VAL SER PHE GLY ASN TYR THR
           AAC AAC AGA GAT CTC CAC GGC TTT GAC GGC CCA ATC AAG GTT TCC TTT GGA AAC TAC ACG
              160              165                   170            175
           TYR PRO THR CYS GLN ASP PHE LEU ARG ALA ALA GLU SER GLN GLY ILE PRO VAL VAL ASP
           TAT CCT ACG TGC CAG GAC TTC CTG AGA GCA GCA GAG TCG CAG GGA ATT CCT GTT GTG GAC
              180              185                   190            195
           ASP LEU GLU ASP PHE LYS THR SER HIS GLY ALA GLU HIS TRP LEU LYS TRP ILE ASN ARG
           GAC CTC GAG GAC TTC AAG ACA TCG CAT GGT GCA GAG CAC TGG CTC AAG TGG ATT AAC AGA
              200              205                   210            215
           ASP LEU GLY ARG ARG SER ASP SER ALA HIS ALA TYR VAL HIS PRO THR MET ARG ASN LYS
           GAC CTC GGC AGA AGA TCG GAT TCT GCG CAC GCC TAC GTC CAC CCA ACT ATG AGA AAC AAG
              220              225                   230            235
           GLN SER LEU PHE LEU ILE THR SER THR LYS CYS ASP LYS VAL ILE ILE GLU ASP GLY LYS
           CAG AGC CTG TTC CTC ATC ACC TCC ACC AAG TGT GAC AAG GTG ATC ATC GAG GAC GGC AAG
              240              245                   250            255
           ALA VAL ALA VAL ASN THR VAL PRO MET LYS PRO LEU ASN PRO LYS LYS PRO VAL SER ARG
           GCT GTG GCC GTG ACA ACA GTG CCA ATG AAG CCT CTG AAC CCT AAG AAG CCT GTG TCC AGA
              260              265                   270            275
           THR PHE ARG ALA ARG LYS GLN ILE VAL ILE SER CYS GLY THR ILE SER SER PRO LEU VAL
           ACC TTC AGA GCC AGA AAG CAG ATT GTG ATC TCC TGC GGA ACC ATC TCG TCT CCT CTG GTG
              280              285                   290            295
           LEU GLN ARG SER GLY ILE GLY ALA ALA HIS HIS LEU ARG SER VAL GLY VAL LYS PRO ILE
           CTC CAG AGA TCT GGT ATT GGT GCA GCT CAC CAC TTG AGA TCC GTG GGG GTC AAG CCA ATC
```

41

Fig. 11B

```
        300                     305                     310                     315
VAL ASP LEU PRO GLY VAL GLY GLU ASN PHE GLN ASP HIS TYR CYS PHE PHE THR PRO THR
GTC GAC CTG CCA GGT GTG GGT GAG AAT TTC CAG GAC CAC TAC TGT TTC TTC ACT CCA TAC
        320                     325                     330                     335
TYR VAL LYS PRO ASP VAL PRO THR PHE ASP ASP PHE VAL ARG GLY ASP PRO VAL ALA GLN
TAC GTC AAG CCT GAC GTT CCT ACG TTC GAC GAC TTT GTC AGG GGC GAC CCA GTT GCC CAG
        340                     345                     350                     355
LYS ALA ALA PHE ASP GLN TRP TYR SER ASN LYS ASP GLY PRO LEU THR THR ASN GLY ILE
AAG GCC GCT TTC GAC CAG TGG TAC TCC AAC AAG GAC GGT CCA TTG ACC ACC AAC GGT ATT
        360                     365                     370                     375
GLU ALA GLY VAL LYS ILE ARG PRO THR GLU GLU GLU LEU ALA THR ALA ASP GLU ASP PHE
GAA GCC GGA GTC AAG ATC ACA CCT ACC GAA GAG GAG CTG GCT ACC GCC GAC GAG GAC TTC
        380                     385                     390                     395
ARG ARG GLY TYR ALA GLU TYR PHE GLU ASN LYS PRO ASP LYS PRO LEU MET HIS TYR SER
AGA CGC GGC TAC GCA GAG TAC TTC GAG AAC AAG CCA GAC AAG CCT CTC ATG CAC TAC TCT
        400                     405                     410                     415
VAL ILE SER GLY PHE PHE GLY ASP HIS THR LYS ILE PRO ASN GLY LYS PHE MET THR MET
GTC ATC TCC GGC TTC TTT GGA GAC CAC ACC AAG ATT CCT AAC GGC AAG TTC ATG ACC ATG
        420                     425                     430                     435
PHE HIS PHE LEU GLU TYR PRO PHE SER ARG GLY PHE VAL ARG ILE THR SER ALA ASN PRO
TTC CAC TTC CTG GAG TAT CCA TTC TCC AGA GGA TTT GTT AGA ATC ACC TCG GCA AAC CCA
        440                     445                     450                     455
TYR ASP ALA PRO ASP PHE ASP PRO GLY PHE LEU ASN ASP GLU ARG ASP LEU TRP PRO MET
TAC GAC GCT CCT GAC TTC GAT CCC GGC TTC CTC AAT GAC GAA AGA GAC CTG TGG CCT ATC
        460                     465                     470                     475
VAL TRP ALA TYR LYS LYS SER ARG GLU THR ALA ARG ARG MET GLU SER PHE ALA GLY GLU
GTC TGG GCA TAC AAG AAG TCC AGA GAG ACG GCC AGA ATG GAG AGC TTT GCA GGA GAG
        480                     485                     490                     495
VAL THR SER MET HIS PRO LEU PHE LYS VAL ASP SER PRO ALA ARG ALA ARG ASP LEU ASP
GTC ACC TCG CAC CAC CCA TTG TTC AAG GTT GAC TCG CCA GCC AGA GCC AGA GAC CTG GAC
        500                     505                     510                     515
LEU GLU THR CYS SER ALA TYR ALA GLY PRO LYS HIS LEU THR ALA ASN LEU TYR HIS GLY
CTC GAG ACA TGC AGT GCA TAT GCC GGT CCT AAG CAC CTC ACT GCC AAC CTG TAC CAC GGC
        520                     525                     530                     535
SER TRP THR VAL PRO ILE ASP LYS PRO THR PRO LYS ASN ASP PHE HIS VAL THR SER ASN
TCG TGG ACC GTT CCT ATC GAC AAG CCA ACC CCT AAG AAC GAT TTC CAC GTG ACC TCC AAC
        540                     545                     550                     555
GLN VAL GLN LEU HIS SER ASP ILE GLU TYR THR GLU GLU ASP HIS GLU ALA ILE VAL ASN
CAA GTC CAA CTG CAC TCC GAC ATC GAG TAC ACC GAG GAG GAC CAC GAG GCC ATC GTC AAC
        560                     565                     570                     575
TYR ILE LYS GLU HIS THR GLU THR THR TRP HIS CYS LEU GLY THR CYS SER MET ALA PRO
TAC ATT AAG GAA CAC ACC GAG ACC ACT TGG CAC TGT CTC GGT ACC TGC TCG ATG GCC CCA
        580                     585                     590                     595
ARG GLU GLY SER LYS ILE ALA PRO LYS GLY GLY VAL LEU ASP ALA ARG LEU ASN VAL TYR
AGA GAG GGT ACT AAG ATT GCT CCT AAG GGA GGT GTC TTG GAC GCC AGA CTC AAC GTT TAC
        600                     605                     610                     615
GLY VAL GLN ASN LEU LYS VAL ALA ASP LEU SER VAL CYS PRO ASP ASN VAL GLY CYS-ASN
GGA GTC CAG AAC CTC AAG GTT GCG GAC CTT TCT GTT TGT CCC GAC AAC GTT GGA TGC AAC
        620                     625                     630                     635
THR TYR SER THR ALA LEU THR ILE GLY GLU LYS ALA ALA THR LEU VAL ALA GLU ASP LEU
ACC TAC TCT ACT GCA TTG ACC ATC GGT GAG AAG GCT GCC ACT CTT GTT GCT GAA GAT CTT
        640                     645                     650                     655
GLY TYR SER GLY SER ASP LEU ASP MET THR ILE PRO ASN PHE ARG LEU GLY THR TYR GLU
GGC TAC TCA GGC TCC GAC CTG GAC ATG ACG ATT CCA AAC TTC AGA CTC GGA ACT TAC GAG
        660
GLU THR GLY LEU ALA ARG PHE ***
GAG ACC GGA CTT GCC AGA TTC TAA GGAG ACCTGGAACG ACATACCCCT TTTGACAAGC
                                               2000
        CTGTTTGAAA ATAGTTCTTT TTCTGGTTTA TATCGTTTAT GAACTGATGA GATGAAAAGC
                     2050
        TGAAATAGCG AGTATACGAA AATTTAATGA AAATTAAATT AAATATTTTC TTACGCTATT
                2100
        AGTCACCTTC AAAATGCCCG CCGCTTCTAA GAACGTTGTC ATGATCGACA ACTACGACTC
2150                                                                2200
        GTTTACCTGG AACCTCTACC AGTACCTGTG TCAGGAGGGA GCCAATGTCG AGGTTTTCAG
                                               2250
        GAACGATCAG ATCACCATTC CGGACATTGA GCAGCTCAAG CCCGACCTTG TGGTGATATC
                                2300
        CCCTGGTCCT GGCCATCCAA GAACAGACTC GGGAATATCT CGCGACGTGA TCAGCCATTT
                     2350
        TAAAGGCAAG ATTCCTGTCT TTGGTGTCTG TATGGGCCAG CACTGTATCT TCCAGGACTT
                2400
        TGGCGGAGAC GTCGAGTATG CGGGCGACAT TGTCCATGGA AAAACGTCCA CTGTTAAGCA
2450                                                                2500
        CGACAACAAG GGAATGTTCA AAAACGTTCC GCAAGATGTT GCTGTCACCA GATACCACTC
                                               2550
        GCTGCCCGGA ACGCTCAAGT CGCTTCCGGA CTGTCTAGAG ATCACTGCTC GCACACACAA
                                2600
        CGGGATCATT ATGGGTGTGA GACACAAGAA GTACACCATC GACGCCGTCC AGTTTCATCC
                     2650
        AGAGAGCATT CTGACCGAGG AGGGCCATCT GATGATCCAG AATATCCTCA ACGTTTCCGG
                ?700
        TGGTTACTGG GAGGAAAATG CCAACGGCGC GGCTCAGAGA AAGGAAAGCA TATTGGACAA
2750                                                                2800
        AATATACGCG CAGAGACGAA AAGACTACGA GTTTGACATG AACAGACCGG GGCGGCAGATT
                                               2850
        TGCTGATCTA GAACTGTACT TGTCCATGGC ACTGCACCGC CGCTAATCAA TTTTTACGAC
                                2900
        AGATTGGAGC AGAACATCAG CGCCCGCCAAG GTTGCAATTC TCAGCGAAAT CAAGAGACCG
                     2950
        TCGCCTTCTA AAGCCCGTCAT CGACCGACAG GCTAACGCTG CCAAACAGGC CCTCAACTAC
                3000
        CCCAAGGCTG GAGTTGCCCAC AATTTCTGTT TTGACCGAGC CAACCTGGTT TAAAGGGAAAT
3050                                                                3100
        ATCCAGGACC TGGAGGTCGC CACAAAAGCCC ATTGACTCTG TGGCCAATAG ACCGTGTATT
                                               3150
        TTGCCGGAAGG AGTTTATCTT CAACAAGTAC CAAATTCTAG AGGCCCGACT GCCGGGAGCCA
                                3200
        GACACGGTTC TGCTGATTGT CAAGATGCTG ACCTC
                3250
```

Fig. 12A

λ MOX 4 (fig. 6)        Transposon Tn 5        M13 mp 9

↓ Sal I              ↓ Sal I           ↓ Sal I alkaline phosphatase

isolate 2.4 kb fragment

↓ Xma III       ↓ Xma III

isolate 380 bp fragment    isolate 1.1 kb fragment    alcohol precipitation

↓ T4 ligase

pUR 3101

Xma III   Sma I
Sal I      Sal I
Hin dIII  pr. MOX  NEO

λ MOX 4 (fig. 6)

5' TGGC—NEO3—NEO7—CTCCGGCCGCTTG 3'
3' ACGTGCCA———————GAGGCCGGCGAACTTAA 5'
  HgiAl   NEO 6  NEO 8    Xma III    EcoRI

M13 mp 9
↓ Sal I  EcoRI
isolate vector

↓ Sal I

isolate 2.4 kb fragment

↓ phosphorylate NEO 6 and 7 with T4 polynucleotide kinase and rATP

↓ Hgi Al

↓ NEO 3, 6, 7 and 8 T4 ligase

isolate 86 bp fragment

isolate 1.5 kb fragment

↓ T4 polynucleotide kinase and rATP

↓ T4 ligase

pUR 3102

Xma III   HgiAl
Sal I     Xma III
Hin dIII  pr. MOX  EcoRI
          NEO

Fig. 12B

Promoter MOX-Neomycinphosphotransferase adaptor fragments

NEO3  5'CGGTGGTGACATCAATCTAAAGTACAAA 3'

NEO6  5'TCATTTTGTTTTTGTACTTTAGATTGATGTCACCACCGTGCA 3'

NEO7 5'AACAAAATGATTGAACAAGATGGATTGCACGCAGGTTCTCCGGCCGCTTG 3'

NEO8 5'AATTCAAGCGGCCGGAGAACCTGCGTGCAATCCATCTTGTTCAA 3'

Fig. 120

<u>Fig. 12</u>

```
<--------------- PROMOTER MOX/AAO ADAPTOR-------------------->>
    -34                                      1
   CGGTGG TGACATCAAT CTAAAGTACA AAAACAAAAT GAGAGTTGTC GTTATTGGTG
   ACGTGCCACC ACTGTAGTTA GATTTCATGT TTTTGTTTTA CTCTCAACAG CAATAACCAC
   HgiaI                                     Met


<<--------------->
                                          62
   CCGGTGTCAT CGGTCTGTCG ACCGCCCTGT GTATCCACGA GAGATACCAC TCCGTTCTGC
   GGCCACAGTA GCCAGACAGC TGGCGGGACA CATAGGTGCT CTCTATGGTG AGGCAAGACG
                  . SalI


                                          122
   AGCCTCTGGA CGTTAAGGTC TACGCCGACA GATTCACCCC TTTCACCACC ACCGACGTTG
   TCGGAGACCT GCAATTCCAG ATGCGGCTGT CTAAGTGGGG AAAGTGGTGG TGGCTGCAAC


                                          182
   CCGCCGGTCT GTGGCAGCCT TACACCTCCG AGCCTTCCAA CCCTCAGGAG GCCAACTGGA
   GGCGGCCAGA CACCGTCGGA ATGTGGAGGC TCGGAAGGTT GGGAGTCCTC CGGTTGACCT


                                          242
   ACCAGCAGAC CTTCAACTAC CTCCTCTCCC ACATCGGTTC GCCTAACGCC GCCAACATGG
   TGGTCGTCTG GAAGTTGATG GAGGAGAGGG TGTAGCCAAG CGGATTGCGG CGGTTGTACC


                                          302
   GTCTGACCCC TGTCTCGGGT TACAACCTGT TCAGAGAGGC CGTTCCTGAC CCTTACTGGA
   CAGACTGGGG ACAGAGCCCA ATGTTGGACA AGTCTCTCCG GCAAGGACTG GGAATGACCT


                                          362
   AGGACATGGT CCTCGGTTTC AGAAAGCTTA CCCCTAGAGA GCTGGACATG TTCCCTGACT
   TCCTGTACCA GGAGCCAAAG TCTTTCGAAT GGGGATCTCT CGACCTGTAC AAGGGACTGA
                        HindIII


                                          422
   ACAGATACGG TTGGTTCAAC ACCTCCCTGA TCCTGGAGGG TAGAAAGTAC CTGCAGTGGC
   TGTCTATGCC AACCAAGTTG TGGAGGGACT AGGACCTCCC ATCTTTCATG GACGTCACCG


                                          482
   TGACCGAGAG ACTGACCGAG AGAGGTGTTA AGTTCTTCCT GAGAAAGGTC GAGTCCTTCG
   ACTGGCTCTC TGACTGGCTC TCTCCACAAT TCAAGAAGGA CTCTTTCCAG CTCAGGAAGC


                                          542
   AGGAGGTTGC CAGAGGTGGT GCCGACGTCA TCATCATGTG TACCGGTGTC TGGGCCGGTG
   TCCTCCAACG GTCTCCACCA CGGCTGCAGT AGTAGTACAC ATGGCCACAG ACCCGGCCAC


                                          602
   TCCTGCAGCC TGACCCTCTG CTGCAGCCCG GGAGAGGTCA GATCATTAAG GTTGACGCCC
   AGGACGTCGG ACTGGGAGAC GACGTCGGGC CCTCTCCAGT CTAGTAATTC CAACTGCGGG
                            XmaI


                                          662
   CATGGCTGAA GAACTTCATC ATTACCCACG ACCTGGAGAG AGGTATCTAC AACTCCCCTT
   GTACCGACTT CTTGAAGTAG TAATGGGTGC TGGACCTCTC TCCATAGATG TTGAGGGGAA


                                          722
   ACATTATCCC TGGTCTGCAG GCCGTCACCC TGGGTGGTAC CTTCCAGGTC GGTAACTGGA
   TGTAATAGGG ACCAGACGTC CGGCAGTGGG ACCCACCATG GAAGGTCCAG CCATTGACCT
                                             KpnI


                                          782
   ACGAGATCAA CAACATCCAG GACCACAACA CCATCTGGGA GGGTTGTTGT AGACTGGAGC
   TGCTCTAGTT GTTGTAGGTC CTGGTGTTGT GGTAGACCCT CCCAACAACA TCTGACCTCG


                                          842
   CTACCCTGAA GGACGCCAAG ATCGTTGGTG AGTACACCGG TTTCAGACCT GTTAGACCTC
   GATGGGACTT CCTGCGGTTC TAGCAACCAC TCATGTGGCC AAAGTCTGGA CAATCTGGAG


                                          902
   AGGTCAGACT GGAGAGAGAG CAGCTGAGAT TCGGTTCCTC CAACACCGAG GTCATTCACA
   TCCAGTCTGA CCTCTCTCTC GTCGACTCTA AGCCAAGGAG GTTGTGGCTC CAGTAAGTGT


                                          962
   ACTACGGTCA CGGTGGTTAC GGTCTGACCA TCCACTTGGG TTGTGCCCTG GAGGTTGCCA
   TGATGCCAGT GCCACCAATG CCAGACTGGT AGGTGAACCC AACACGGGAC CTCCAACGGT


                                          1022
   AGCTGTTCGG TAAGGTCCTG GAGGAGAGAA ACCTGCTGAC CATGCCTCCA TCCCACCTGT
   TCGACAAGCC ATTCCAGGAC CTCCTCTCTT GGACGACTG GTACGGAGGT AGGGTGGACA
                                                               *


   GAG
   CTCAGCT
   **SalI
```

Fig. 14A

Fig. 14

Fig. 14C

```
                         <------PROMOTER MOX-HGRF ADAPTOR------->>
                           -34
                              CGGTG GTGACATCAA TCTAAAGTA CAAAAACAAA
                           ACGTGCCAC CACTGTAGTT AGATTTCAT GTTTTTGTTT
                           HgiAI

     <<------------------------------------------------------------------------------------>>
     1
     ATGTACGCCG ACGCCATCTT CACCAACTCC TACAGAAAGG TTCTGGGTCA GCTCTCGGCC
     TACATGCGGC TGCGGTAGAA GTGGTTGAGG ATGTCTTTCC AAGACCCAGT CGAGAGCCGG
     Met

     --->
     61
     AGAAAGCTTC TGCAGGACAT CATGTCGAGA CAGCAGGGTG AGTCCAACCA GGAGAGAGGT
     TCTTTCGAAG ACGTCCTGTA GTACAGCTCT GTCGTCCCAC TCAGGTTGGT CCTCTCTCCA
       HindIII PstI

     121
     GCCAGAGCCA GACTGTGAG
     CGGTCTCGGT CTGACACTCA GCT                         Fig. 15
                        *** SalI
```

EP 0 173 378 B1

Fig. 16A

Fig. 16B

pURS 528-03        pUR 3203

| Hpa I          |  Sal I
| Sal I          |

isolate Leu 2
containing
fragment

T4 ligase

klenow polymerase

T4 ligase

pUR 3204

Hgi AI        Hin dIII
HGRF
Hin dIII
pr.MOX
Leu 2
Eco RI
MOX TERM
Eco RI        Sac I

Sac I

transformation

Fig. 16C

pHARS I                          pUR 3203

↓ EcoRI                          ↓ EcoRI
  partial Hindlll                  partial Hindlll

isolate                          isolate 4kb fragment
linearized plasmid               containing pr.MOX,HGRF and
containing HARS I                MOX TERM

| T4 ligase

PvuII ▽

pUR 3205

URA3        AmpR

EcoRI
Sac I

Sal I
Hin dlll
Sal I
Bam HI    Hin dlll    Hgi Al

HARS
MOX pr.
HGRF
MOX TERM

Sal I
Hin dlll

Fig. 16D

Fig. 16E

p HARS I                                    pUR 3209

│ partial *Hin* dIII                        │ partial *Hin* dIII
│ *Eco* RI                                   │ partial *Eco* RI
▼                                           ▼

isolate                            isolate 4kb fragment
linearized plasmid                 containing pr.MOX, MOX,
containing HARS I                  HGRF and MOX TERM

                        │ T4 ligase
                        ▼

*Pvu* II
URA 3
Amp R
pUR 3210
*Sal* I
*Hin* dIII
*Sal* I
*Bam* HI
*Hin* dIII  *Sph* I
HARS
pr.MOX MOX HGRF MOX TERM
*Eco* RI
*Eco* RI  *Kpn* I
*Eco* RI *Sac* I
*Kpn* I
*Hin* dIII

Fig. 16F

pURS 528-03        pUR 3209

| Hpa I | partial Kpn I |
| Sal I | klenow |
| klenow | polymerase |
| polymerase | |

isolate Leu 2
containing fragment

T4 ligase

pUR 3211

transformation

```
1
CATGTACGCCG ACGCCATCTT CACCAACTCC TACAGAAAGG TTCTGGGTCA GCTCTCGGCC
CATGGTACATGCGGC TGCGGTAGAA GTGGTTGAGG ATGTCTTTCC AAGACCCAGT CGAGAGCCGG
KpnI Met

61
AGAAAGCTTC TGCAGGACAT CTGTTCGAGA CAGCAGGGTG AGTCCAACCA GGAGAGAGGT
TCTTTCGAAG ACGTCCTGTA GACAAGCTCT GTCGTCCCAC TCAGGTTGGT CCTCTCTCCA
    HindIII PstI           cys

121
GCCAGAGCCA GACTGTGAGGTAC
CGGTCTCGGT CTGACACTC
                *** KpnI
```

Fig. 17

EP 0 173 378 B1

Fig. 18A

```
                                                                    G GATCCACCTG
                                                                      -2125
CTTGGCCAAT GATTCAGCTG CTGGACCGAA AACGCCTCTT TTGGCCAAAA AAAGCCCACC
           -2104
GTTGATAACT GCGGAGGCCA TATTTCAAAG AACAGCGAAT AAGAAAAAAA GGTGAATGAA
-2054                                                             -2004
ATGCGCGAAA CGATACCACT TATTAGCATA AACAAAAAAA AAAAAAATCT ATTAGCTGTT
                                            -1954
ATTATAATTA GTTCAATAAT TTCATAAGCA TCATGGTTGG GCGGCCTATT GTCATCAGTG
                                 -1904
GTCCCTCTGG AACAGGTAAA TCCACTTTGC TGAAGAAGCT GTTTGCTGAG TTCCCAGACA
                      -1854
AGTTTGGATT TTCCGTGTCC AACACCACGA GAAAACCTAG ACCTGGTGAA AAAGACGGTG
           -1804
TCGATTACCA CTTCACCACG GTAGAGGACT TCAAGAAGAT GATTGAAGAA AACAAATTCA
-1754                                                             -1704
TTGAATGGGC CCAGTTCTCC GGCAACTACT ACGGCACCTC TGTGAAAGCT GTGCAAGACG
                                            -1654
TGGCCGAAGT GATGAAGAGA ACGTGTATTT TGGACATTGA TATGCAGGGT GTCAAGAGCG
                                 -1604
TCAAGAAGAC CAACCTGGGA GCCCGATTCC TCTTTATTTC TCCTCCGTCC ATCGAAGAGC
                      -1554
TCAAGAAGAG GCTCGAGAGC CGTGGAACAG AGACCCCTGA ATCTCTTGCC AAGCGGCTTG
           -1504
CTGCTGCATC TGCGGAGATG GAGTACGCCA GGGCAGTGGA CACGACAAGG TCATTGTCAA
-1454                                                             -1404
CGATGACCTT GAGAAGGCGT ACTCTGAGCT GAAGGAGTTC ATTTTCGCCG AGCCCATCTA
                                            ·-1354
AGCATTCATA AATTTTTAAT ATCTAGAGCT CTCATACGGG ACAGTATCTC CTCCAACCTT
                                 -1304
GCGTCAAGCT TGTCCTCTTC ATGCTCCTCA ACAGTCATGG CATCCAGCTG CTGCTGCTTT
                      -1254
TGCTCCAGCC TGGCATATAT GTCGCCATAC AGCTTGAGTT GGATTTTGAT GAAACTCTCA
                                 -1204
AAGGTAGGGT CCACCAGTGA CAGTCGCAGC GCAATGAACT GCTCGATTTC GTTCTTGAGC
-1154                                                             -1104
CGTGTGTTGA TGTCCGTGTA GATATTTTCT GCCTCGTCGT ACTCAACTTT GAACTTCTGC
                                            -1054
AGCTTGTCCA GGCTCTTCTG TAACTGGTCT GTTTTCTCGG TGTGATGCTG CTCGGTCACC
                      -1004
TGTCGCTCAA TCGCTTCGTA CTCGCTCTGC AGCTTCGAAA GCTTGAATCG TGAAACGTCG
           -954
TAATCCACCT TTTTGCGTGC GCGCTTCTTG ATCAGCTTGT TGATCTCGTC GTTGTACTTC
           -904
TTCAGCTCGT TAATCGGCTC CACGACCGTG ATGCTCATTG GCTCCAGAAT TTCTGGCAGA
-854                                              ·               -804
ATATTGTCTT TGATGTCTTC CACCATCTGC AGATAATTCA GAGAAATACC ATCTCTGGGG
                                            -754
TTCACCTTGT GCTCTTCTGG CCGTTCCGCA GCTTCCGACC GCTTATCAGC CTTGAGCTCA
                      -704
AAGCTATAGT CTCCGTAAAA CGAGTCCAGT GTTCTAGCCA TATTTATCTG AGTCTCGAGC
                      -654
AGATTCTCCG AAAATTGCCCA CAAAACGGCC TAGTTCCTGG TCCAGCTCGT TGGTGTAAGT
           -604
CTCGAGTTTG CGGAAATTGG CCTCCTGGAC GTCAAACTCA GGATCAACAG AGGGCTCACC
-554                                                             -504
TTTGTTTGTG CGTAGTATCA CATGTGCTCC GGCACGATTG ACAGCTTTTT TAAACCCAAC
                                 -454
CCATGACATG TCGAGGAAAG GGTCGTTTCG GGGAGTTAAA TATTTTTGGC TATGTAGCAG
                      -404
ACATGTTTCG ACGCTGCCGT CGCGTCGATC GGAAAATATT ACCCCAGGAA CAAGCACTTG
                 -354
CTTGGGTTAG CCACCACCCT GCGCAAGCCT TTTTGCCGGC TCTACACAGG GCCAATGAAA
           -304
TCTGGGCGGA ATCTGAAACC GATGAAACGG ACGACACTGG CAACAAGCTC ACTGCACTAT
-254                                                             -204
```

58

Fig. 183

```
TTTTTTTTTC TAGTGAAATA GCCTATCCTC GTCTCGCTCC CCTCATACCT GTAAAGGGGT
                                                            -154
GCAATTTAGC CTCGTTCCAG CCATTCACGG GCCACTCAAC AACACGTCGG CTACCATGGG
                                                            -104
GTGCTTGGGC ACCAAAAGGC CTATAAATAG GCCCCCATCC GTCTGCTACA CAGTCATCTC
                                        -54
                    1              5                    10                   15
                    MET SER MET ARG ILE PRO LYS ALA ALA SER VAL ASN ASP GLU GLN HIS
TGTCTTTTCTTCCC ATG AGT ATG AGA ATC CCT AAA GCA GCG TCG GTC AAC GAC GAA CAA CAC
-14
            20                    25                    30                   35
GLN ARG ILE ILE LYS TYR GLY ARG ALA LEU VAL LEU ASP ILE VAL GLU GLN TYR GLY GLY
CAG AGA ATC ATC AAG TAC GGT CGT GCT CTT GTC CTG GAC ATT GTC GAG CAG TAC GGA GGA
            40                    45                    50                   55
GLY HIS PRO GLY SER ALA MET GLY ALA MET ALA ILE GLY ILE ALA LEU TRP LYS TYR THR
GGC CAC CCG GGC TCG GCC ATG GGC GCC ATG GCT ATC GGA ATT GCT CTG TGG AAA TAC ACC
            60                    65                    70                   75
LEU LYS TYR ALA PRO ASN ASP PRO ASN TYR PHE ASN ARG ASP ARG PHE VAL LEU SER ASN
CTG AAA TAT GCT CCC AAC GAC CCT AAC TAC TTC AAC AGA GAC AGG TTT GTC CTG TCG AAC
            80                    85                    90                   95
GLY HIS VAL CYS LEU PHE GLN TYR ILE PHE GLN HIS LEU TYR GLY LEU LYS SER MET THR
GGT CAC GTG TGT CTG TTC CAG TAT ATC TTC CAG CAC CTG TAC GGT CTC AAG TCG ATG ACC
            100                   105                   110                  115
MET ALA GLN LEU LYS SER TYR HIS SER ASN ASP PHE HIS SER LEU CYS PRO GLY HIS PRO
ATG GCG CAG CTG AAG TCC TAC CAC TCG AAT GAC TTC CAC TCG CTG TGT CCC GGT CAC CCA
            120                   125                   130                  135
GLU ILE GLU HIS ASP ALA VAL GLU VAL THR THR GLY PRO LEU GLY GLN GLY ILE SER ASN
GAA ATC GAG CAC GAC GCC GTC GAG GTC ACA ACG GGC CCG CTC GGC CAG GGT ATC TCG AAC
            140                   145                   150                  155
SER VAL GLY LEU ALA ILE ALA THR LYS ASN LEU ALA ALA THR TYR ASN LYS PRO GLY PHE
TCT GTT GGT CTG GCC ATA GCC ACC AAA AAC CTG GCT GCC ACG TAC AAC AAG CCG GGC TTT
            160                   165                   170                  175
ASP ILE ILE THR ASN LYS VAL TYR CYS MET VAL GLY ASP ALA CYS LEU GLN GLU GLY PRO
GAT ATC ATC ACC AAC AAG GTG TAC TGC ATG GTT GGC GAT GCC TGC TTG CAG GAG GGC CCT
            180                   185                   190                  195
ALA LEU GLU SER ILE SER LEU ALA GLY HIS MET GLY LEU ASP ASN LEU ILE VAL LEU TYR
GCT CTC GAG TCG ATC TCG CTG GCC GGC CAC ATG GGG CTG GAC AAT CTG ATT GTG CTC TAC
            200                   205                   210                  215
ASP ASN ASN GLN VAL CYS CYS ASP GLY SER VAL ASP ILE ALA ASN THR GLU ASP ILE SER
GAC AAC AAC CAG GTC TGC TGT GAC GGC AGT GTT GAC ATT GCC AAC ACG GAG GAC ATC AGT
            220                   225                   230                  235
ALA LYS PHE LYS ALA CYS ASN TRP ASN VAL ILE GLU VAL GLU ASN ALA SER GLU ASP VAL
GCC AAG TTC AAG GCC TGC AAC TGG AAC GTG ATC GAG GTC GAG AAC GCT TCC GAG GAC GTG
            240                   245                   250                  255
ALA THR ILE VAL LYS ALA LEU GLU TYR ALA GLN ALA GLU LYS HIS ARG PRO THR LEU ILE
GCT ACC ATT GTC AAG GCC TTG GAG TAC GCG CAG GCC GAG AAG CAC AGA CCA ACA CTT ATC
            260                   265                   270                  275
ASN CYS ARG THR VAL ILE GLY SER GLY ALA ALA PHE GLU ASN HIS CYS ALA ALA HIS GLY
AAC TGC AGA ACT GTG ATT GGA TCG GGT GCT GCG TTC GAG AAC CAC TGT GCT GCG CAC GGT
            280                   285                   290                  295
ASN ALA LEU GLY GLU ASP GLY VAL ARG GLU LEU LYS ILE LYS TYR GLY MET ASN PRO ALA
AAC GCT CTG GGC GAG GAC GGT GTG CGC GAG CTC AAA ATC AAG TAC GGC ATG AAC CCG GCC
            300                   305                   310                  315
GLN LYS PHE TYR ILE PRO GLN ASP VAL TYR ASP PHE PHE LYS GLU LYS PRO ALA GLU GLY
CAG AAG TTC TAC ATT CCG CAG GAC GTG TAC GAC TTC TTC AAG GAG AAG CCG GCC GAG GGC
            320                   325                   330                  335
ASP LYS LEU VAL ALA GLU TRP LYS SER LEU VAL ALA LYS TYR VAL LYS ALA TYR PRO GLU
GAC AAG CTG GTG GCC GAA TGG AAG AGT CTC GTG GCC AAG TAC GTC AAG GCG TAC CCT GAG
            340                   345                   350                  355
GLU GLY GLN GLU PHE LEU ALA ARG MET ARG GLY GLU LEU PRO LYS ASN TRP LYS SER PHE
GAG GGC CAG GAG TTT TTC GCG CGG ATG AGA GGC GAG CTG CCA AAG AAC TGG AAG TCG TTC
            360                   365                   370                  375
```

Fig. 18C

```
LEU PRO GLN GLN GLU PHE THR GLY ASP ALA PRO THR ARG ALA ALA ALA ARG GLU LEU VAL
CTG CCG CAG CAG GAA TTC ACC GGC GAC GCT CCT ACA AGG GCC GCT GCC AGA GAG CTT GTG
        380               385                   390                   395
ARG ALA LEU GLY GLN ASN CYS LYS SER VAL ILE ALA GLY CYS ALA ASP LEU SER VAL SER
AGA GCC CTG GGG CAG AAC TGC AAG TCG GTG ATT GCC GGT TGC GCA GAC CTG TCT GTG TCT
        400               405                   410                   415
VAL ASN LEU GLN TRP PRO GLY VAL LYS TYR PHE MET ASP PRO SER LEU SER THR GLN CYS
GTC AAT TTG CAG TGG CCA GGG GTG AAA TAT TTC ATG GAC CCC TCG CTG TCC ACG CAG TGT
        420               425                   430                   435
GLY LEU SER GLY ASP TYR SER GLY ARG TYR ILE GLU TYR GLY ILE ARG GLU HIS ALA MET
GGC CTG AGC GGC GAC TAC TCC GGC AGA TAC ATT GAG TAC GGA ATC AGA GAA CAC GCC ATG
        440               445                   450                   455
CYS ALA ILE ALA ASN GLY LEU ALA ALA TYR ASN LYS GLY THR PHE LEU PRO ILE THR SER
TGT GCT ATC GCC AAT GGC CTT GCC GCC TAC AAC AAG GGC ACG TTC CTG CCG ATC ACG TCG
        460               465                   470                   475
THR PHE PHE MET PHE TYR LEU TYR ALA ALA PRO ALA ILE ARG MET ALA GLY LEU GLN GLU
ACT TTC TTC ATG TTC TAC CTG TAC GCT GCC CCA GCC ATC AGA ATG GCC GGC CTG CAG GAG
        480               485                   490                   495
LEU LYS ALA ILE HIS ILE GLY THR HIS ASP SER ILE ASN GLU GLY GLU ASN GLY PRO THR
CTC AAG GCG ATC CAC ATC GGC ACC CAC GAC TCG ATC AAT GAG GGT GAG AAC GGC CCT ACG
        500               505                   510                   515
HIS GLN PRO VAL GLU SER PRO ALA LEU PHE ARG ALA TYR ALA ASN ILE TYR TYR MET ARG
CAC CAG CCG GTC GAG TCG CCA GCA TTG TTC CGG GCC TAT GCA AAC ATT TAC TAC ATG AGA
        520               525                   530                   535
PRO VAL ASP SER ALA GLU VAL PHE GLY LEU PHE GLN LYS ALA VAL GLU LEU PRO PHE SER
CCG GTC GAC TCT GCA GAA GTG TTT GGC CTG TTC CAA AAA GCC GTC GAG CTG CCA TTC AGC
        540               545                   550                   555
SER ILE LEU SER LEU SER ARG ASN GLU VAL LEU GLN TYR LEU ALA SER ARG ALA GLN ARG
TCG ATT CTG TCG CTC TCG AGA AAC GAG GTG CTG CAA TAC CTG GCA AGT CGA GCG CAG AGA
        560               565                   570                   575
ARG ARG ASN ALA ALA GLY TYR ILE LEU GLU ASP ALA GLU ASN ALA GLU VAL GLN ILE ILE
AGG CGC AAC GCG GCC GGC TAT ATT CTG GAG GAT GCG GAG AAC GCC GAG GTG CAG ATT ATT
        580               585                   590                   595
GLY VAL GLY ALA GLU MET GLU PHE ALA ASP LYS ALA ALA LYS ILE LEU GLY ARG LYS PHE
GGA GTT GGT GCA GAG ATG GAG TTT GCA GAC AAG GCC GCC AAG ATC TTG GGC AGA AAG TTC
        600               605                   610                   615
ARG THR ARG VAL LEU SER ILE PRO CYS THR ARG LEU PHE ASP GLU GLN SER ILE GLY TYR
AGG ACC AGA GTT CTC TCC ATC CCA TGC ACG CGG CTG TTT GAC GAG CAG TCG ATC GGC TAT
        620               625                   630                   635
ARG ARG SER VAL LEU ARG LYS ASP GLY ARG GLN VAL PRO THR VAL VAL VAL ASP GLY HIS
AGA CGC TCG GTT TTG AGA AAG GAC GGC AGA CAG GTG CCA ACG GTG GTG GTG GAC GGC CAC
        640               645                   650                   655
VAL ALA PHE GLY TRP GLU ARG TYR ALA THR ALA SER TYR CYS MET ASN THR TYR GLY LYS
GTT GCG TTC GGC TGG GAG AGA TAC GCT ACG GCG TCC TAC TGT ATG AAC ACG TAC GGC AAG
       · 660               665                   670                   675
SER LEU PRO PRO GLU VAL ILE TYR GLU TYR PHE GLY TYR ASN PRO ALA THR ILE ALA LYS
TCT CTG CCT CCA GAA GTG ATC TAC GAG TAC TTT GGA TAC AAC CCG GCA ACG ATT GCC AAG
        680               685                   690                   695
LYS VAL GLU ALA TYR VAL ARG ALA CYS GLN ARG ASP PRO LEU LEU LEU HIS ARG LEU PRO
AAG GTC GAA GCG TAC GTC CGG GCG TGC CAA AGA GAC CCT TTG CTG CTC CAC CGA CTT CCT
        700
GLY PRO GLU GLY LYS ALA ***
GGA CCT GAA GGA AAA GCC TAA CCACGAT AAAGTAAATA AGCTCTGATT AAGTAAGATG
                            2110
          AATAAGTTCT TTGTCTGTGA ATGCCACCCC ACAATAACCC CACAAATAAA ACTTTCACAC
          2160                                                   2210
          TTGCGTCAGA AACTGTCGAG CCGCACGGGA CTGACTGTTT GGCCGGCGTGC CTCTGTCCCC
                                                      2260
          ACACGGATAT TTCGCACGGA ACAGAAACCA TTCGACAAGG GGTTGCTGCC GATACCAAAT
                                           2310
          AGAATCGATC GGATCC
                2350
```

60

Fig. 19

Fig. 20

Identical sequences in -1000 region of DAS and MOX genes


DAS -1076
    TAGATATTTTCTGCCTCGTCGTACTCA-54N-GTGTGATG-8N-TCACC-9N-
    * ** ***** ************* * ******** *****


       * ** ********* **********************
      TCGAAATTTTTGCCGTCGTCGTACAGTGTGATGTCACC
  MOX -1052

   DAS                    -937
      ATCGCTTCGTACTCGCTCTGCAGCTTCGA
      **** * *** * * ************

      **** **** ** ********* ***
      ATCGAATGTAATGAGCTGCAGCTTGCGA
  MOX               -987